# EUROPEAN PATENT SPECIFICATION

(11) **EP 3 019 193 B1**
(45) Date of publication and mention of the grant of the patent: **25.09.2019**
(21) Application number: 14822269.8
(22) Date of filing: 10.07.2014
(51) Int. Cl.: A61K 39/395, A61P 37/00, A61K 39/00, A61K 39/008, A61K 45/06, A61K 35/15, C07K 16/28

(54) **APOPTOTIC CELL-MEDIATED INDUCTION OF ANTIGEN SPECIFIC REGULATORY T-CELLS FOR THE THERAPY OF AUTOIMMUNE DISEASES IN ANIMALS AND HUMANS**
DURCH APOPTOTISCHE ZELLEN VERMITTELTE EINLEITUNG ANTIGENSPEZIFISCHER REGULATORISCHER T-ZELLEN ZUR THERAPIE VON AUTOIMMUNKRANKHEITEN BEI TIEREN UND MENSCHEN
INDUCTION DE LYMPHOCYTES T RÉGULATEURS SPÉCIFIQUES D'ANTIGÈNES, MÉDIÉE PAR DES CELLULES APOPTOTIQUES, POUR LE TRAITEMENT DE MALADIES AUTO-IMMUNES CHEZ L'HOMME ET LES ANIMAUX

(30) Priority: 10.07.2013 US 201361844564 P
(43) Date of publication of application: 18.05.2016
(73) Proprietor: The U.S.A. as represented by the Secretary, Department of Health and Human Services, Rockville, MD 20852 (US)
(72) Inventor: CHEN, Wan, Jun, Bethesda, MD 20892 (US); KASAGI, Shimpei, Bethesda, MD 20892 (US); ZHANG, Pin, Bethesda, MD 20892 (US)
(74) Representative: Kasche, André
(86) International application number: PCT/US2014/046065
(87) International publication number: WO 2015/006519

(56) References cited:
- WO-A1-92/11869
- WO-A1-97/09351
- WO-A2-01/26679
- WO-A2-2004/091543
- US-A1- 2008 253 991
- US-A1- 2009 053 249
- US-A1- 2011 229 465
- US-A1- 2012 107 301
- RAMÓN MERINO ET AL: "Effect of Long-Term Anti-CD4 or Anti-CD8 Treatment on the Development oflprCD4-CD8-Double Negative T Cells and of the Autoimmune Syndrome in MRL-lpr/lprMice", JOURNAL OF AUTOIMMUNITY, vol. 8, no. 1, 1 February 1995 (1995-02-01), pages 33-45, XP055341924, GB ISSN: 0896-8411, DOI: 10.1006/jaut.1995.0003
- NASH RICHARD A ET AL: "High-dose immunosuppressive therapy and autologous peripheral blood stem cell transplantation for severe multiple sclerosis", BLOOD, AMERICAN SOCIETY OF HEMATOLOGY, US, vol. 102, no. 7, 1 October 2003 (2003-10-01), pages 2364-2372, XP002370122, ISSN: 0006-4971, DOI: 10.1182/BLOOD-2002-12-3908

## Description

### BACKGROUND OF THE INVENTION

Regulatory T cells (T_{reg} cells) hold promise for autoimmune disease therapy. However, a challenge remains as to how to induce antigen-specific T_{reg} cells that only target inflammatory immune cells without compromising the entire immune response. Peripheral immune tolerance is key to preventing overreactivity of the immune system to various antigens. CD4+CD25+Foxp3+ regulatory T (Treg) cells are critical for maintaining immune tolerance, and deficiency of Treg cells causes severe autoimmune diseases and chronic inflammation. Indeed, the emergence and characterization of CD4+ CD25+ Foxp3+ T_{reg} cells have offered the hope of developing novel immunotherapy for human autoimmune diseases and chronic inflammation.¹⁻⁴ However, the lack of knowledge of antigen specificity and the difficulty of expanding thymus-derived T_{reg} cells (tT_{reg} cells) have limited their potential clinical application. The discovery of TGFβ induction of T_{reg} cells (iT_{reg} cells) from peripheral naive CD4+ T cells has brought new hope of inducing antigen-specific T_{reg} cells for autoimmune disease therapy^{5,6}. However, published studies have been limited to the prevention of experimental diseases by pre-injection of in vitro induced antigen-specific iT_{reg} cells into unmanipulated mice or in vivo by induction of antigen-specific T_{reg} cells in naive mice before the disease is established. There is a considerable difference in the immune status of an unmanipulated, naive mouse and a mouse with an established disease. The immune tolerance toward self-tissues in naive mice is broken in mice with autoimmunity, where the autoantigen-responsive immune cells are uncontrollably activated and proinflammatory cytokines are produced. Treg cells that fully exhibit immunosuppressive capacity in the immune quiescent state in naive mice may lose their suppressive activity or even convert to effector cells under the dysregulated inflammation in mice with autoimmune diseases. This problem is particularly salient in clinical settings, in which patients with autoimmune disease present with an already dysregulated immune response. Therefore, a challenge is to make T_{reg} cells in the inflammatory, dysregulated immune system in animals with established autoimmune diseases, and ultimately in patients, that can specifically inhibit inflammation in the organs/tissues affected and treat the diseases, i.e. to reprogram the dysregulated immune system in animals and patients so that it is restored, or to direct it to an immune-tolerant state to the target auto-antigens in the tissues affected with autoimmunity.

US 2008/253991 A1 discloses a method for the treatment of Type I diabetes comprising administering anti-T-cell therapy (e.g. antibodies against CD4 or CD8) followed by administration of an autoantigen (e.g. GAD65).

Accordingly, there is a need in the art for novel autoimmune disease therapies.

### SUMMARY OF THE INVENTION

Disclosed is a therapeutic method for the treatment of autoimmune or autoinflammatory diseases by first breaking down the dysregulated immune system and then reprogramming the immune system to restore tolerance to the patient's self-antigens by induction of antigen specific regulatory T cells. It has been shown here that only with the combination of apoptosis, phagocytes, and antigen can antigen-specific T_{reg} cells be optimally generated and long-term immune tolerance developed, i.e., the proper antigenic peptide needs to be introduced in a timely manner into subjects in which an immunoregulatory milieu was created by apoptosis-triggered phagocytes.

Exemplary tolerizing and/or treatment methods involve a) identifying a subject as suffering from an autoimmune disease or disorder; performing at least one of the following steps: b) administering an effective amount of an anti-CD8 antibody and an anti-CD-20 antibody to the subject to induce depletion and apoptosis of B cells and T cells of the subject suffering from the autoimmune disease or disorder; and c) administering an autoantigen specific to the autoimmune disease or disorder that the subject is suffering from, whereby the subject is tolerized to the antigen of the autoimmune or autoinflammatory disease and the disease or disorder is treated. The invention also features kits for carrying out the methods.

Also disclosed is a method of tolerizing a subject suffering from an autoimmune or autoinflammatory disease or disorder to an antigen associated with the autoimmune disease or disorder comprising steps a to c in order: a) identifying a subject as suffering from an autoimmune disease or disorder; b) administering an effective amount of an anti-CD4 antibody, anti-CD8 antibody, or both to the subject to induce apoptosis in T cells of the subject suffering from the autoimmune disease or disorder; and c) administering an autoantigen specific to the autoimmune disease or disorder that the subject is suffering from, whereby the subject is tolerized to the antigen of the autoimmune or autoinflammatory disease.

Also disclosed is a method of treating a subject suffering from an autoimmune or autoinflammatory disease or disorder comprising steps a to c in order: a) identifying a subject as suffering from an autoimmune disease or disorder; b) administering an effective amount of an anti-CD4 antibody, anti-CD8 antibody, or both to the subject to induce apoptosis in T cells of the subject suffering from the autoimmune disease or disorder; and c) administering an autoantigen specific to the autoimmune disease or disorder that the subject is suffering from, whereby the subject is tolerized to the autoantigen, thereby treating the autoimmune or autoinflammatory disease or disorder.

In one embodiment, the autoantigen is one or more autoantigens selected from the group consisting of: the myelin basic protein (MBP), the myelin proteolipid protein (PLP), insulin, GAD65 (glutamic acid decarboxylase), DiaPep277, heat-shock proteins (Hsp65, Hsp90, DnaJ), immunoglobulin binding protein (BiP), heterogeneous nuclear RNPs, annexin V, calpastatin, type II collagen, glucose-6-phosphate isomerase (GPI), elongation factor human cartilage gp39, and mannose binding lectin (MBL).

In another embodiment of the above aspects, step b is performed more than once prior to the performance of step c. In a further embodiment of the above aspects, the time for performance of step b and the time of performance of step c are separated by 3 to 14 days. In still another embodiment of the above aspects, step b induces apoptosis in a subset of T cells. In another embodiment of the above aspects, performance of steps a, b, and c is more effective than the performance of either steps a and b or steps a and c alone.

In one embodiment of the above aspects, the method further comprises monitoring the subject for amelioration of at least one sign or symptom of an autoimmune disease or disorder.

In another embodiment of the above aspects, the autoimmune disease or disorder is selected from the group consisting of multiple sclerosis, diabetes mellitus and rheumatoid arthritis, Sjögren'ssyndrome, and systemic sclerosis.

In certain embodiments, the monitoring comprises a diagnostic test or assessment. In another embodiment, the diagnostic test or assessment is selected from the Expanded Disability Status Scale, the timed 25-foot walk test or the nine-hole peg test. In another related embodiment, the diagnostic test or assessment is selected from an oral glucose tolerance test (OGTT), glycosylated hemoglobin test or fasting plasma glucose test. In a further related embodiment, the diagnostic test or assessment is selected from the American College of Rheumatology (ACR) response, the Simplified Disease Activity Index (SDAI), the Clinical Disease Activity Index (CDAI) or the Global Arthritis Score (GAS).

In certain embodiments, the diagnostic test or assessment comprises determining the amount of inflammatory cell infiltration.

In another embodiment, the subject suffering from an autoimmune disease or disorder is at a late stage of disease.

In another embodiment, the method further comprises administration of an additional agent.

In one embodiment, the autoimmune disease or disorder is type 1 diabetes mellitus.

Also disclosed is a method of treating a subject suffering from an autoimmune disease or disorder that involves performing the following steps in order: a) identifying a subject as suffering from an autoimmune disease or disorder; b) administering an amount of an anti-CD8 antibody and an anti-CD-20 antibody to the subject effective to induce depletion and/or apoptosis of B cells and T cells (e.g., CD8⁺ T cells) of the subject suffering from the autoimmune disease or disorder; and c) administering an autoantigen specific to the autoimmune disease or disorder that the subject is suffering from, where the subject is tolerized to the autoantigen, thereby treating the autoimmune disease or disorder.

In another aspect, the invention features a kit comprising an effective amount of an anti-CD4 antibody, anti-CD8 antibody in a pharmaceutical carrier; an autoantigen specific to an autoimmune or autoinflammatory disease or disorder; and instructions for use in treating the autoimmune or autoinflammatory disease or disorder.

In a further aspect, the invention provides a kit comprising an effective amount of an autoantigen specific to an autoimmune disease or disorder; and instructions for use in treating or preventing the autoimmune disease or disorder in a subject, optionally when used in combination with administration of one or more of the following: administering an amount of an anti-CD8 antibody and an anti-CD-20 antibody to the subject effective to induce depletion and/or apoptosis of B cells and T cells (e.g., CD8⁺ T cells) of the subject suffering from the autoimmune disease or disorder.

### Definitions

To facilitate an understanding of the present invention, a number of terms and phrases are defined below.

As used herein, the singular forms "a", "an", and "the" include plural forms unless the context clearly dictates otherwise. Thus, for example, reference to "a biomarker" includes reference to more than one biomarker.

Unless specifically stated or obvious from context, as used herein, the term "or" is understood to be inclusive.

As used herein, the terms "comprises," "comprising," "containing," "having" and the like can have the meaning ascribed to them in U.S. Patent law and can mean "includes," "including," and the like; "consisting essentially of' or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

"Absence" of an autoantibody means that an autoantibody which is indicative for at least one autoimmune disorder is not immunologically detectable. Accordingly, the autoantibody is not bound to an autoantigen. Any immunoassay known in the art can be used, including an exemplary assay such as ELISA performed upon blood obtained from a subject (initially) identified as having an autoimmune disease or disorder and optionally undergoing a treatment as described herein.

As used herein, the term "antibody" is meant to refer to a full-length (i.e., naturally occurring or formed by normal immunoglobulin gene fragment recombinatorial processes) immunoglobulin molecule or an immunologically active (i.e., antigen-binding) portion of an immunoglobulin molecule, like an antibody fragment. The antibody can be a anti-CD4 or anti-CD8.

As used herein, the term "agent" is meant any small molecule chemical compound, antibody, nucleic acid molecule, or polypeptide, or fragments thereof.

As used herein, the term "autoantigen" is meant to refer to any antigen that stimulates autoantibodies in the organism that produced it.

As used herein, the term "autoimmune disease or disorder" is meant to refer to a disease state caused by an inappropriate immune response that is directed to a self-encoded entity which is known as an autoantigen. Examples of autoimmune diseases include vasculitis, arthritis, autoimmune diseases of the connective tissue, inflammatory bowel diseases, autoimmune diseases of the liver and the bile duct, autoimmune disease of the thyroid gland, dermatologic autoimmune diseases, neurologic immune diseases, Diabetes type I. Exemplary vasculitis can be selected from medium to small vessel vasculitis or large vessel vasculitis, exemplary arthritis can be selected from seronegative and seropositive rheumatoid arthritis, psoriatic arthritis, Bechterew's disease, juvenile idiopathic arthritis; exemplary inflammatory bowel diseases can be selected from Crohn's disease or ulcerative colitis; exemplary diseases of the liver and the bile duct can be selected from autoimmunohepatitis, primary biliary cirrhosis and primary sclerosing Cholangitis; exemplary autoimmune diseases of the thyroid gland can be selected from Hashimoto's thyreoiditis and Grave's disease; exemplary autoimmune diseases of the connective tissue can be selected from systemic lupus erythematosus (SLE) disease, Sjogren's syndrome (SS), scleroderma, dermato- and poly-myositis, Sharp syndrome, systemic sclerosis and CREST syndrome; exemplary neurologic autoimmune diseases can be selected from multiple sclerosis (MS), chronic inflammatory demyelating polyneuropathy (CI DP) and myasthenia gravis. Medium to small vasculitis can optionally be selected from classical panarteritis nodosa, granulomatosis with polyangiitis, microscopic panarteritis, Churg-Strauss syndrome Behcet's disease and the large vessel vasculitis can optionally be selected from giant cell arteritis, polymyalgia rheumatic and Takayasu's arteritis. An autoimmune disease or disorder in a subject can be identified by any art-recognized method, including by assessment of symptoms or by evaluation of marker levels (e.g., autoantibody levels).

As used herein, the term "autoinflammatory disease or disorder" is meant to refer to a group of disorders characterized by seemingly unprovoked inflammation.

As used herein, the terms "determining", "assessing", "assaying", "measuring" and "detecting" refer to both quantitative and qualitative determinations, and as such, the term "determining" is used interchangeably herein with "assaying," "measuring," and the like.

The term "subject" refers to an animal which is the object of treatment, observation, or experiment. By way of example only, a subject includes, but is not limited to, a mammal, including, but not limited to, a human or a non-human mammal, such as a non-human primate, murine, bovine, equine, canine, ovine, or feline.

As used herein, the term "tolerizing" is meant to refer to a failure to attack the body's own proteins and other antigens. The term "tolerizing" may also include inducing tolerance, inducing immunological tolerance, or rendering nonimmunogenic.

As used herein, the term "treating" is meant to include alleviating, preventing and/or eliminating one or more symptoms associated with inflammatory responses or an autoimmune disease. It will be appreciated that, although not precluded, treating a disease or condition does not require that the disease, condition, or symptoms associated therewith be completely eliminated.

### DESCRIPTION OF THE DRAWINGS

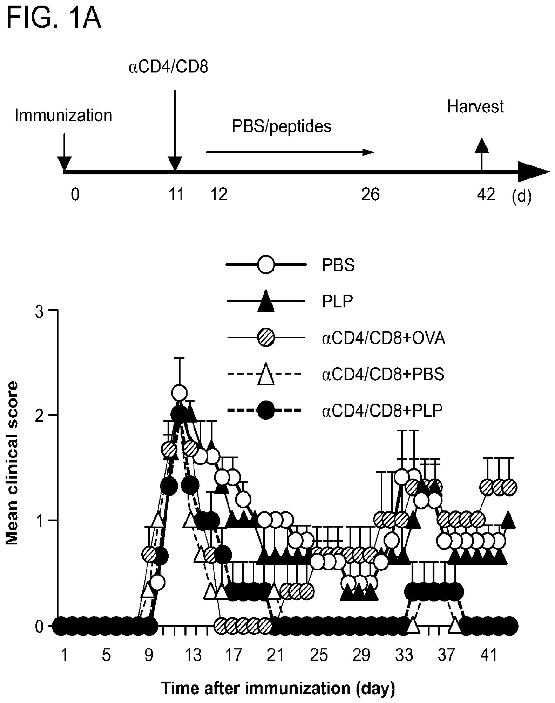

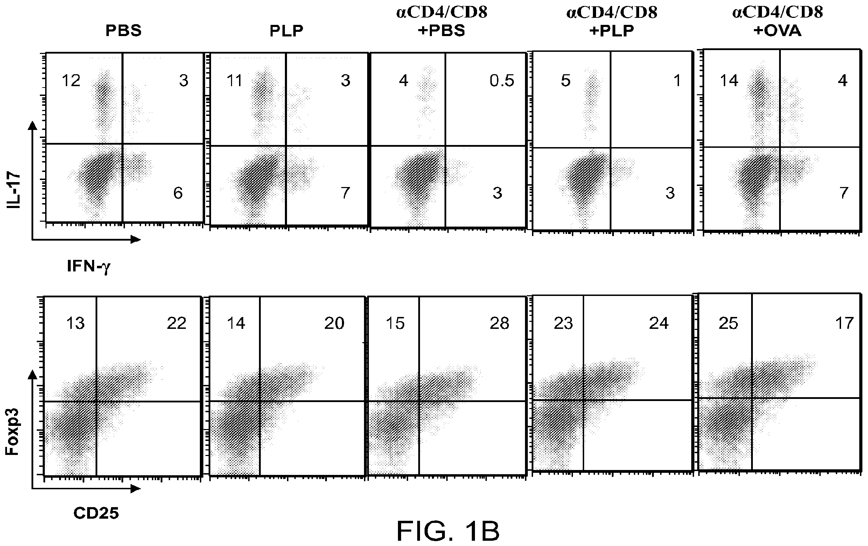

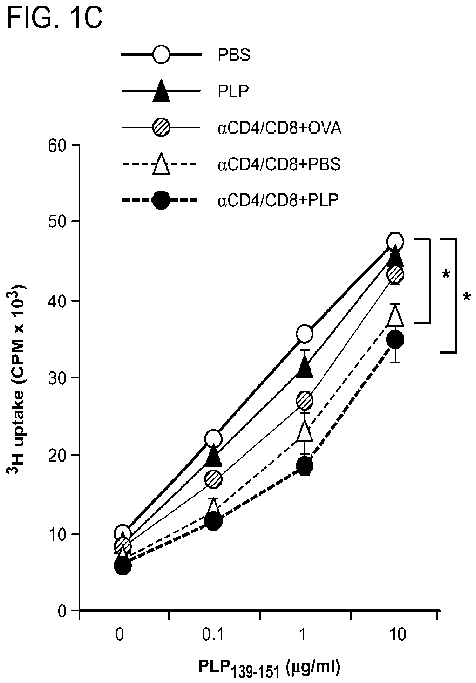

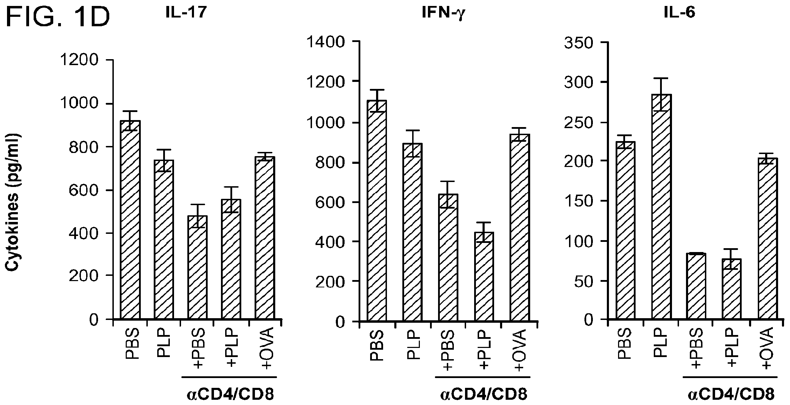

***Figures 1a to 1d*** show that the combination of T cell apoptosis and peptide administration suppressed Experimental autoimmune encephalomyelitisEAE. SJL mice were immunized with pPLP peptides to induce EAE (day 0). In Figure 1a, the upper panel shows the experimental scheme, while the lower panelshows clinical scores of EAE in SJL mice (mean ± s.e.m, n=3 mice). 5µg of pPLP or pOVA was injected each day. PBS (untreated), PLP (pPLP alone), αCD4/CD8+PBS (deletion alone), αCD4/CD8+PLP (deletion plus pPLP), αCD4/CD8+OVA (deletion plus pOVA). Figure 1b shows flow cytometry results for IL-17+ versus IFN-γ+ (upper panel) or CD25+ versus Foxp3+ (lower panel) within CD4+ T cells in the spinal cords (pooled in each group) at the end of the experiments. In Figure 1c, splenocytes (pooled from each group) were stimulated by pPLP, and T cell proliferation was assessed by H-thymidine incorporation (mean ± s.d. of triplicate measurements). In Figure 1d, protein levels of IL-17, IFN-γ, and IL-6 in the cultured supernatants of the same splenocytes as in Figure 1c were measured by ELISA (mean ± s.d. of duplicate measurements). * P<0.05 determined by Student's t test. Data of a representative example selected from two independent experiments are shown.

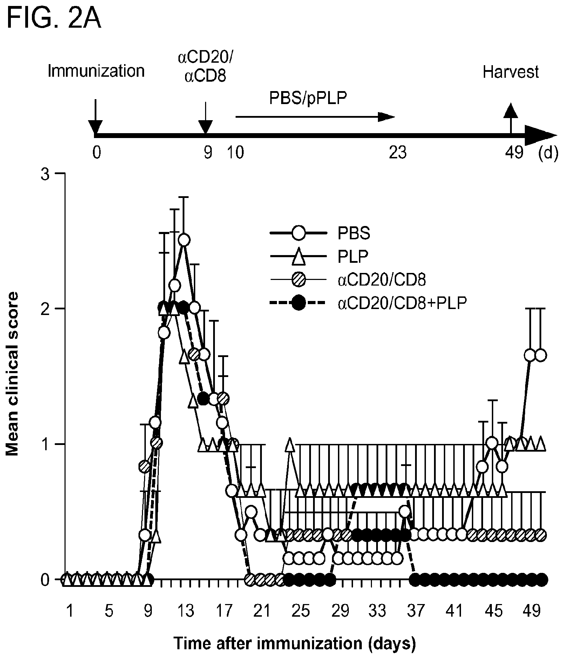

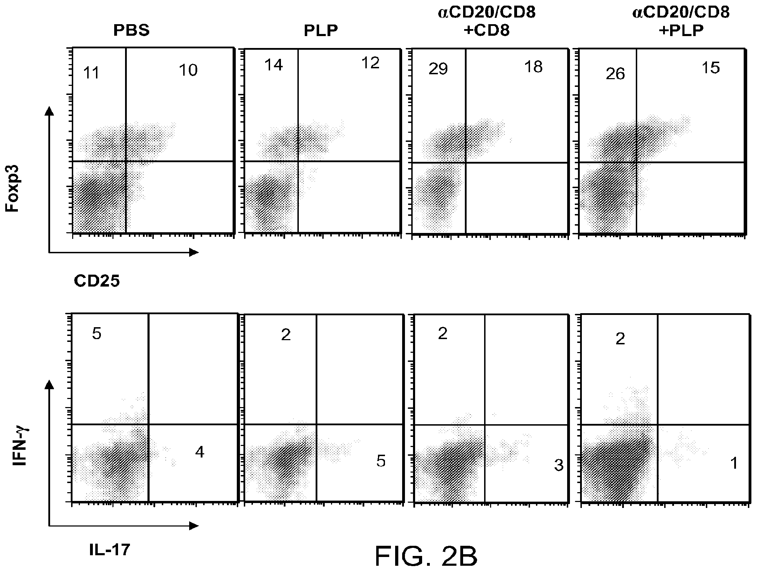

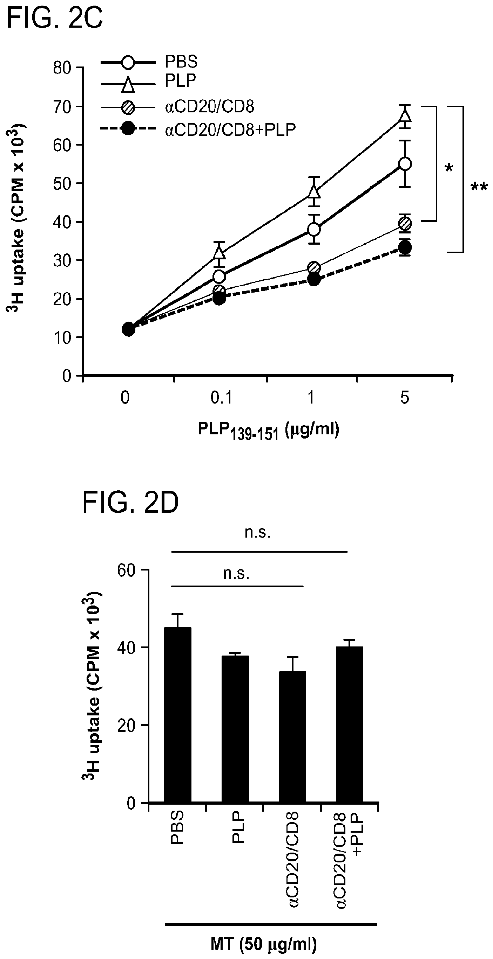

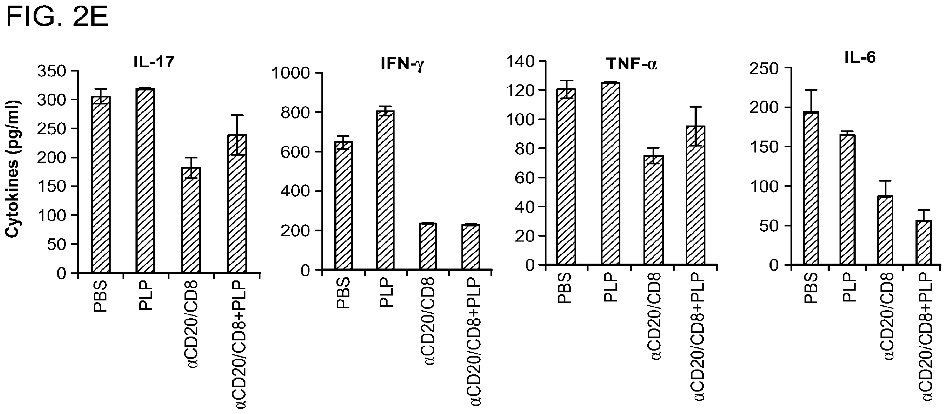

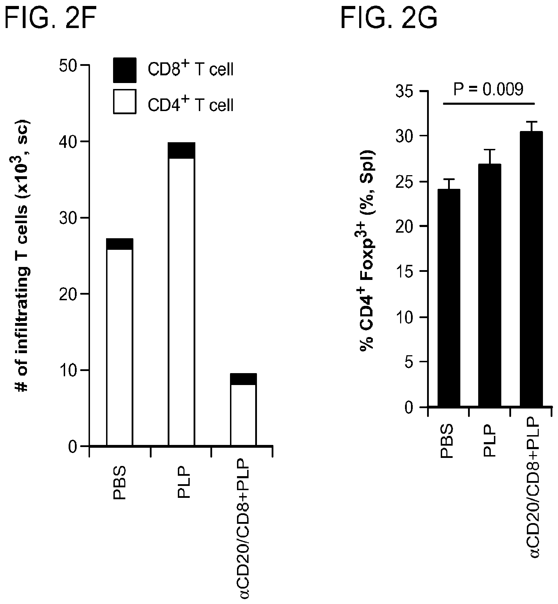

***Figures 2a to 2g*** show the therapeutic effects of B cell apoptosis and peptide administration on EAE. SJL mice were immunized with pPLP and CFA (day 0). 9 days after immunization, mice were injected with anti-CD8- and CD20- specific antibodies, followed by 5 µg of pPLP i.p. every other day for 14 days. In Figure 2a, The mean clinical scores of EAE are shown (mean ± s.e.m.) in mice treated with PBS (PBS), pPLP alone (PLP), CD8- and CD20-specific antibody (αCD20/CD8), or CD8- and CD20- specific antibody plus PLP (αCD20/CD8+PLP). Each group contained 3 mice. Figure 2b presents flow cytometry results for CD4+ T cells in the spinal cords. The numbers indicate the frequencies of Foxp3+ cells (Upper panel) or IL-17+ IFN-γ- and IL-17- IFN-γ+ cells (Lower panel). In Figures 2c and 2d, splenocyes (pooled in each group of the mice) were stimulated by either pPLP (c) or MT (d), and T cell proliferation was assessed by H-thymidine incorporation (mean ± s.d. of triplicate measurements). In Figure 2e, pPLP (1.0 µg/ml)-specific IL-17, IFN-γ, TNF-α and IL-6 cytokines in the cultured supernatants of the same splenocytes as in Figures 2c and 2d were measured by ELISA. Figure 2f shows the total number of infiltrating T cell in the spinal cords determined by FACS, while Figure 2g shows the frequency of Foxp3+ T cells in CD4+ T cells in the spleen. *P<0.05, **P<0.01, determined by Student's t test. Data of a representative example selected from two independent experiments are shown.

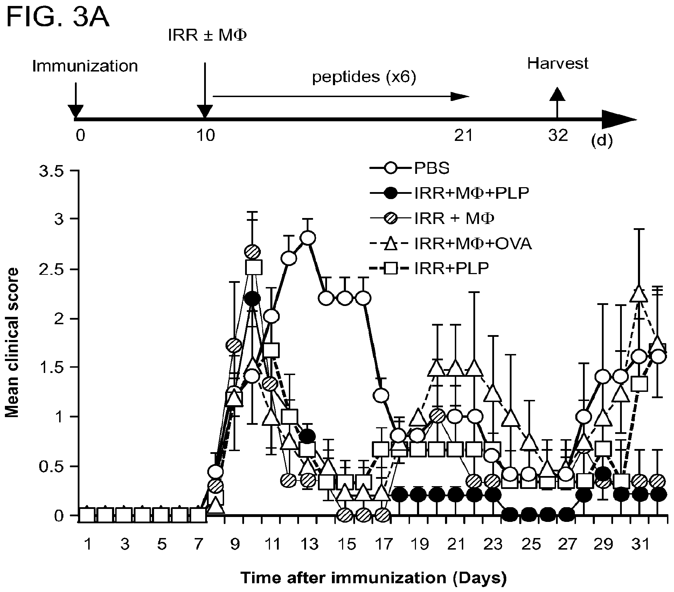

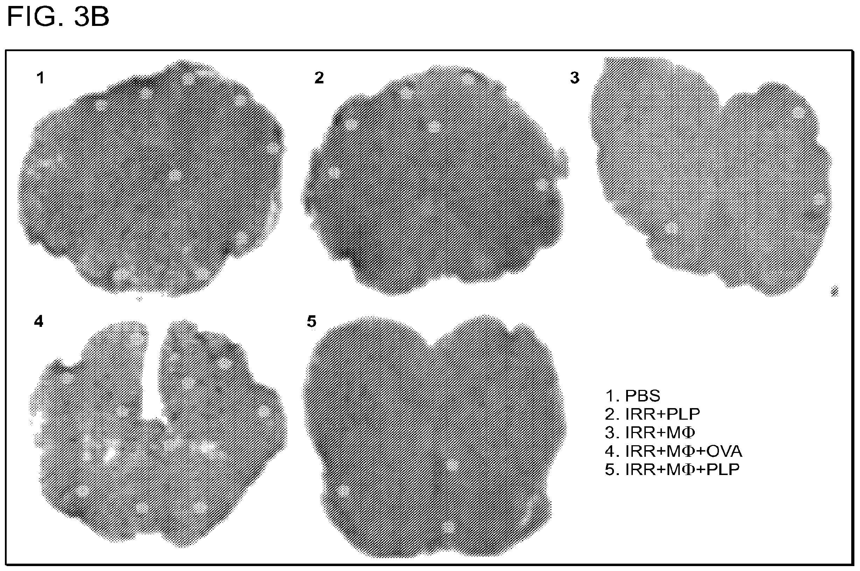

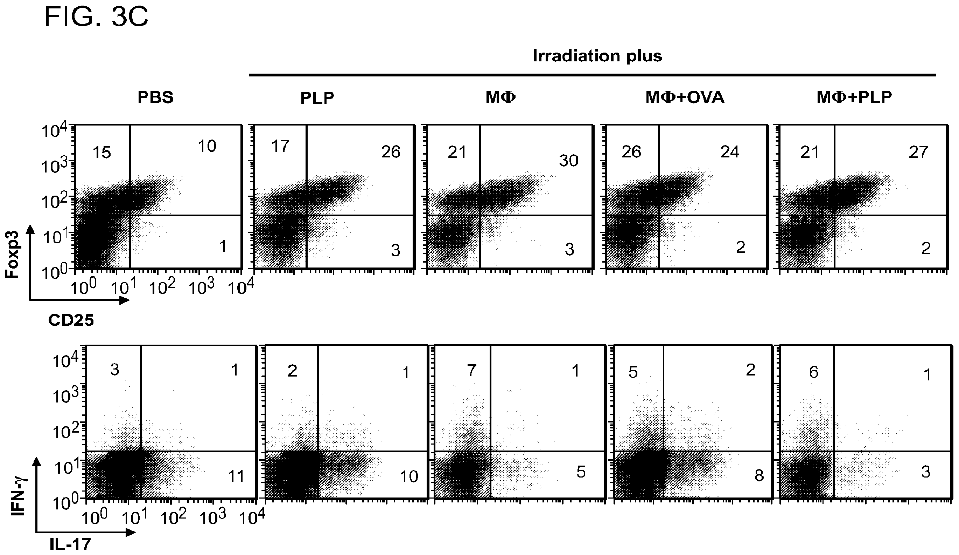

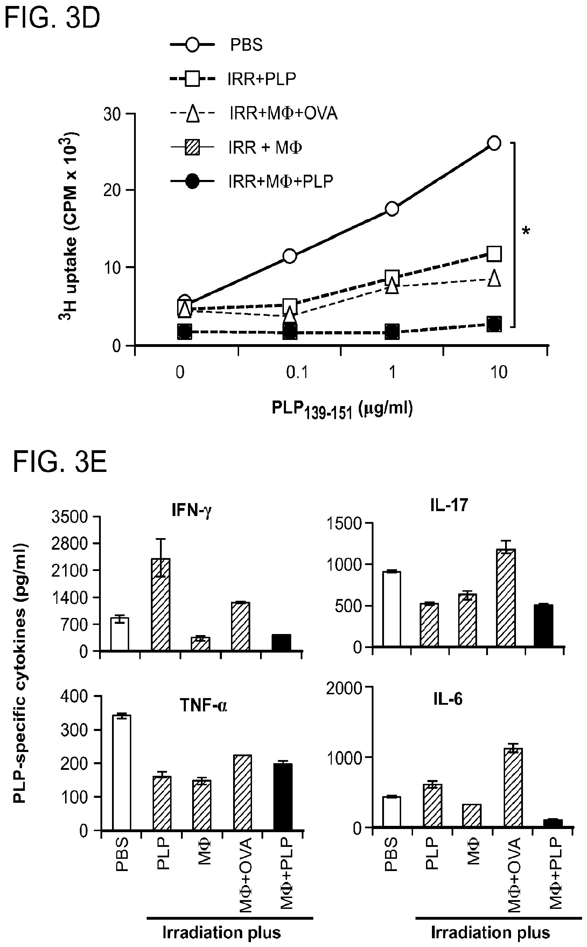

***Figures 3a to 3e*** show the function of professional phagocytes in apoptosis-antigen mediated therapy of EAE. SJL mice were immunized with pPLP and CFA to induce EAE (Day 0). Mice were either left untreated or irradiated with γ-irradiation (IRR) 10 days after immunization. Some mice received normal splenic macrophages and DCs (Mϕ) as indicated. Some mice were administered with 5µg of pPLP or pOVA every other day as indicated. In Figure 3a, the upper panel shows the experimental scheme, while the lower panel shows the clinical mean scores of EAE (mean ± s.e.m., n= 3-4 mice) for PBS (untreated control), IRR+Mϕ+PLP (irradiation plus Mϕ plus pPLP), IRR+Mϕ+OVA (irradiation plus Mϕ plus pOVA), IRR+Mϕ (irradiation plus Mϕ), and IRR+PLP (irradiation plus pPLP) treatments. Figure 3b shows histological analysis of spinal cord sections obtained from representative mice from indicated groups. Yellow dots indicate inflammatory infiltrates. Figure 3c shows flow cytometry results for CD4+ T cells in the spinal cords. The numbers in the upper panels indicate the frequencies of CD25+ Foxp3- (lower right) CD25+ Foxp3+ (upper right) and CD25- Foxp3+ (upper left) cells, while numbers in the lower panels indicate the frequencies of IL- 17+ IFN-γ- (lower right) IL-17+ IFN-γ+ (upper right) and IL-17- IFN-γ+ (upper left) cells. In Figure 3d, splenocytes were stimulated by pPLP, and antigen-specific T cell proliferation was assessed by H-thymidine incorporation (mean ± s.d. of triplicate measurements). In Figure 3e, the protein levels of pPLP-specific IL-17, IFN-γ, TNF-α, and IL-6 in the cultured supernatants of same splenocytes as in Figure 3d were measured by ELISA. *p < 0.01 determined by Student's t test The data were representative two independent experiments.

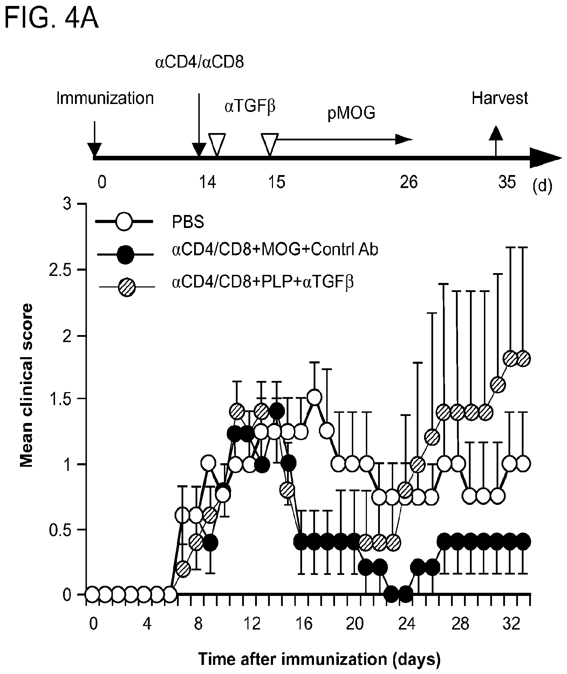

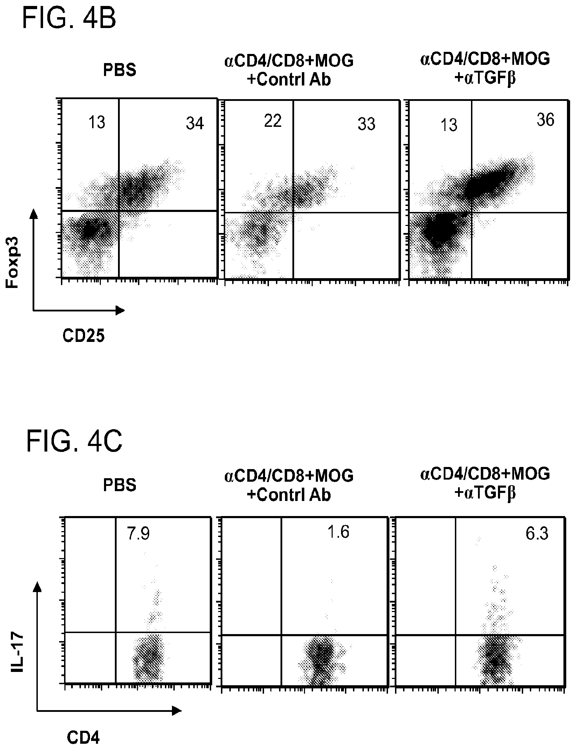

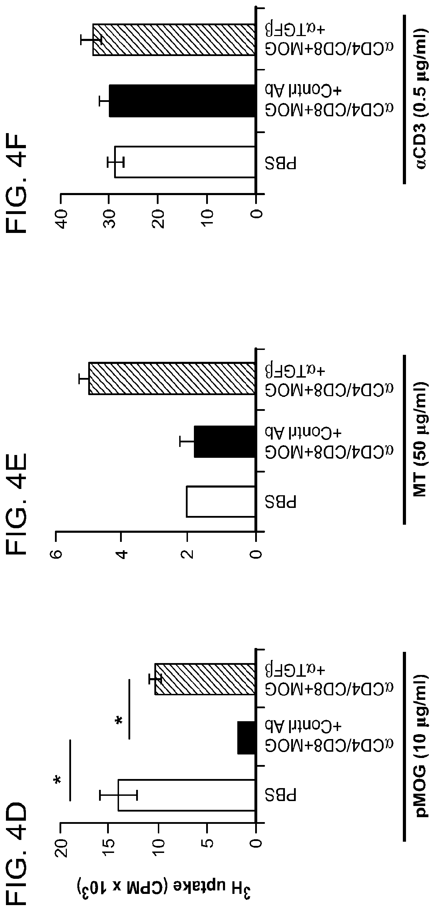

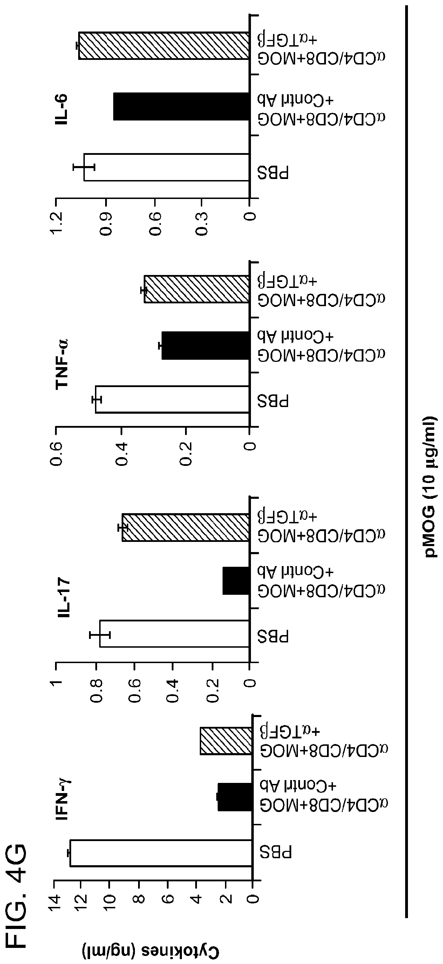

***Figures 4a to 4g*** demonstrate that TGFβ plays a key role in apoptosis-antigen combined therapy of EAE. C57BL/6J mice were immunized with pMOG (myelin oligodendrocyte glycoprotein (MOG)) to induce EAE (day 0) and were given anti-CD4- and CD8- specific antibody (αCD4/CD8) at day 14 to induce T cell apoptosis. Mice were treated with either anti-TGF-β (αTOPEβ or isotype control antibody mouse IgG1 (control Ab) twice from day 14 to 15 (indicated as inverted open trianglestriangles in the upper panel of Figure 4a). Figure 4a shows the clinical mean scores of EAE (mean ± s.e.m.) for PBS (untreated control, 3 mice), αCD4/CD8+MOG+ αTGFβ (αCD4/CD8 plus pMOG plus anti-TGF-β antibody, 5 mice), and αCD4/CD8+MOG+ Control Ab (αCD4/CD8 plus pMOG plus control antibody, 5 mice) treatments. Figures 4b and 4c show flow cytometry of CD4+ T cells in the spinal cords of the indicated groups. In Figure 4b, the numbers indicate the frequencies of CD25+ Foxp3+ (upper right) and CD25- Foxp3+ (upper left), while in Figure 4c, the numbers indicate the frequencies of IL-17+ CD4+ cells. In Figures 4d to 4f, splenocyes (pooled in each group before culture) were stimulated by pMOG (d), MT (e), and anti-CD3 (f), respectively, and T cell proliferation was assessed by 3H-thymidine incorporation (mean ± s.d. of tripilicate measurements). In Figure 4g, the protein levels of pMOG-specific IL-17, IFN-γ, TNF-α and IL-6 in the cultured supernatants of the same splenocytes as in Figure 4d was measured by ELISA (mean ± s.d. of duplicate measurements). * P < 0.01 determined by Student's t test. The inverted open trianglestriangles indicate the frequency of anti-TGFβ or control antibody (200 µg/day/mouse) treatment. Data of a representative example selected from four independent experiments are shown.

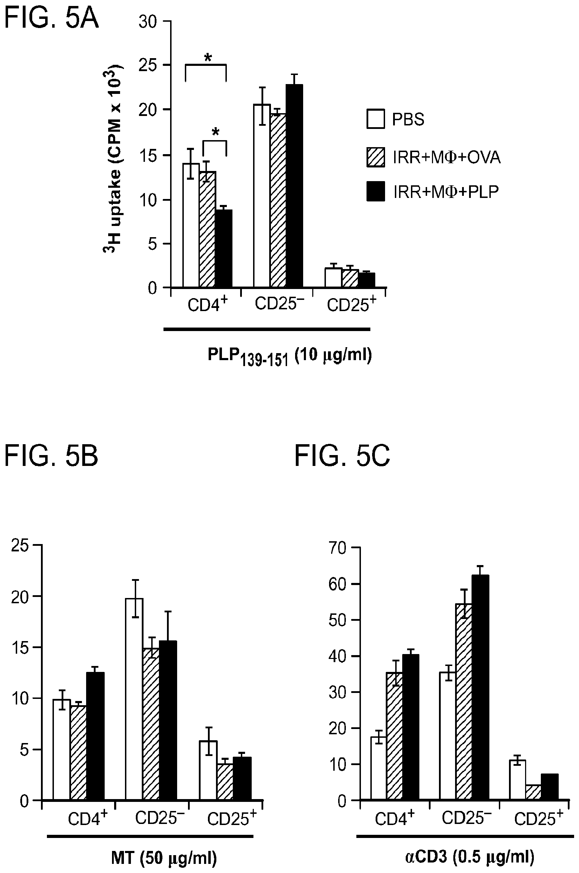

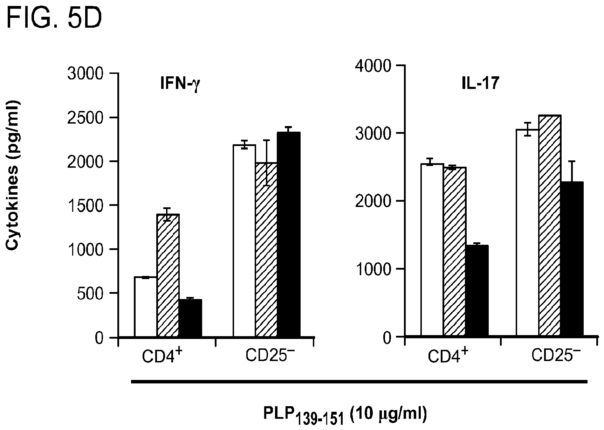

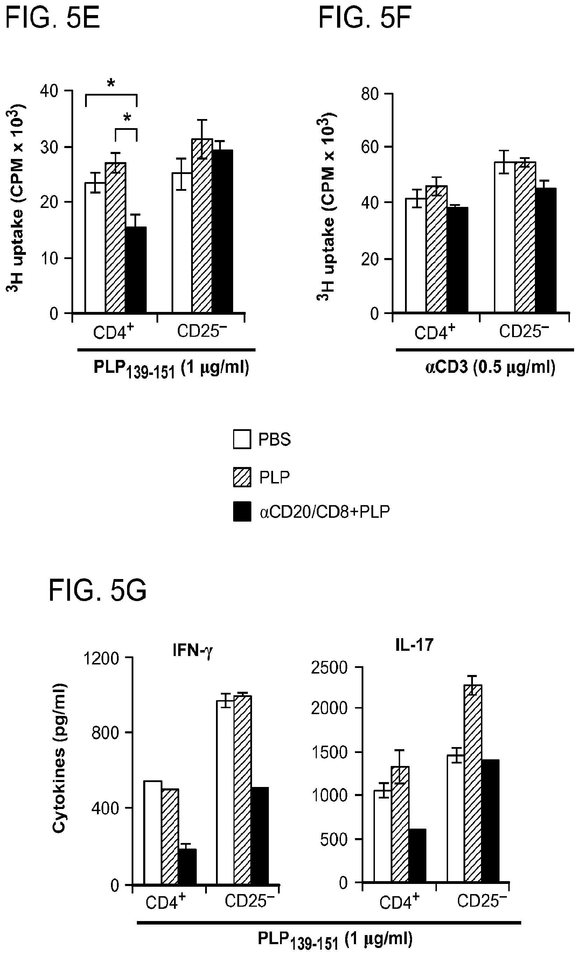

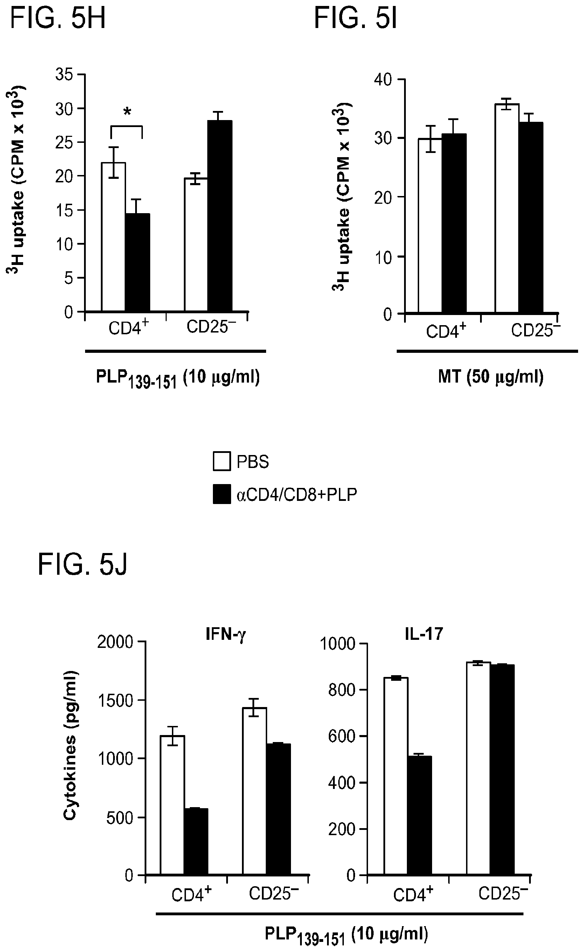

***Figures 5a to 5j*** show generation of antigen-specific CD4+ CD25+ T_{reg} cells in mice with long-term remission of EAE induced by apoptosis-antigen treatment. Splenocytes were isolated from the SJL/J mice shown in Figure 3a (Figures 5a-5d), Figure 2a (Figures 5e-5g), and Figure 1a (Figures 5h-5j). In each experiment, splenocytes were pooled from mice in each group and CD4+ , CD4+ CD25-, and CD4+ CD25+ T cells were purified and cultured with irradiated APCs in the presence of either pPLP (a, e, h) or MT (b, i) or anti-CD3 (c, f). T cell proliferation was assessed by H-thymidine incorporation (mean ± s.d. of triplicate wells). In Figures 5d, 5g and 5j, the supernatant levels of pPLP-specific IL-17 and IFN-γ of the indicated CD4+ T cell subsets were determined by ELISA (mean ± s.d. of duplicate wells). *P < 0.05, determined by Student's t test. Data of a representative example selected from two independent experiments are shown.

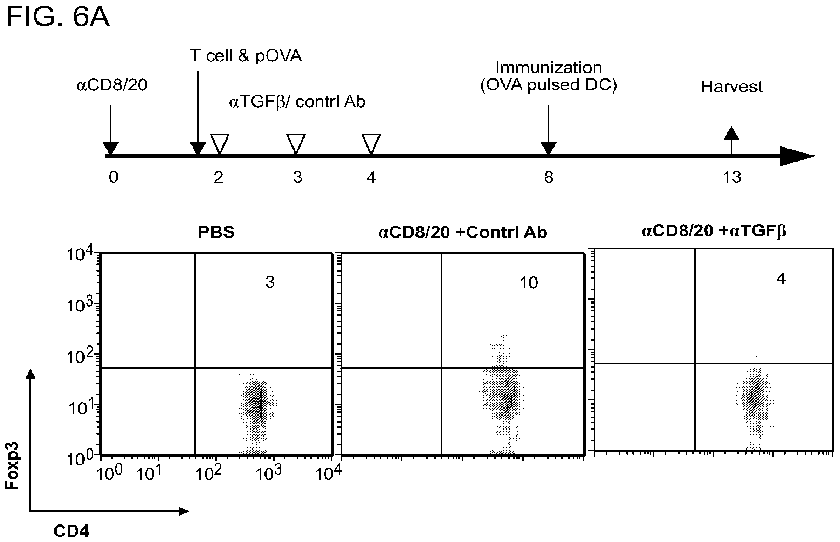

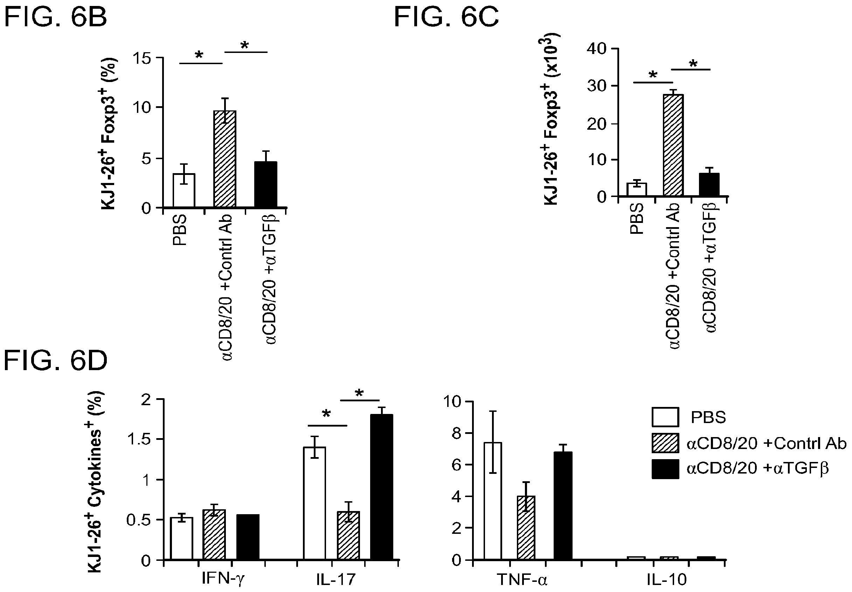

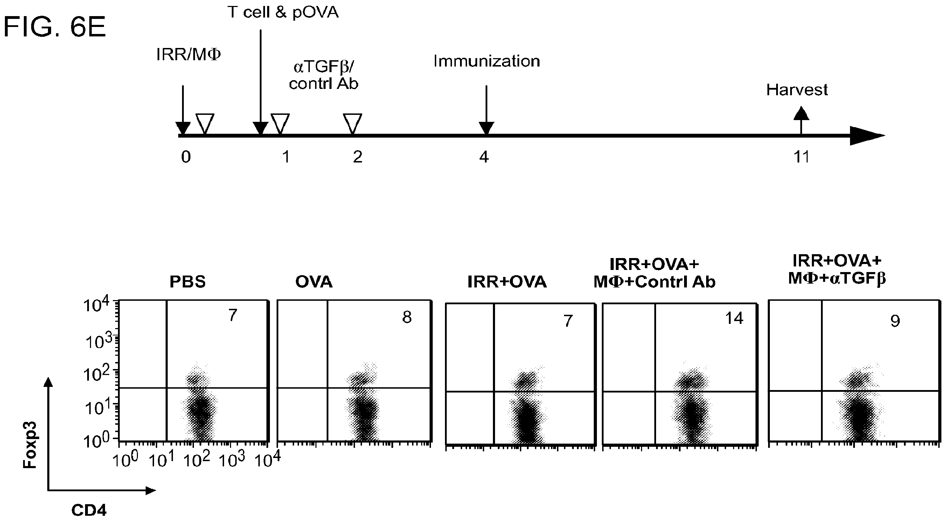

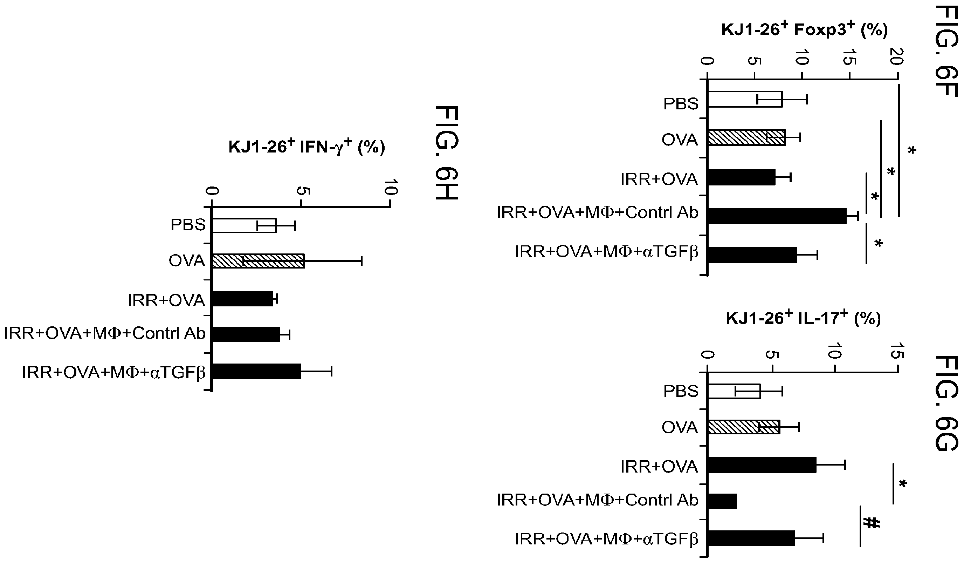

***Figures 6a to 6h*** show antigen-specific Foxp3+ T_{reg} cells were converted from naive CD4+ T cells by apoptosis-antigen combined therapy *in vivo.* Figures 6a to 6d demonstrate the conversion of pOVA-specific T_{reg} cells by anti-CD8 and CD20 specific antibodies (αCD8/CD20) plus pOVA *in vivo.* In Figure 6a, the upper panel depicts the experimental scheme, while in the lower panel, flow cytometry data for splenic CD4+ KJ1-26+ Foxp3+ T_{reg} cells in the BALB/c recipients are shown. Figures 6b and 6c show quantitative analysis of frequency (b) and total number (c) of the CD4+ KJ1-26+ Foxp3+ T_{reg} cells of Figure 6a. Figure 6d shows analysis of cytokine positive cells within CD4+ KJ1-26+ T cells in the BALB/c mice. Data are shown as mean ± s.d. of individual mice (n=3), and constitute a representative example selected from two independent experiments. Figures 6e to 6h show the conversion of pOVA-specific T_{reg} cells by γ- irradiation followed by macrophages plus pOVA administration *in vivo.* In Figure 6e, the upper panel shows the experimental scheme, while the lower panel shows a representative flow cytometry profile of splenic CD4+ KJ1-26+ Foxp3+ T_{reg} cells in BALB/c recipients. Figures 6f to 6h show the frequencies of Foxp3+ (f), IL-17+ (g) and IFN-γ+ (h) cells in the same CD4+ KJ1-26+ T cells of Figure 6e (mean ± s.d., n=3 mice per group) *P < 0.05, P =0.053, determined by Student's t test. The inverted triangles indicate the frequency of anti-TGFβ or control antibody (200 µg/day/mouse) treatment.

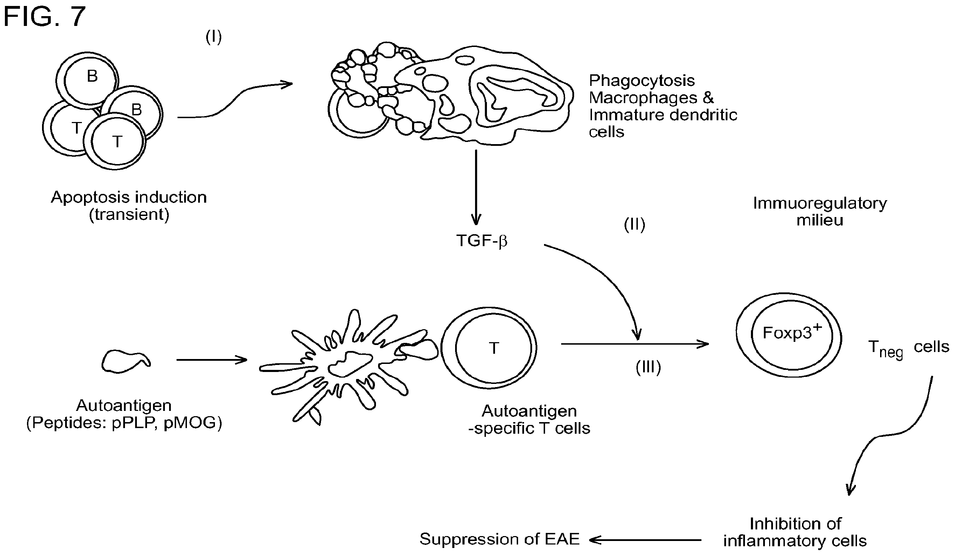

***Figure 7*** shows a schematic design of the study identifying therapy of EAE by generation of auto-antigen-specific Foxp3+ T_{reg} cells *in vivo.* The process can be divided into three steps. (I), Induction of transient yet sufficient number of apoptotic immune cells in vivo; (II), Apoptotic cells trigger professional phagocytes to produce immunosuppressive cytokine TGFβ, which then creates an immmunoregulatory milieu; and (III), Specific auto-antigenic-peptides are administered into mice that had a TGFβ-rich immunoregulatory microenvironment, under which naive CD4+ T cells are directed to differentiate into Foxp3+ T_{reg} cells rather than T effector cells. These generated antigen-specific T_{reg} cells may further prevent and suppress potential auto-antigen-specific inflammatory T effector cell differentiation to lead to a state of immune tolerance. Using this strategy, The dysregulated immune system in the mice with EAE was corrected and re-programmed. The disease of EAE is suppressed.

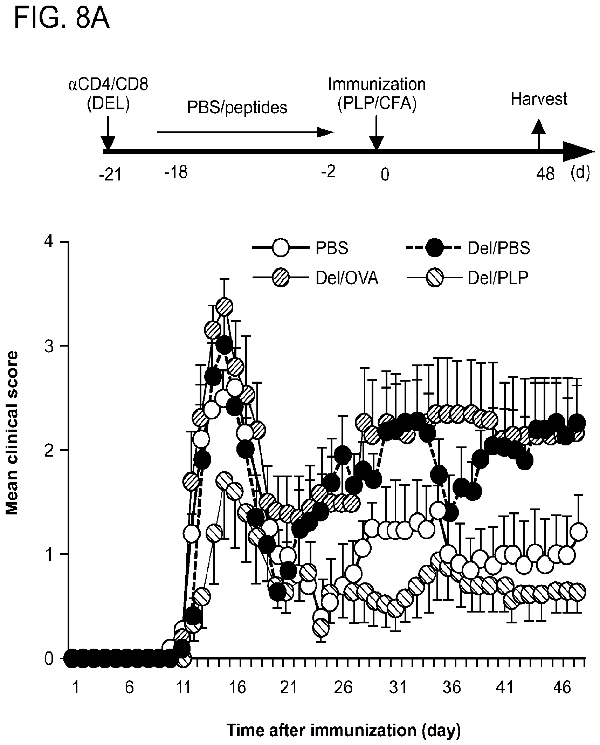

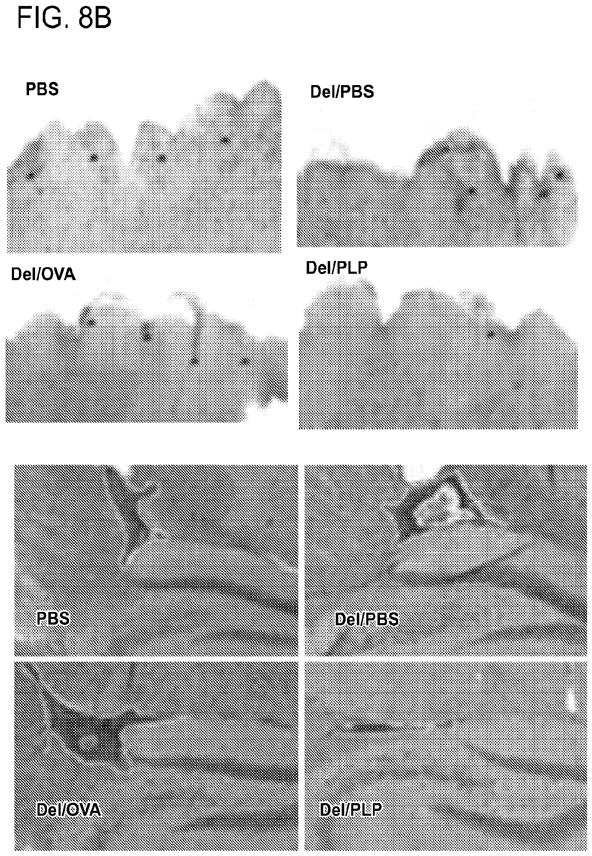

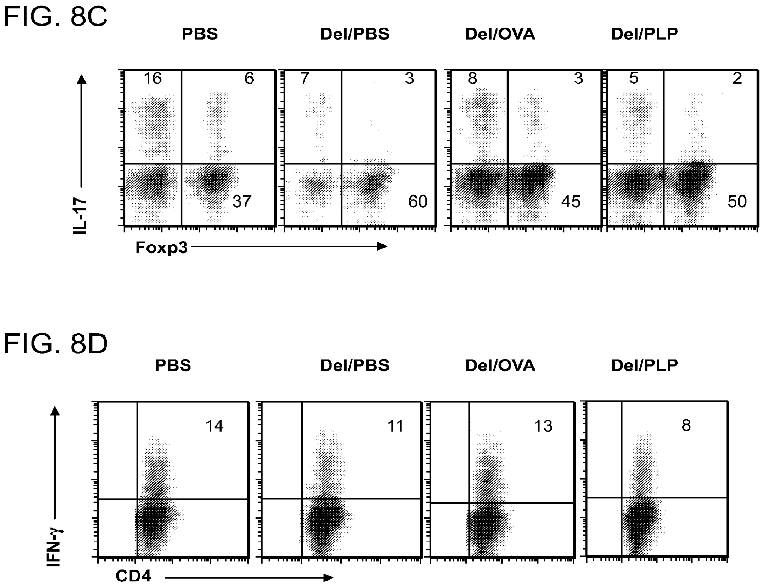

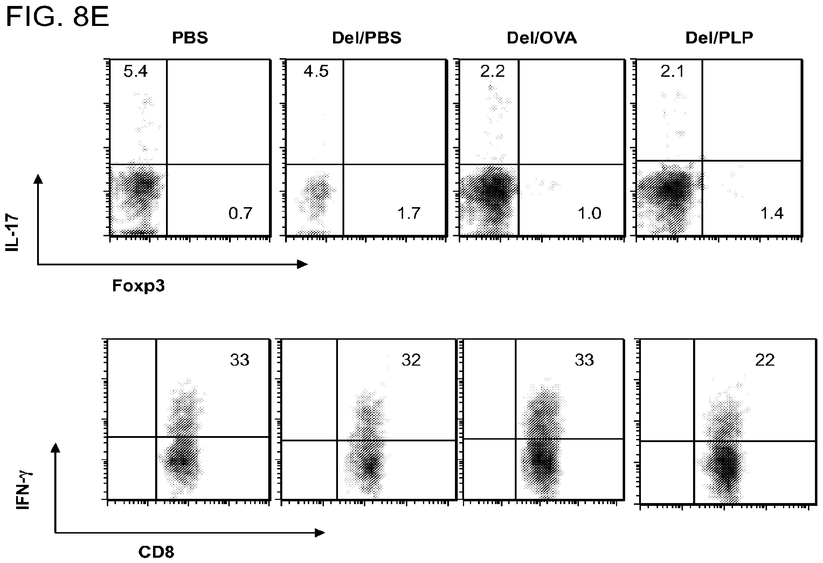

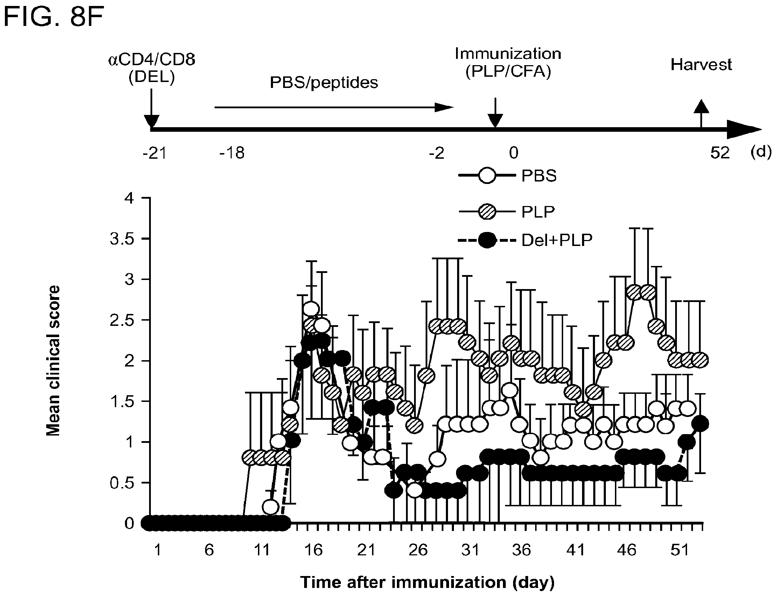

***Figures 8a to 8f*** show that the combination of T cell apoptosis and peptide administration prevented EAE. SJL mice were treated with CD4- and CD8- specific antibody (Deletion) 21 days before immunization, followed by pPLP administration (25µg/every other day for 16 days). Mice were sacrificed at day 48 after immunization. In Figure 8a, the upper panel shows the experimental scheme, while the lower panel shows the mean clinical score of EAE in SJL mice (mean ± s.e.m.) for PBS (untreated control, 10 mice), DEL/PBS (αCD4/CD8 plus PBS, 10 mice), DEL/PLP (αCD4/CD8 plus pPLP administration, 10 mice), and DEL/OVA (αCD4/CD8 plus pOVA administration, 10 mice) treatments. Data of two independent experiments were combined. Figure 8b shows results of histological analysis of brain and spinal cord. Data are shown as H&E staining of formalin-fixed sections obtained from representative mice from each group. Blue dots or areas surrounded by yellow dashed lines indicate inflammatory infiltrates. Figures 8c and 8d show flow cytometry data for IL-17, IFN-α, and Foxp3 expression within CD4+ T cells in the spinal cords (cells were pooled in each group). Figure 8e shows flow cytometry data for IL-17, IFN-ã, and Foxp3 expression in CD8+ T cells in the spinal cords (cells were pooled in each group). Figure 8f shows that administration of pPLP alone failed to prevent EAE. In some experiments, SJL mice were treated with PBS (PBS, 5 mice), with pPLP alone (PLP, 5 mice) or αCD4/CD8 plus pPLP (DEL+PLP, 5 mice) before immunization. Upper panel, the experimental scheme, Lower panel, the mean clinical score of EAE in SJL mice (mean ± s.e.m). Data of a representative example selected from two independent experiments are shown.

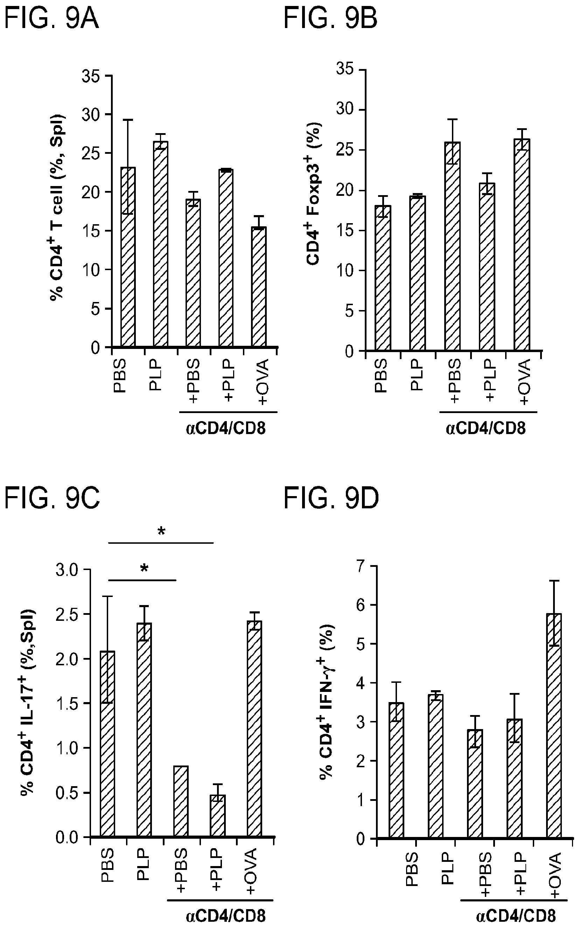

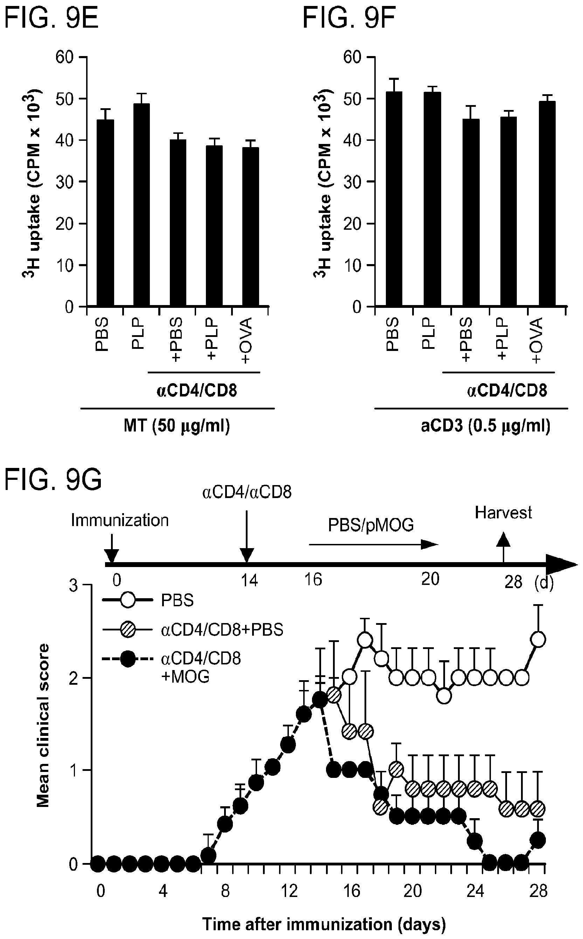

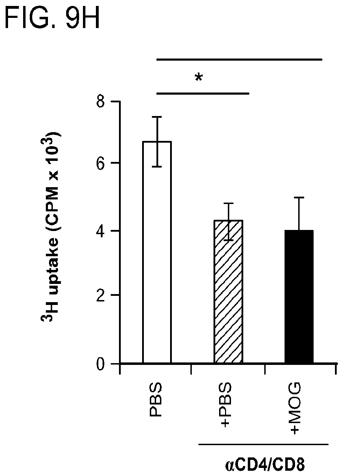

***Figures 9a to 9h*** show the therapeutic effect of T cell apoptosis-antigen administration in mice with established EAE induced by pPLP (SJL mice, a-f) or pMOG (C57BL/6 mice, g-h). Figure 9a shows the frequency of CD4+ T cells of splenocytes of SJL mice in the indicated groups. Frequencies of Foxp3+ (b), IL-17+ (c), and IFN-γ+(d) within CD4+ cells in the spleens were also determined by flow cytometry. Splenocytes from the mice in Figure 1a were pooled together in each group and re-stimulated by either MT(50 µg/ml) (e) or anti-CD3 (0.5 µg/ml) (f), and the respective T cell proliferative responses were assessed by 3H-thymidine incorporation. In Figure 9g, the upper panel shows the experimental scheme, while the lower panel shows the mean clinical scores of EAE in C57BL/6 mice. PBS (untreated control, 5 mice), αCD4/ CD8+PBS (αCD4/8 plus PBS, 5 mice), and αCD4/CD8+MOG (αCD4/8 plus pMOG injection, 5 mice). In Figure 9h, splenocytes from the mice in Figure 9g were pooled together in each group and re-stimulated by pMOG (10 µg/ml) in cultures. pMOG-specific T cell proliferation was assessed by 3H-thymidine incorporation. Data are shown as mean ± s.e.m in Figure 9g, and mean ± s.d. in the rest of the panels. Data of a representative example selected from four independent experiments are shown. *P<0.05, determined by student's t test.

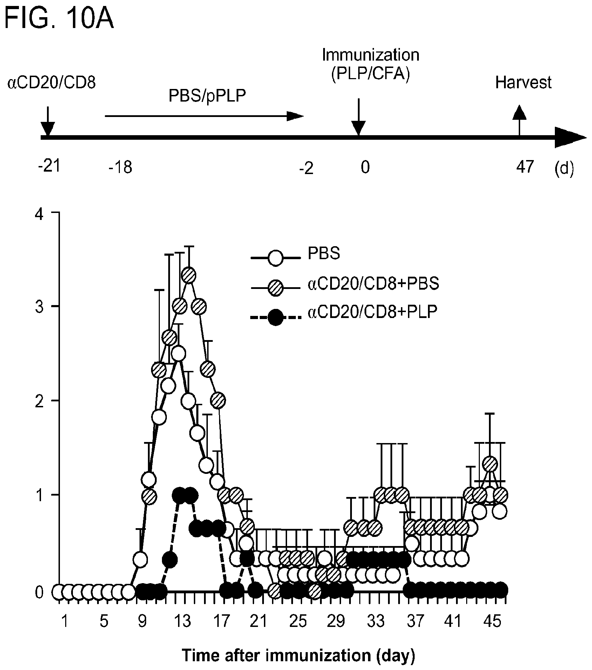

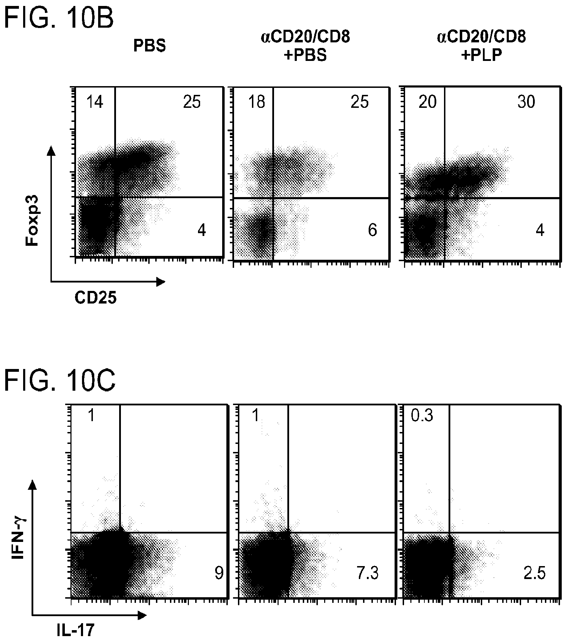

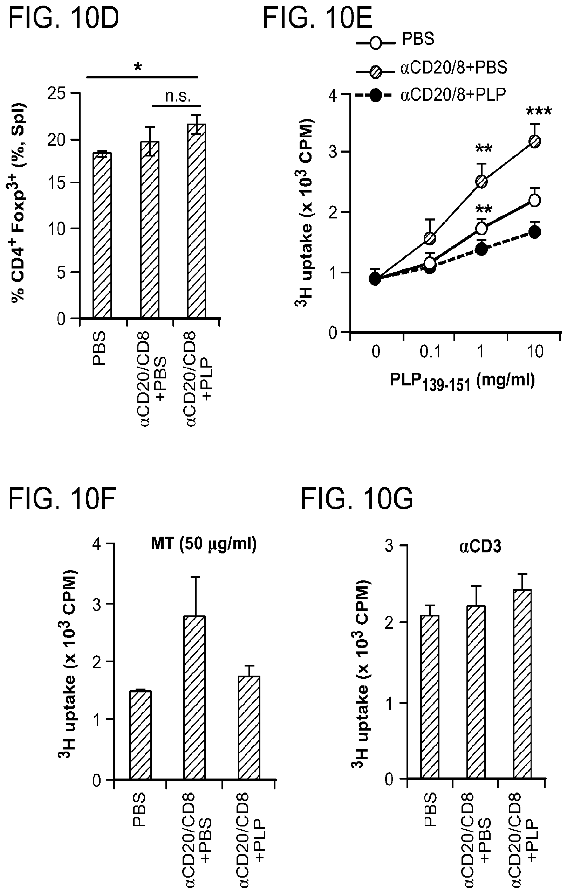

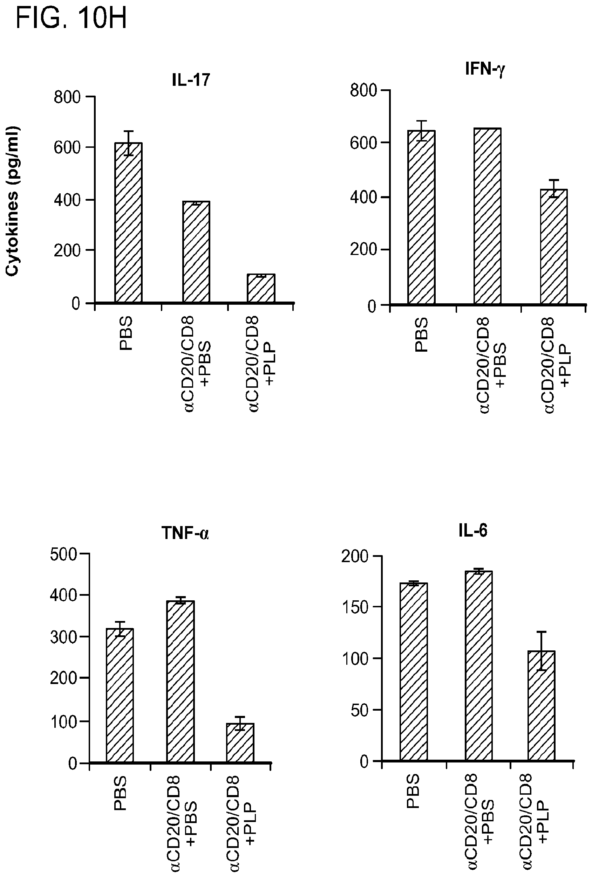

***Figures 10a to 10h*** show that a combination of B cell and CD8 T cell depletion and peptide administration prevents EAE in SJL mice. In Figure 10a, the upper panel depicts the experimental scheme, while in the lower panel, the mean clinical scores of EAE are plotted for (mean ± s.e.m.) PBS (untreated control, 3 mice), αCD20/CD8+PBS (αCD20/CD8 antibody treatment plus PBS, 3 mice), and αCD20/CD8+PLP (CD20/CD8 antibody treatment plus pPLP administration, 3 mice) treatments. Figure 10b shows results of flow cytometry for T_{reg} cells in gated CD4+ T cells in the spinal cords. Numbers in the quadrants indicate CD25+Foxp3- (upper left), CD25+Foxp3+ (upper right), and CD25-Foxp3+ (bottom right). Figure 10c shows results of flow cytometry of CD4+ T cells for cytokine producing cells in the spinal cords. The numbers in the quadrants indicate IL-17+IFN-α- (upper left) and IL-17-IFN-α+ (bottom right). Figure 10d shows a histogram of the frequency of Foxp3+CD4+ T cells in the spleens of the indicated groups (mean ± s.d. n = 3 mice), * P<0.005 (Student's t test). In Figures 10e to 10g, splenocytes were pooled in each group and re-stimulated by either pPLP (0-5 µg/ml, Figure 10e) or MT (50 µg/ml, Figure 10f) or anti-CD3 (0.5 µg/ ml, Figure 10g) for 3 days. The respective T cell proliferative responses were assessed by 3H-thymidine (mean ± s.d. of triplicate samples). ** P<0.05 (PBS vs. αCD20/8+PLP, αCD20/8+PBS vs. αCD20/8+PLP); ***P<0.01 (αCD20/8+PBS vs. αCD20/8+PLP). (Student's t test). In Figure 10h, the protein levels of IL-17, IFN-α, TNF-α and IL-6 in the culture supernatant of the same splenocytes were measured by ELISA (mean ± s.d. of duplicate samples). Data of a representative example selected from two independent experiments are shown.

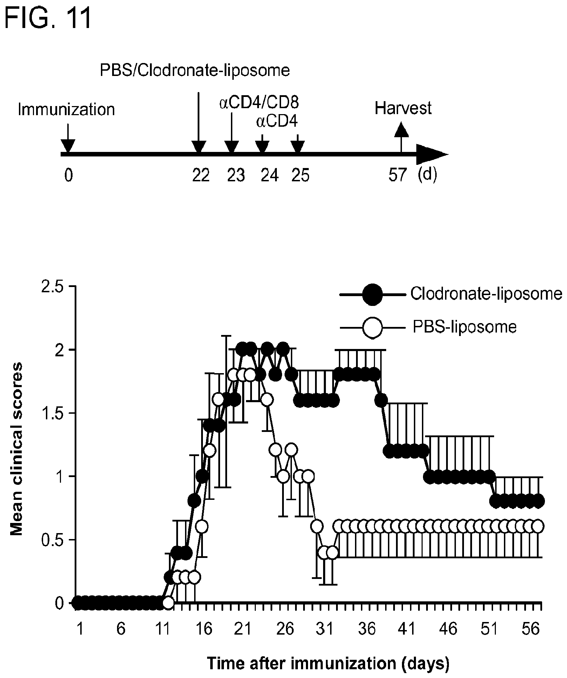

***Figure 11*** shows mean clinical scores for C57BL/6 mice treated with either PBS-liposome (n=5) or Clodranate-liposome (n=5), at day 22 after immunization. All mice received anti-CD4+anti-CD8 antibody (αCD4/CD8) (day 23) and anti-CD4 antibody (αCD4) (day 24-25) to deplete T cells, and were sacrificed at day 57. The upper panel depicts the experimental scheme, while the lower panel plots the clinical scores of EAE (mean ± s.e.m.)

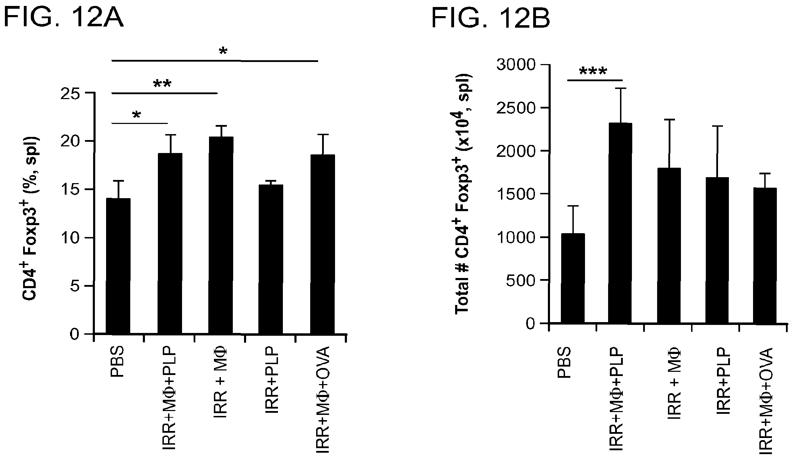

***Figures 12a and 12b*** show that CD4+Foxp3+ T_{reg} cells were increased in the EAE mice threated with irradiation plus phagocytes and pPLP in SJL mice. Frequency (a) and total number (b) of Foxp3+ CD4+ cells in the spleen of mice in the indicated groups were determined by flow cytometry. The data shown here are from the same mice of Fig. 3a. * P<0.05, ** P<0.01, *** P<0.005, determined by student's t test.

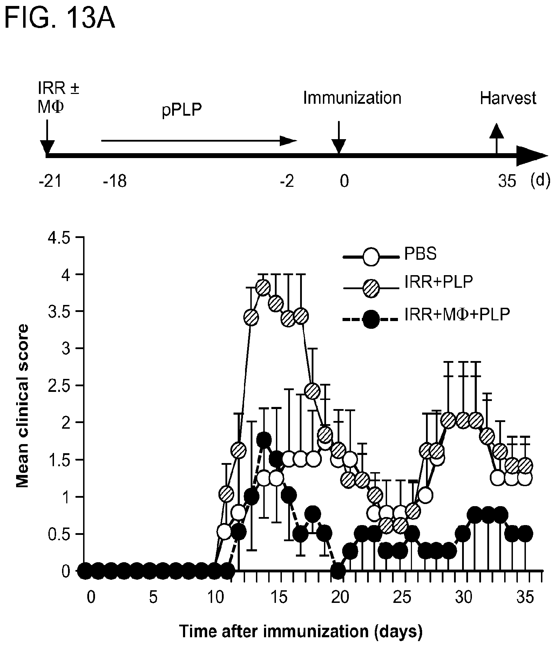

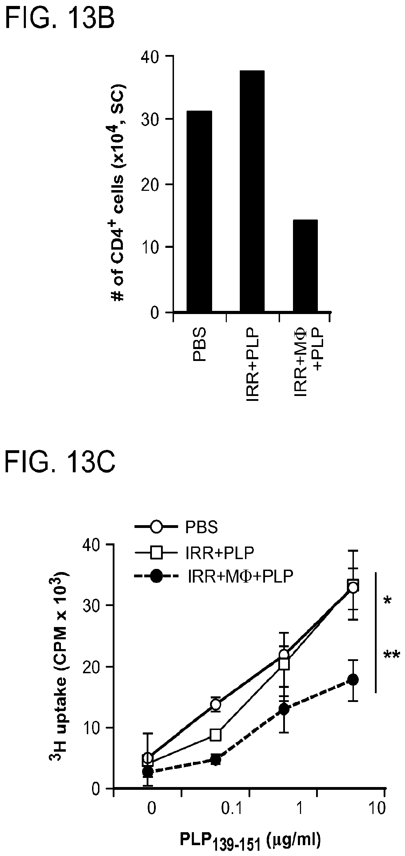

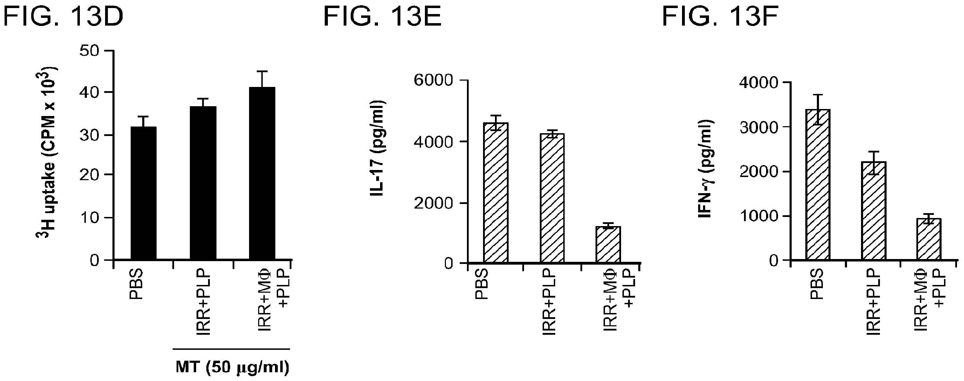

***Figures 13a to 13f*** show the function of professional phagocytes in apoptosis-antigen mediated prevention of remitting-relapsing EAE in SJL mice. The upper panel of Figure 13a depicts the experimental scheme, while the lower panel plots the mean clinical scores of EAE (mean ± s.e.m.) for PBS (untreated control, 4 mice), IRR+PLP (irradiation plus pPLP, 5 mice), IRR+Mϕ+PLP (irradiation plus macrophages and DCs plus pPLP, 4 mice) treatments. In Figure 13b, the number of infiltrating CD4+ T cells in the spinal cords (pooled in each group of mice) were detected by flow cytometry. In Figures 13c and 13d, splenocytes in the mice of Figure 13a were pooled together in each group and re-stimulated by either pPLP (0-10 µg/ml, c) or MT (50 µg/ml, d). The respective T cell proliferative responses were assessed by 3H-thymidine (mean ± s.d. of triplicate samples). *P=0.014 (IRR+Mϕ+PLP vs. IRR+PLP); **P=0.004 (IRR+Mϕ+PLP vs. PBS), determined by student's t test. In Figures 13e and 13f, the protein levels of IL-17 (e) and IFN-γ (f) in the culture supernatants of the splenocytes were measured by ELISA (mean ± s.d. of duplicate samples). Data of a representative example selected from two independent experiments are shown.

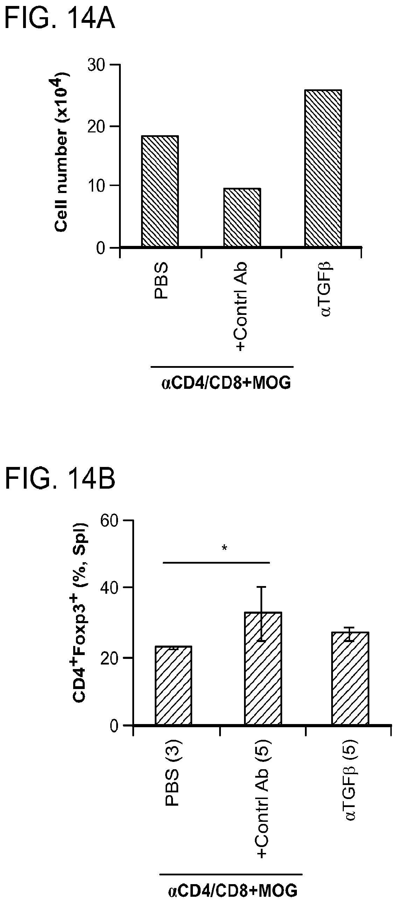

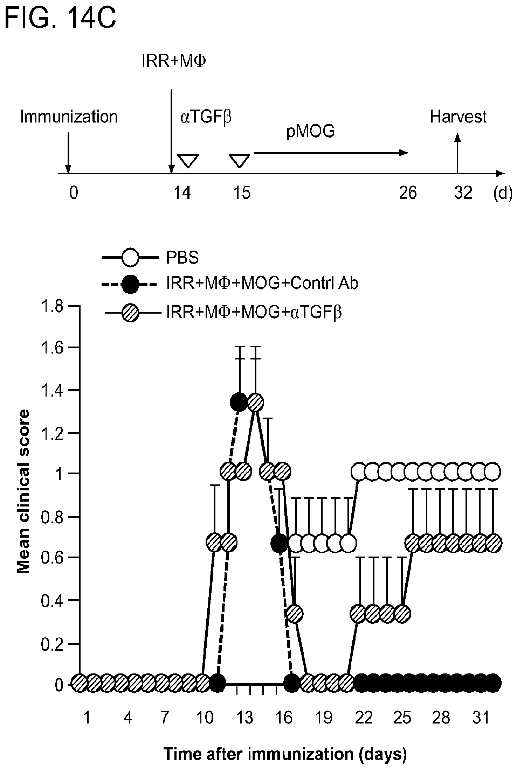

***Figures 14a to 14g*** show a key role for TGFβ in apoptosis-antigen mediated treatment of established EAE. In Figures 14a and 14b, infiltrated immune cells were isolated from the spinal cords of C57BL/6 mice, as in Figure 4a. Figure 14a shows the total number of infiltrated immune cells in the spinal cords, while Figure 14b shows the frequency of Foxp3+ CD4+ T cells in gated CD4+ T cells in the spinal cords (mean ± s.d.), which was determined by flow cytometry. n= 3-5 per group. In Figures 14c to 14g, C57BL/6 mice were immunized at day 0 and irradiated with γ-irradiation at the peak of the disease (day 14), followed by macrophage and DC (herein Mϕ) administration. In irradiated mice, animals were treated with either anti-TGFβ (αTGFβ or isotype control Ab (control Ab) at day 14-15. pMOG was administered every other day for 12 days. Mice were sacrificed at day 32. PBS (untreated control, 3 mice), IRR+Mϕ+MOG+Control Ab (3 mice), and IRR +Mϕ+MOG+αTGFα (3 mice) treatments are depicted. In Figure 14c, the upper panel depicts the experimental scheme, while the lower panel shows the mean clinical scores of EAE (mean ± s.e.m. n =3 mice). Splenocytes of the indicated groups were pooled and re-stimulated by pMOG (0-10 µg/ml)(d) or MT (50 µg/ml) (e), and the respective T cell proliferative responses were assessed by 3H-thymidine incorporation (mean ± s.d. of triplicate measurements). The same splenocytes were stimulated by pMOG (10 µg/ml) (g) or MT (50 µg/ml) (h) for 3 days, and the protein levels of IL-17, IFN-γ in the culture supernatants were measured by ELISA (mean ± s.d. of duplicate wells). *P<0.05, determined by student's t test.

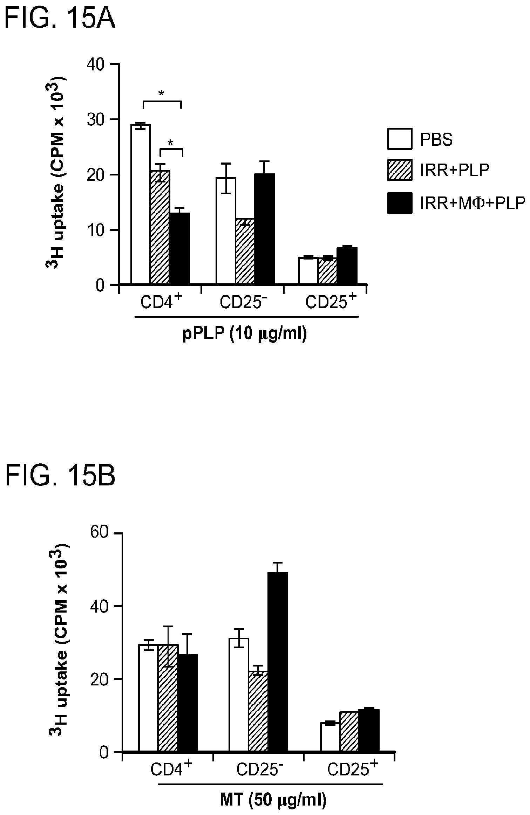

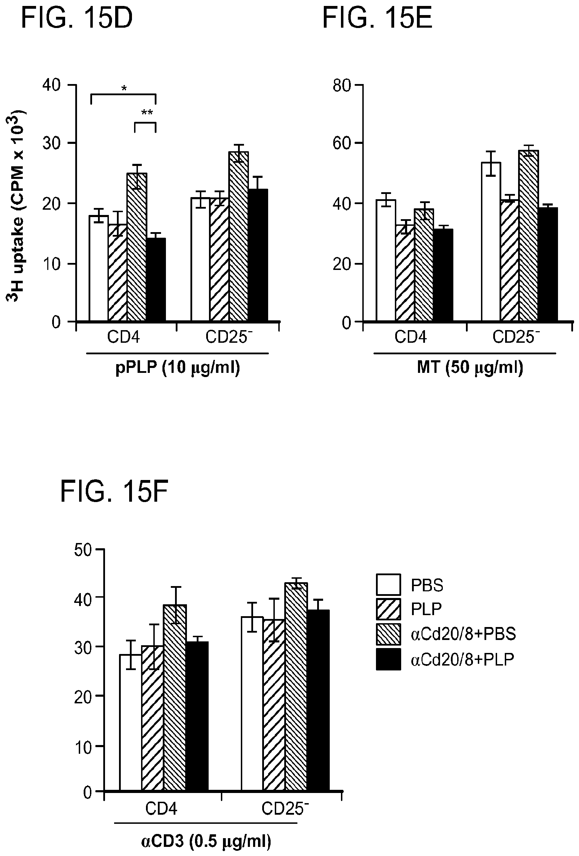

***Figures 15a to 15g*** show that generation of antigen-specific CD4+CD25+ T_{reg} cells occurred in mice with long-term remission of EAE induced by apoptosis-antigen treatment in the prevention models. Splenocytes were isolated from the SJL mice shown in Figures 10a (Figures 15a to 15c) and 13a (Figures 15d to 15f). In each experiment, splenocytes were pooled from the mice in each group and CD4+, CD4+CD25-, and CD4+CD25+ T cells were purified and cultured with irradiated APCs in the presence of either pPLP (10 µg/ml) (a,c,d,g), MT (50 µg/ml) (b,e) or anti-CD3 antibody (0.5 µg/ml) (f). The respective T cell proliferative responses were assessed by ³H-thymidine (mean ± s.d. of triplicate samples). For cytokine induction, the indicated CD4+ T cell subpopulations were cultured with pPLP (10 µg/ml) and APCs, and the protein levels of IL-17(c, g, 72 h culture), IFN-γ (c, g, 72 h culture), IL-4(c, 24 h culture), IL-6 (g, 72 h culture) and IL-9 (c, 72 h culture) in the supernatants of the indicated groups was measured by ELISA (mean ± s.d. of duplicate wells). *P<0.05, **P<0.01, determined by Student's t test. Data of a representative example selected from two independent experiments are shown.

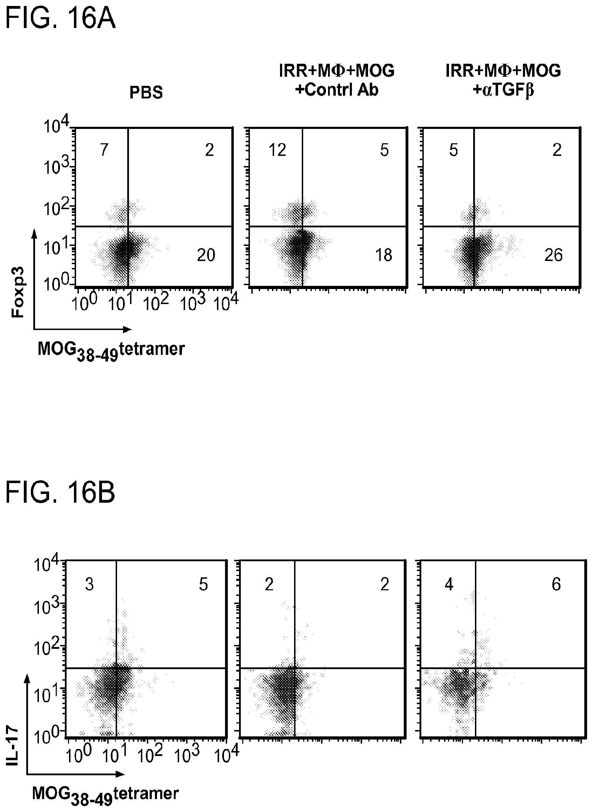

***Figures 16a to 16e*** show that TGFβ is required for generation of MOG-specific CD4+Foxp3+ T_{reg} cells in tolerized mice induced by apoptosis-antigen combination. C57BL/6 mice were treated as in Fig. 14c. In Figures 16a to 16c, tetramers recognizing MOG(38-49)-specific T cells were utilized to identify MOG-specific CD4+ T cells in the spinal cords. Flow cytometry was performed for Foxp3+ (a), IL-17+ (b), and IFN-γ+ (c) in gated CD4+ T cells in the spinal cords. The numbers indicate the tetramer-negative (upper left) and tetramer-positive (upper right) of T cells in each FACS profile. CD4+ and CD4+CD25- T cells in the spleens of mice in each group (pooled) were cultured with irradiated APCs in the presence of either pMOG (10 µg/ml)(d) or MT (50 µg/ml)(e) for 3 days. T cell proliferation was assessed by 3H-thymidine incorporation. Data are shown as mean ± s.d. of triplicate measurements. * P=0.003, determined by Student's t test.

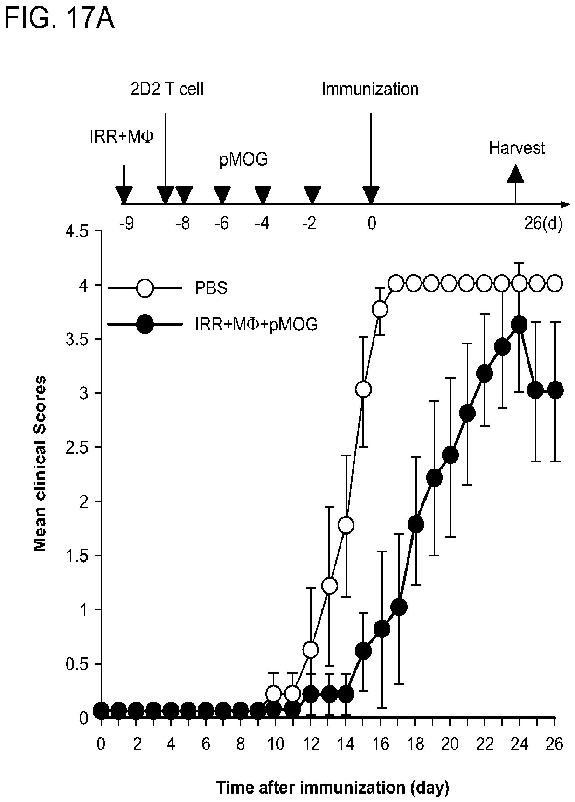

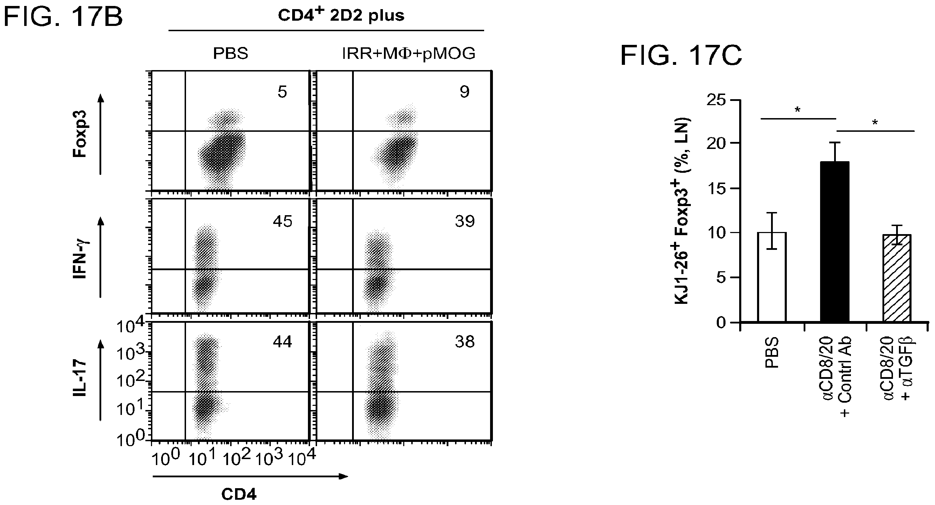

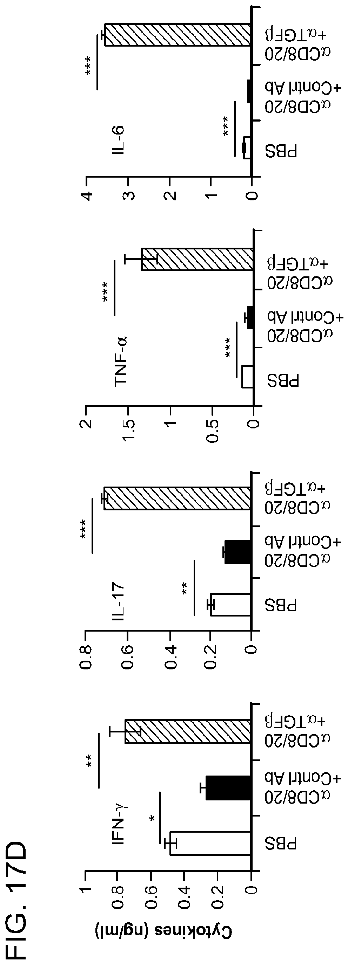

***Figures 17a to 17d*** show that antigen-specific T_{reg} cells were converted from naive CD4+ cells *in vivo.* Syngenic C57BL/6 (CD45.1) mice were either irradiated with γ-irradiation followed by macrophage and DC (Mϕ) administration or were left untreated (PBS) before immunization. Both groups were given TCR transgenic CD4+CD25Ð T cells (2D2, CD45.2+) which were specific to MOG₃₅₋₅₅ peptide antigen one day after irradiation. For irradiation-treated group, pMOG was given every other day by i.p. injection 4 times. Each group had 5 mice. The upper panel of Figure 17a depicts the experimental scheme, while the lower panel plots the mean clinical scores of EAE (mean ± s.e.m.) Figure 17b shows representative FACS data for Foxp3, IFN-γ, and IL-17 expression in 2D2 specific cells in the spinal cords of each group (pooled). Data of a representative example selected from two independent experiments are shown. Figures 17c and 17d demonstrate the conversion of OVA-specific T_{reg} cells by apoptosis-antigen treatment *in vivo.* BALB/c mice were treated with anti-CD8 and CD20 specific antibodies (αCD8/20) to deplete B cells and CD8+ T cells. The frequency of the transgenic Foxp3+KJ1-26+ CD4+ T cells in peripheral lymph nodes (c) is shown. In Figure 17d, splenocytes of each group of mice were pooled and stimulated by pOVA (5.0 µg/ml). The concentrations of IL-17, IFN-γ, TNF-α and IL-6 in the culture supernatants were measured by ELISA (mean ± s.d. of duplicate wells). Each group had 3 mice. Data are shown as mean ± s.d. in (c). *p<0.05, ** p<0.01,***p<0.001, determined by Student's t test.

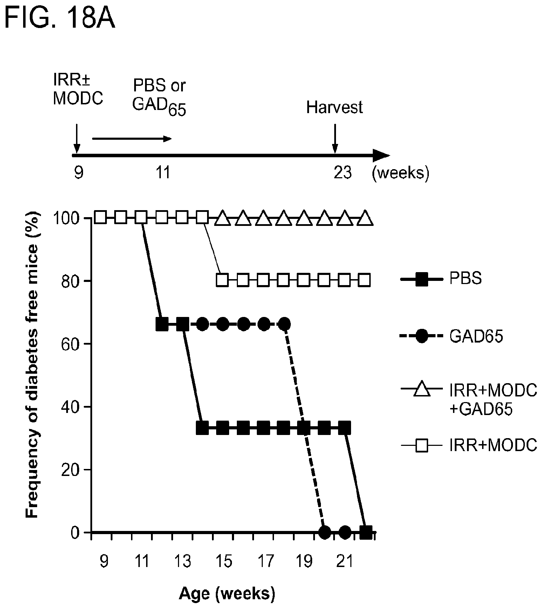

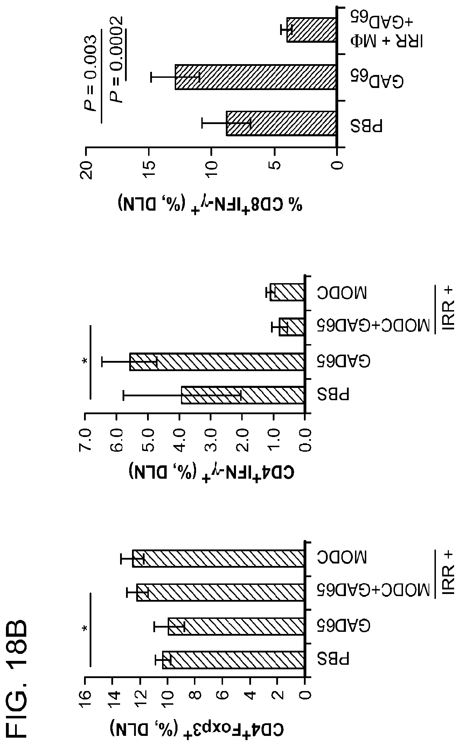

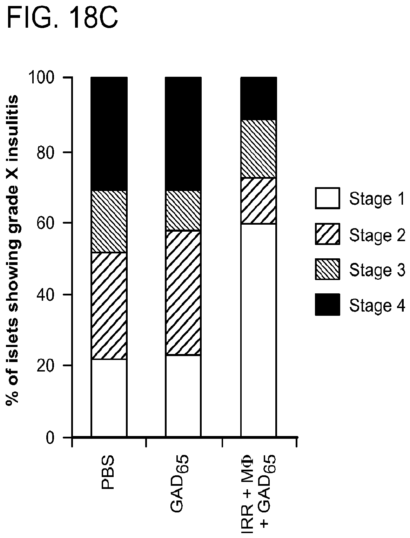

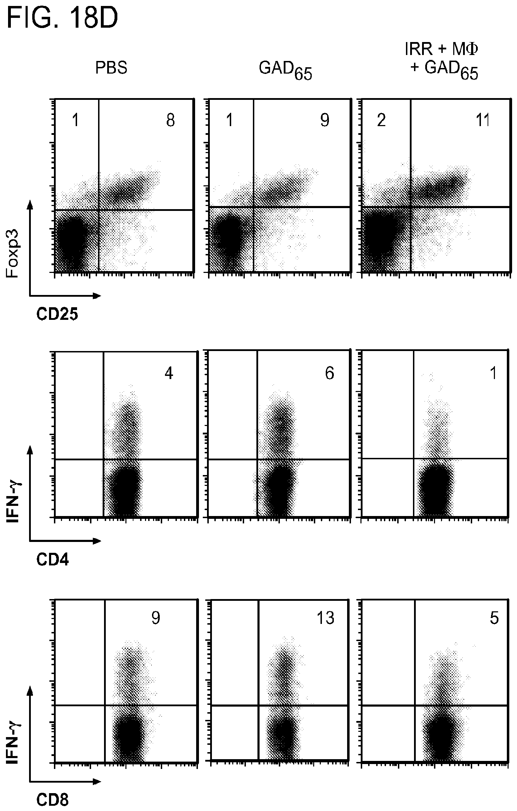

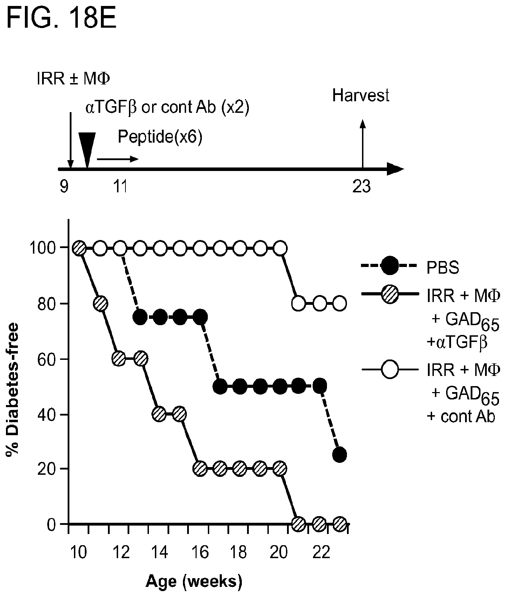

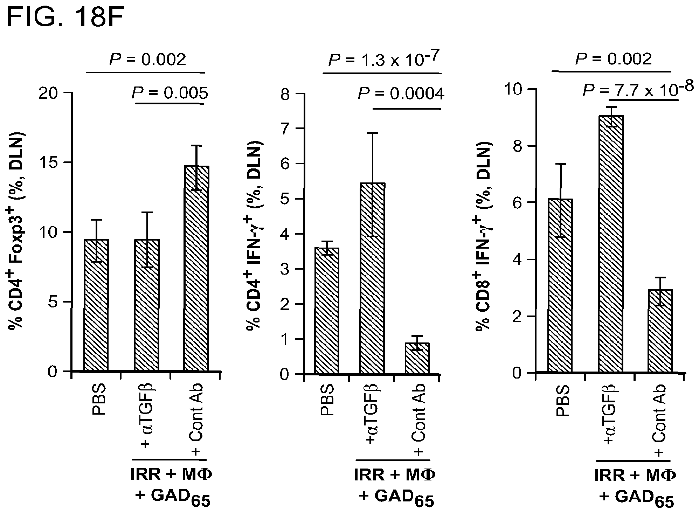

***Figures 18a to 18f*** show results for apoptosis-antigen mediated therapy of type 1 diabetes model in NOD mice. As indicated, 9 wk-old NOD mice were irradiated with γ-irradiation (IRR) with a dose of 200 rad. Some mice received normal splenic macrophages and DCs (MODC). Some mice were administered with 5µg of GAD65 peptide or PBS every other day as indicated. The upper panel of Figure 18a depicts the experimental scheme, while the lower panel plots the frequency of diabetes free mice observed For PBS (untreated control, n=3), GAD65 (GAD65 alone, n=3), IRR+MODC+GAD65 (irradiation plus MODC plus GAD65, n=5) and IRR+MODC (irradiation plus MODC, n=5) treatments. In Figure 18b, the frequency of Foxp3+ (left) and IFN-γ+ (center) cells within CD4+ T cells in the pancreas draining lymph nodes (DLN) are shown, as are the frequency of IFN-γ+ (right) cells within CD8+ T cells in the pancreas DLN, as determined by flow cytometry. Figure 18c shows the frequency of islets showing grade X insulitis in indicated groups. Figure 18d shows representative FACS data of CD4+ T cells and CD8+ T cells in the pancreatic DLN. Figure 18e depicts the experimental scheme (upper panel) and the frequency of type 1 diabetes (T1D)-free mice (lower panel). Mice were treated with either PBS (n=4) or γ-irradiation followed by intraperitoneal injection of MΦ and GAD65 in the presence of either anti-TGFβ (IRR + MΦ + GAD65 + αTGFβ, n = 5 mice) or mouse immunoglobulin G1 (IRR + MΦ + GAD65 + contrl Ab, n = 5 mice). Figure 18f shows the frequencies of Foxp3+ or IFN-γ+ CD4+ T cells in CD4+ T cells or IFN-γ+ CD8+ T cells in CD8+ T cells in mice shown in Figure 18e (mean ± SD). *P<0.05, determined by Student's t test (two-tail). * CD4+IL-17+ T cells were undetectable in pancreas DLN among all groups (not shown).

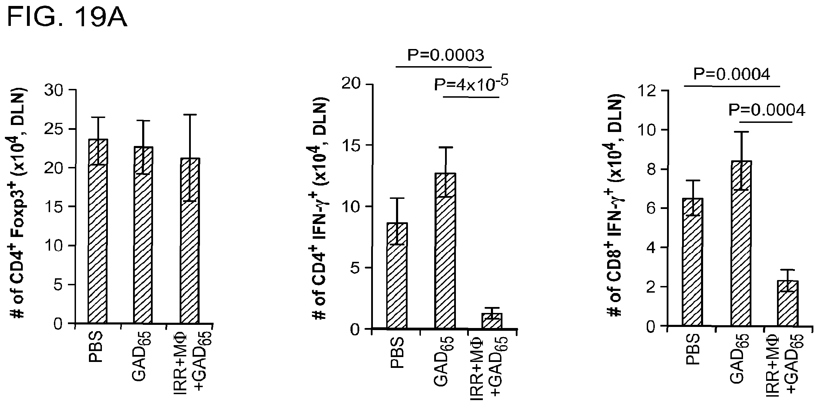

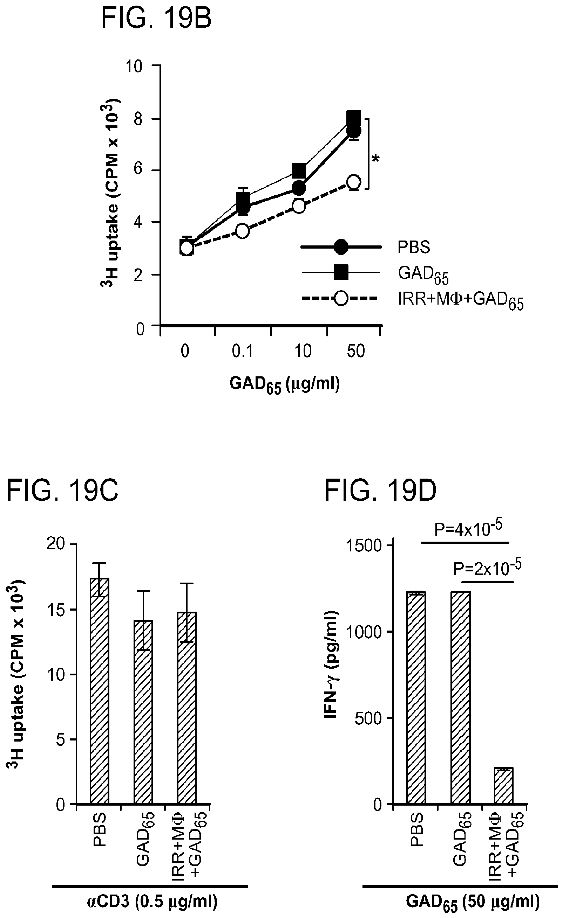

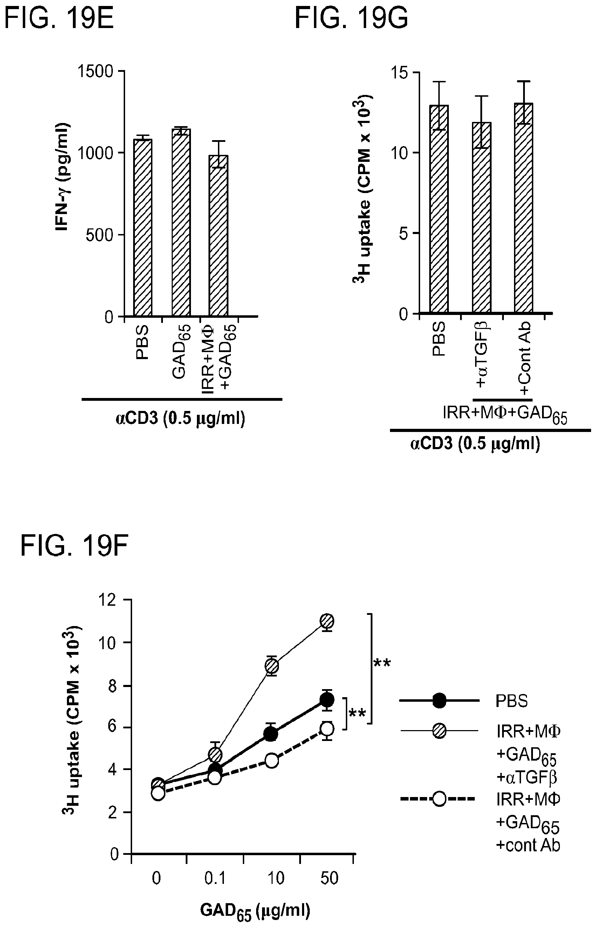

***Figures 19A to 19G*** show that NOD mice treated with irradiation plus phagocytes and GAD65 peptide (IRR+MΦ+GAD65) showed decreased GAD65 -specific T cell response compared to untreated (PBS) or GAD65-treated (GAD65) mice. In Figures 19A to 19E, cells were isolated from the spleen and DLN from the NOD mice shown in Figure 18a and PBS (untreated control, n=3 mice), GAD65 (pGAD65 administration alone, n=3 mice), or IRR+MΦ+ GAD65 (irradiation plus macrophages and iDCs transfer plus GAD65 administration, n=5 mice) treatments were administered. Figure 19A shows the total number of Foxp3+CD4+ (left), IFN-γ+CD4+ (middle), and IFN-γ+CD8+ (right) T cells in the DLN of pancreas (mean ± s.d., n=3-5 mice in each group). In Figures 19B and 19C, splenocytes of the indicated groups were pooled and re-stimulated with GAD65 (0-50 µg/ml) (B) or aCD3 (0.5 µg/ml) (C), and the respective T cell proliferative responses were assessed by 3H-thymidine incorporation (mean ± s.d. of triplicate measurements). *P=0.004 (IRR+MΦ+GAD65 vs. PBS), P=0.0008 (IRR+MΦ+GAD65 vs. GAD65), determined by student's t test. In Figures 19D and 19E, the same splenocytes were stimulated by GAD65 (50 µg/ml) (D) or aCD3 (0.5 µg/ml) (E), for 3 days, and the protein level of IFN-γ in the culture supernatants was measured by ELISA (mean ± s.d. of duplicate wells). In Figures 19F and 19G, cells were isolated from the spleen from the NOD mice shown in Figure 18e. Splenocytes of the indicated groups were pooled and re-stimulated by GAD65 (0-50 µg/ml) (F) or aCD3 (0.5 µg/ml) (G), and the respective T cell proliferative responses were assessed by 3H-thymidine incorporation (mean ± s.d. of triplicate measurements). **P=0.032 (IRR+MΦ+GAD65 v.s. PBS), P=0.0016 (IRR+MΦ+GAD65 v.s. GAD65), determined by student's t test. (A, B, D, F), Statistical analysis was determined by student's t test. (A-G), Data of a representative example selected from two independent experiments are shown.

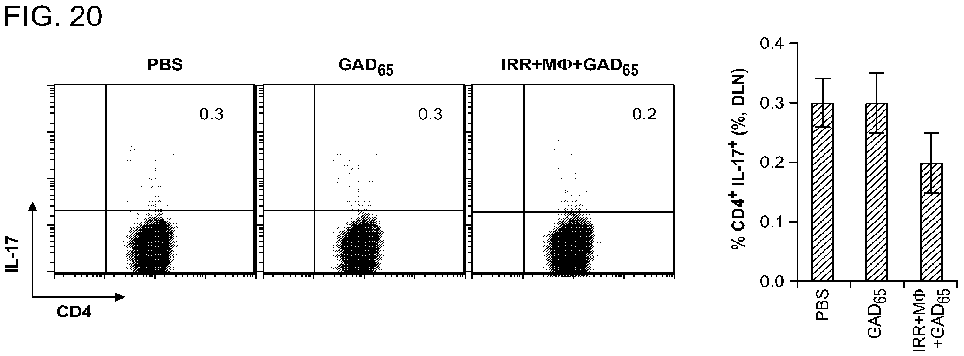

***Figure 20*** shows that Th17 cells were undetectable in NOD mice. NOD mice were either untreated (PBS) or treated with GAD65 or γ-irradiation followed by administration of macrophages and GAD65 (IRR+MΦ+GAD65). Pancreatic draining lymph node was isolated from indicated groups of mice, and the frequency of Th17 was determined by FACS. Data of a representative example selected from two independent experiments are shown.

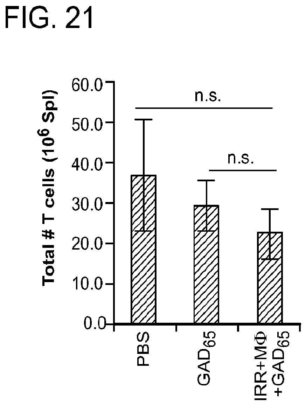

***Figure 21*** shows that the total number of T cells in the spleen was comparable between tolerized mice and untreated mice. NOD mice were either untreated (PBS) or treated with GAD65 or γ-irradiation followed by administration of macrophages and GAD65 (IRR+MΦ+GAD65). Total number of T cells in the spleen obtained from indicated groups of mice was determined by FACS. Data of a representative example selected from two independent experiments are shown.

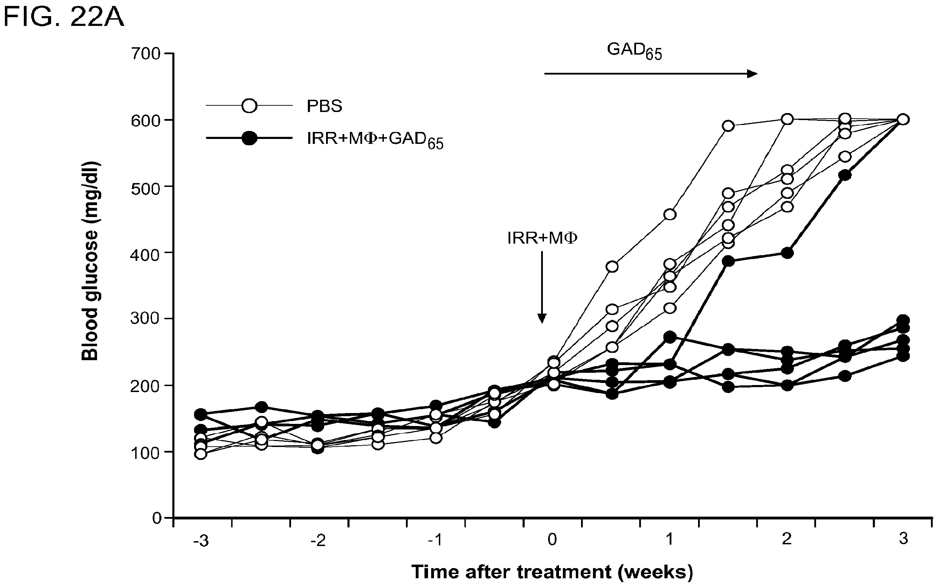

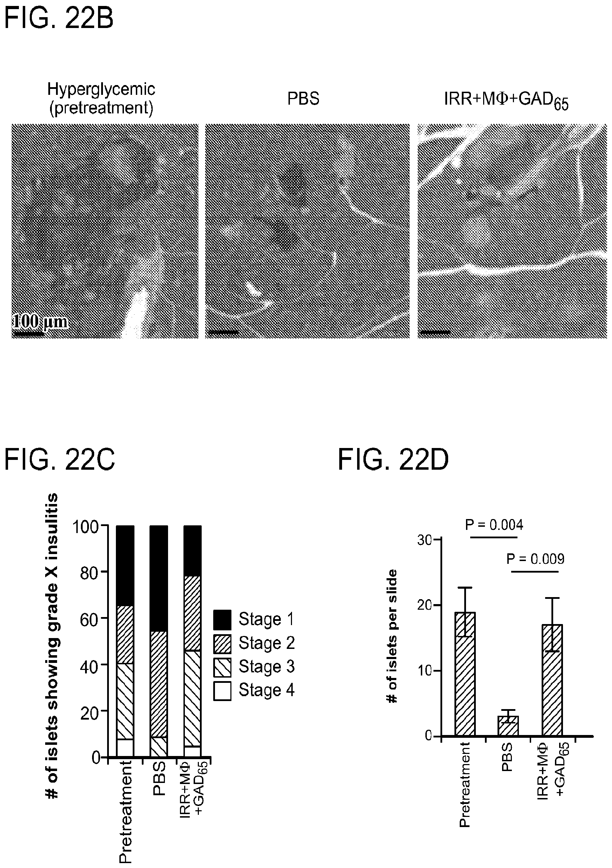

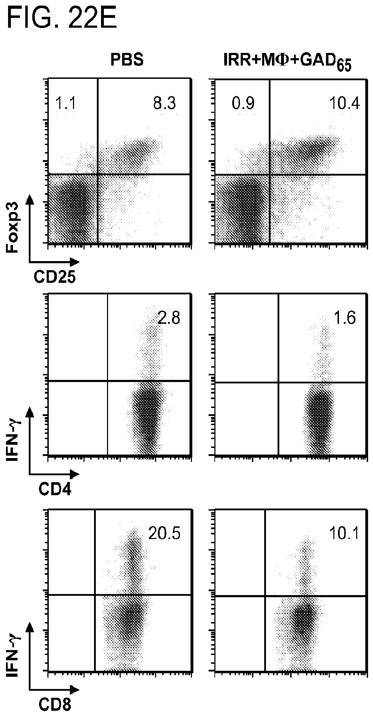

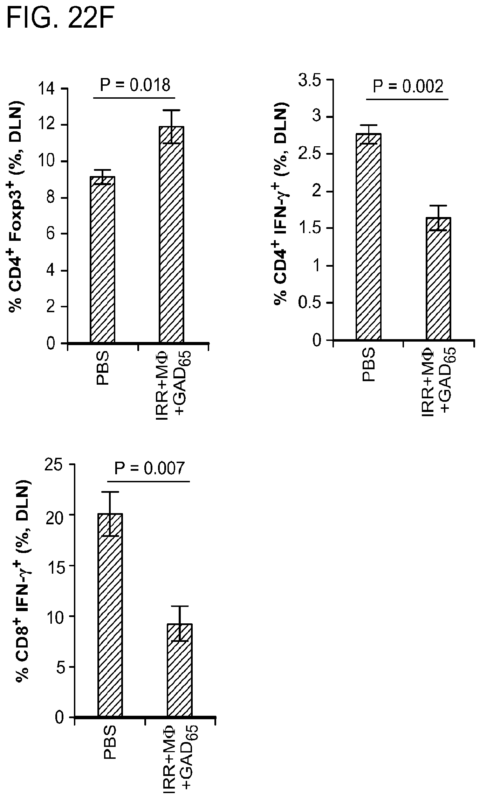

***Figures 22A to 22F*** show that systemic γ-irradiation, together with macrophages and autopeptide, suppresses T1D in recently hyperglycemic NOD mice. Recently hyperglycemic NOD mice were treated with γ-irradiation followed by administration of MΦ and GAD65 peptide as described in Fig. 18 (IRR +MΦ +GAD65, n = 6) or untreated (PBS, n = 6) for 3 weeks. Some of the hyperglycemic NOD mice were immediately sacrificed before treatment as pretreatment controls (Pretreatment, n=3). The date of initiation of the treatment was considered as day 0. Figure 22A shows levels of glucose in the blood of mice before and after treatment. Each line represents blood glucose levels in one mouse. Figure 22B shows histological analysis (hematoxylin and eosin staining) of pancreas sections obtained from representative mice from indicated groups. Figure 22C shows the frequency of islets showing grade X insulitis in the indicated groups. Figure 22D shows the number of islets in the histological pancreas section in the indicated groups (mean ± SD). Figure 22E shows flow cytometry results of gated CD4+ or CD8+ TCRβ+ T cells in the pancreatic DLNs, with frequencies of CD4+Foxp3+CD25- (top left), CD4+Foxp3+CD25+ (top right), CD4+IFN-γ+ (middle row), or CD8+IFN-γ+ (bottom row) cells assessed. In Figure 22F, frequencies of CD4+Foxp3+ (top left), CD4+IFN-γ+ (top right), or CD8+IFN-γ+ (bottom left) cells were assessed (mean ± SD, n = 6) in mice of Figure 22E. Statistical analysis was determined by Student's t test. Data of a representative example selected from two independent experiments are shown.

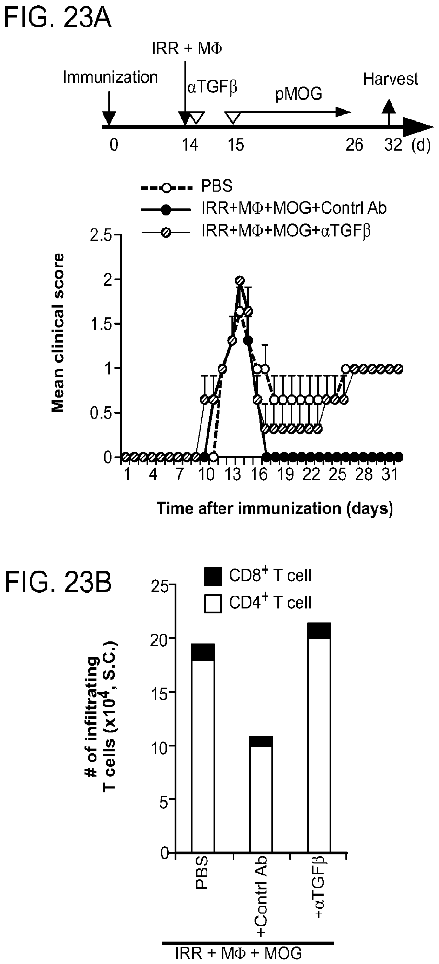

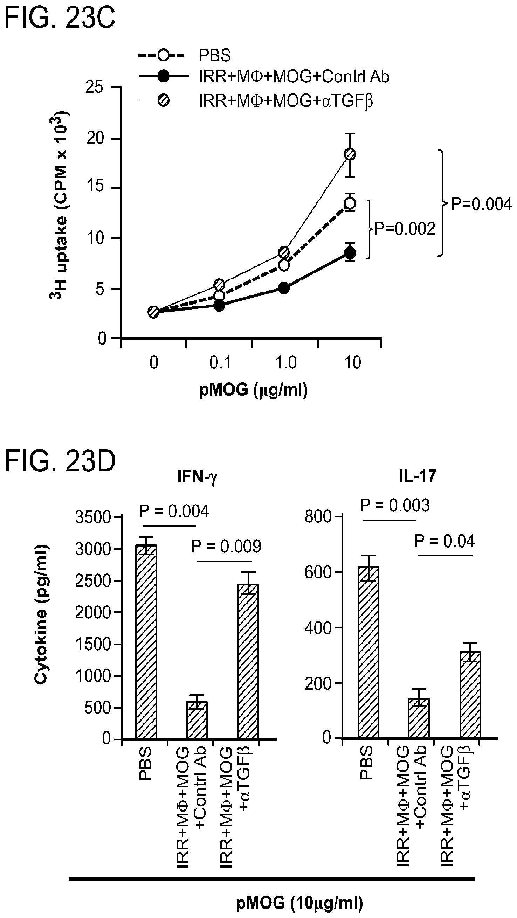

***Figures 23A to 23D*** show that TGFβ plays a key role in apoptosis-antigen combined therapy of EAE. C57BL/6 mice were immunized with pMOG at day 0 and left untreated (PBS) or irradiated with 200 rad of γ-irradiation at the peak of the disease (day 14) followed by macrophage and iDC (herein MΦ) administration. In irradiated mice, mice were treated with either anti-TGFβ (αTGFβ or isotype control Ab (contrl Ab) at day 14 and day 15. Irradiated mice were also treated with i.p. injection of pMOG every other day from day 15 to day 26. Mice were sacrificed at day 32. Mice were either left untreated (PBS, n=3 mice), or treated with γ-irradiation followed by i.p. injection of splenic macrophages and iDCs and administered with MOG peptide in the presence of either anti-TGFβ (IRR+MΦ+MOG+αTGFβ, n=3 mice) or isotype control Ab (IRR+MΦ+MOG+Contrl Ab, n=3 mice). In Figure 23A, the upper panel shows the experimental scheme, while the lower panel shows the mean clinical scores of EAE (mean ± s.e.m.). Figure 23B shows the total number of infiltrating T cells in the spinal cord, as determined by FACS. In Figure 23C, splenocytes of the indicated groups were pooled and re-stimulated by pMOG and the respective T cell proliferative responses were assessed by 3H-thymidine incorporation. (mean ± s.d. of triplicate measurements). In Figure 23D, pMOG (10 µg/ml)-specific IL-17 and IFN-γ in the cultured supernatants of the same splenocytes as in Figure 23C were measured by ELISA (mean ± s.d. of duplicate wells). Statistical analysis was determined by student's t test. Data of a representative example selected from three independent experiments are shown.

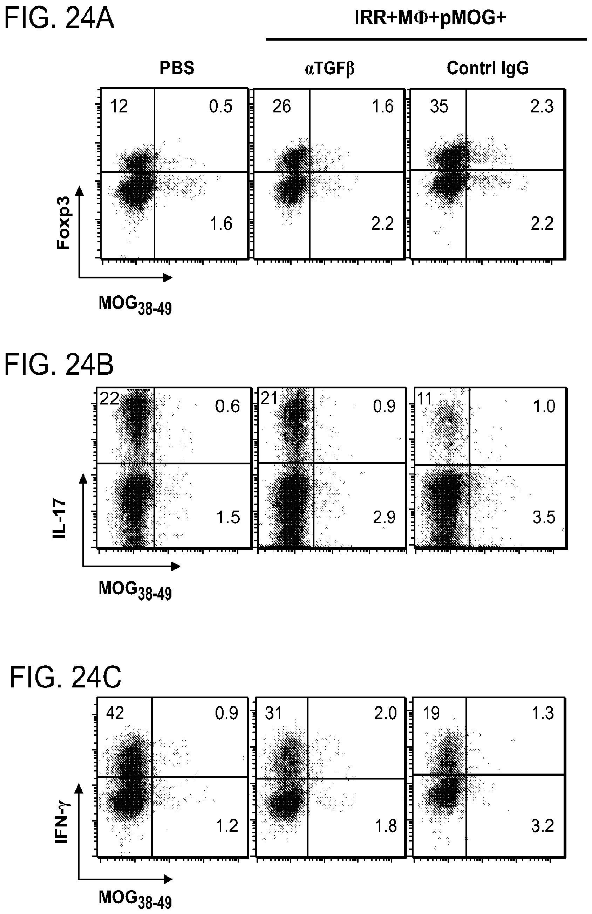

***Figures 24A to 24C*** show that MOG₃₈₋₄₉-Tetramer+ Foxp3+ Treg cells increased in the spinal cords of apoptosis-antigen treated EAE mice. C57BL/6 mice were immunized with pMOG plus CFA to develop EAE. At the peak of EAE (day 14), the mice were treated as described in Figure 23. The infiltrated leukocytes in the spinal cords were isolated at the end of experiments (day 32) and pooled for each group (3 mice per group). The cells were then stained with Tetramers recognizing MOG₃₈₋₄₉-specific T cells together with the indicated antibodies recognizing respective molecules and cytokines and analyzed with flow cytometry. The data show representative FACS profiles of Foxp3+ (A), IL-17+ (B), and IFN-γ+ (C) in gated CD4+ T cells in the spinal cords. The experiment was repeated for three times with similar results.

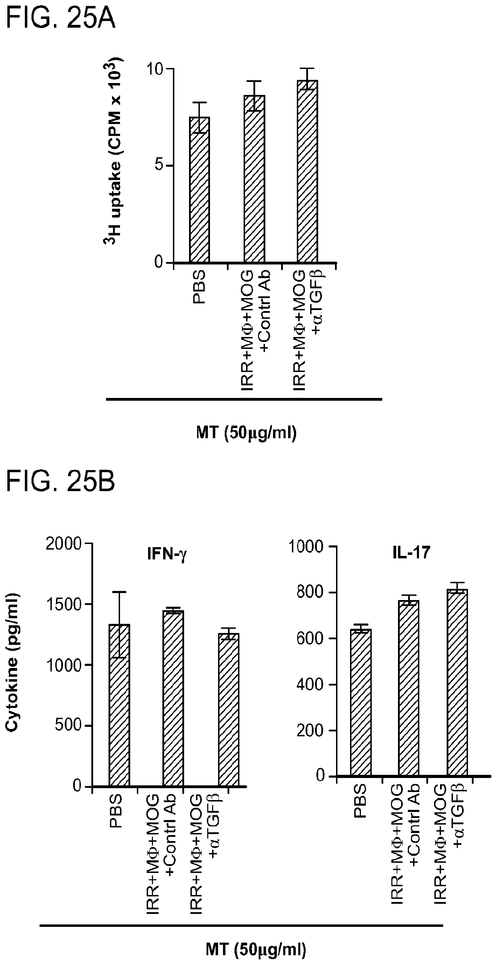

***Figures 25A and 25B*** show MT-driven T cell proliferation and IFN-γ and IL-17 production in IRR+MΦ+MOG+Contrl Ab-treated mice showed similar levels as those in untreated (PBS) mice. Cells were isolated from the spleen from the EAE mice shown in Figure 23A. Mice were sacrificed at day 32. In Figure 25A, splenocytes of the indicated groups were pooled and re-stimulated by MT (50 µg/ml), and the respective T cell proliferative responses were assessed by 3H-thymidine incorporation (mean ± s.d. of triplicate measurements, n=3 mice in each group). In Figure 25B, the same splenocytes were stimulated by MT (50 µg/ml) for 3 days, and the protein levels of IL-17, IFN-γ in the culture supernatants were measured by ELISA (mean ± s.d. of duplicate wells). Data of a representative example selected from three independent experiments are shown.

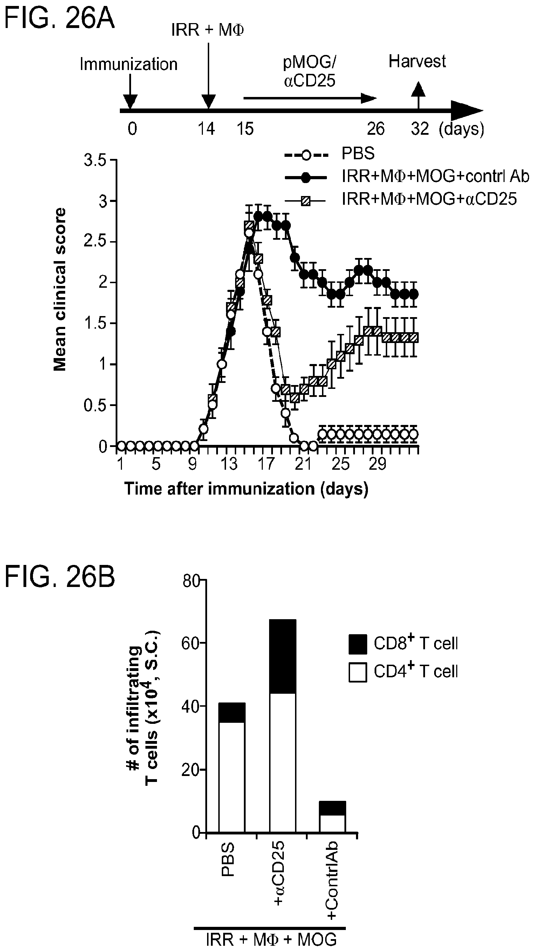

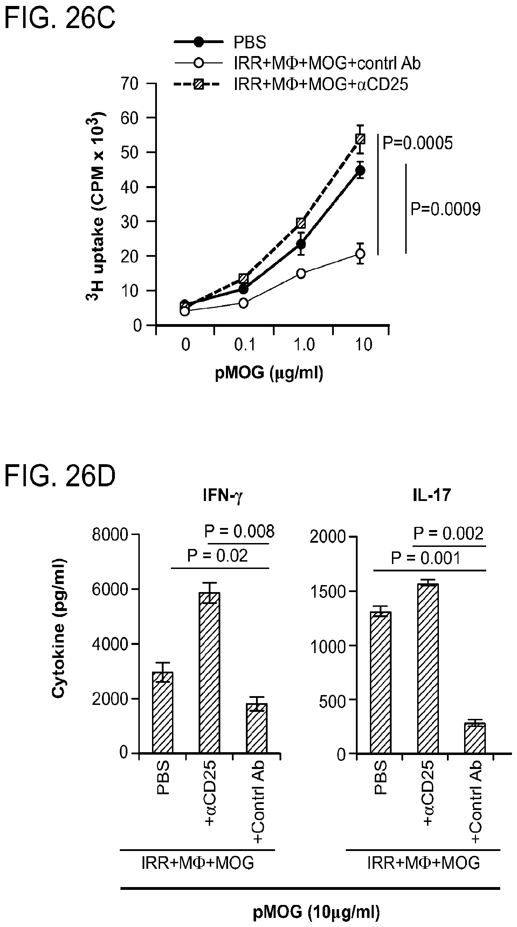

***Figures 26A to 26D*** show Treg cells play a key role in apoptosis-antigen combined therapy of EAE. C57BL/6 mice were immunized with pMOG/FCA. At day 14, the mice were either left untreated (PBS, n = 10 mice) or treated with γ-irradiation (200 rads) and normal splenic macrophage and iDC (MF) administration. In irradiated groups, mice were injected intraperitoneally with pMOG and either anti-CD25 (IRR + MΦ + MOG + aCD25, n = 10 mice) or isotype control antibody (IRR + MΦ + MOG + contrl Ab, n = 10 mice) every other day from day 15 to day 26. Mice were sacrificed at day 32. Figure 26A shows the experimental scheme and mean EAE clinical scores (mean ± SEM). Figure 26B shows the total number of infiltrated T cells detected in the spinal cords. In Figure 26C, splenocytes of each group of mice were pooled and stimulated by pMOG in *in vitro* T cell proliferative responses, which were assessed by [3H]thymidine incorporation (mean ± SD of triplicate measurements). In Figure 26D, pMOG (10 mg/ml)-specific IL-17 and IFN-γ in the cultured supernatants of the same splenocytes as in Figure 26C were measured by ELISA (mean ± SD of duplicate wells). Statistical analysis was determined by Student's t test. Data of a representative example selected from two independent experiments are shown.

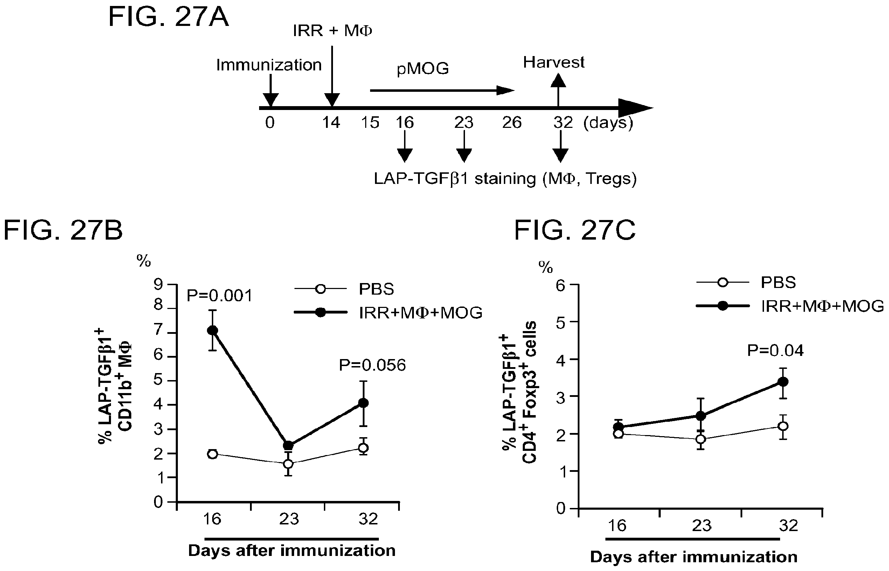

***Figures 27A and 27B*** show that cell-membrane-bound TGF-β1 of macrophages and Treg cells was increased in EAE mice treated with IRR+MΦ+MOG. C57BL/6 mice were immunized with pMOG plus CFA to develop EAE. At the peak of EAE (day 14), the mice were treated with IRR+MΦ+MOG or untreated (PBS) as described in Fig. 26A. Cells were isolated from the spleens of EAE mice at indicated dates. In Figure 27A, the frequency of cell-membrane-bound TGF-β1 (LAP-TGFβ1+ cells in CD11b+ F4/80+ MΦ was determined by flow cytometry. In Figure 27B, the frequency of LAP-TGFβ1 Foxp3+ CD4+ Treg cells in CD4+ T cells was determined by flow cytometry. Data are shown as mean ±s.d. of the values of individual mice (n= 3 mice per group at each time point) Statistical analysis was conducted by student's t test.

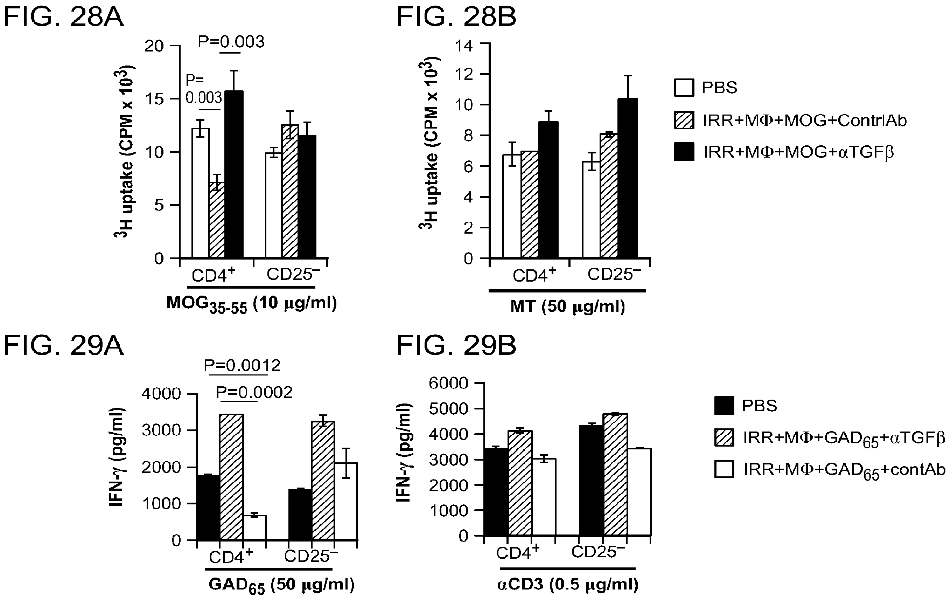

***Figures 28A and 28B*** show that TGFβ was required for the generation of antigen-specific Treg cells in established EAE. Cells were isolated from the spleen from the C57BL/6 EAE mice shown in Figure 23A. CD4+ and CD4+CD25- T cells in the spleens of mice in each group (pooled) were cultured with irradiated antigen presenting cells (APCs) obtained from untreated pMOG-immunized mice (PBS) mice in the presence of either pMOG35-55 (10 µg/ml)(A) or MT (50 µg/ml)(B) for 3 days. T cell proliferation was assessed by 3H-thymidine incorporation. Data are shown as mean ± s.d. of triplicate measurements. Statistical analysis was conducted by student's t test. Data of a representative example selected from two independent experiments are shown.
***Figures 29A and 29B*** demonstrate the generation of antigen-specific CD4+CD25+ Treg cells in mice with long-term remission of T1D induced by apoptosis-antigen treatment. Splenocytes were isolated from the NOD mice shown in Figure 18e. In each experiment, splenocytes were pooled from the mice in each group and CD4+and CD4+CD25- T cells were purified and cultured with irradiated APCs in the presence of either pGAD65 (50 µg/ml) (A) or anti-CD3 antibody (0.5 µg/ml) (B). The protein levels of IFN-γ (72 h culture) in the supernatants of the indicated groups was measured by ELISA (mean ± s.d. of duplicate wells). Statistical analysis was conducted by student's t test. Data of a representative example selected from two independent experiments are shown.

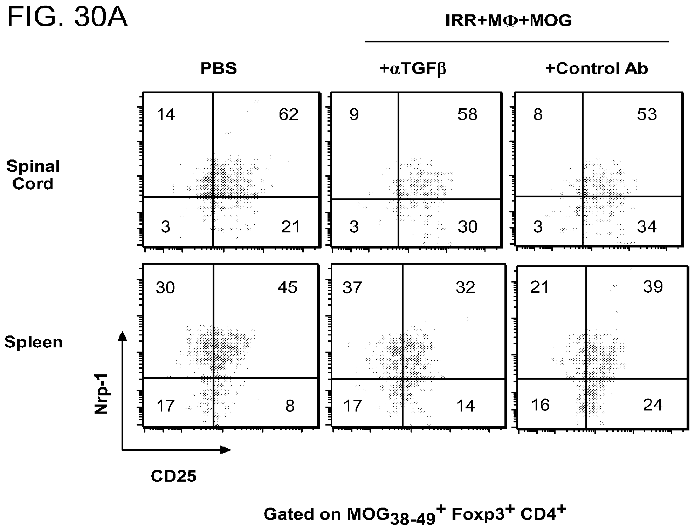

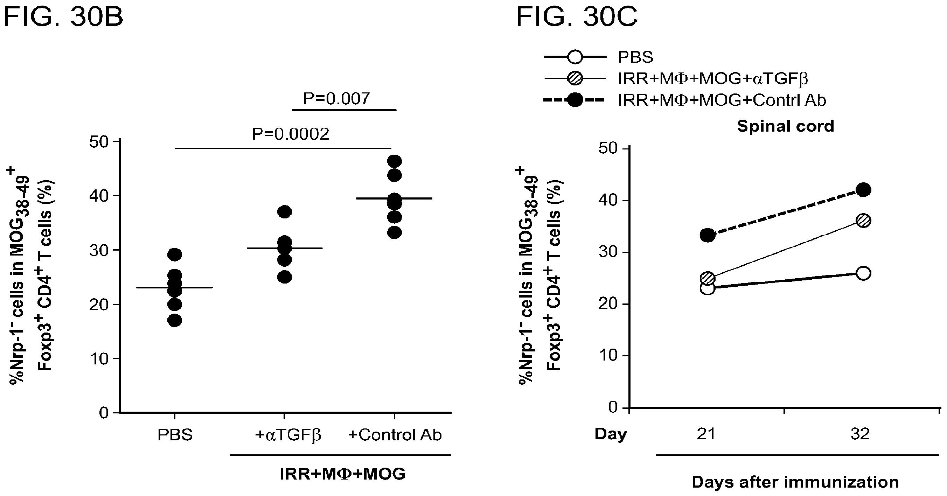

***Figures 30A to 30C*** show that Nrp-1 negative MOG38-49 + Foxp3+ T cells were increased in EAE mice after IRR+MΦ+MOG treatment. C57BL/6 mice were immunized with pMOG plus FCA to develop EAE. At the peak of EAE (day 14), the mice were either left untreated (PBS) or treated with γ-irradiation (200 rad) and normal splenic macrophage and iDC (herein MΦ) administration. In irradiated groups, mice were treated with i.p. injection of pMOG every other day from day 15 through day 26 with either anti-TGFβ (αTGFβ or isotype control Ab (contrl Ab) at day 14 and 15. Figure 30A shows flow cytometry of MOG38-49 + Foxp3+ Treg cells in the spleen and the spinal cords obtained from the mice at the end of experiments (day 32). The spinal cords were obtained from three mice in each group and pooled together before analysis. Figure 30B shows the frequency of Nrp-1 negative cells in MOG38-49 + Foxp3+ Treg cells in the spleen obtained from the mice at the end of experiments (n=6 mice per group). Statistical analysis was conducted by student's t test. Figure 30C shows the frequency of Nrp-1 negative cells in MOG38-49 + Foxp3+ Treg cells in the spinal cords of the mice at indicated days after immunization (3 mice were pooled in each group at each time point). Data of a representative example selected from three independent experiments are shown.

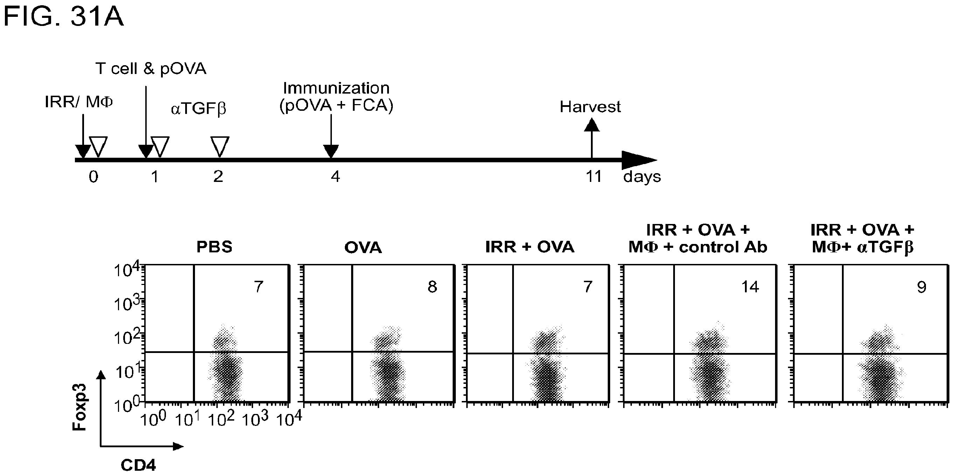

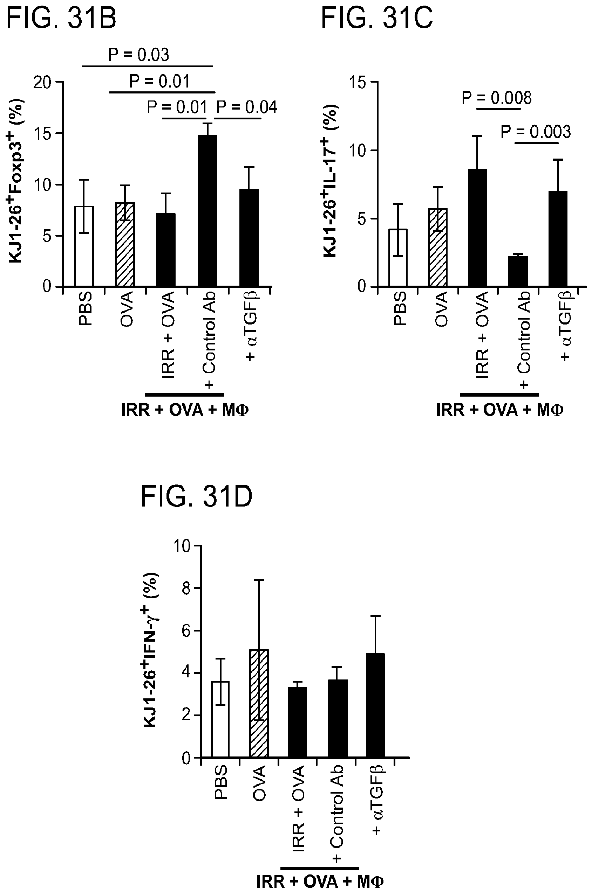

***Figures 31A to 31D*** show that antigen-specific Foxp3+ Treg cells were converted from naive CD4+ T cells by γ-irradiation-induced apoptosis-antigen therapy in vivo. BALB/c mice were either untreated (PBS, n = 3 mice) or treated with γ-irradiation (IRR) on day 0 followed by intraperitoneal injection of pOVA on day 1 (IRR + OVA, n = 3 mice) or treated with pOVA on day 1 alone without γ-irradiation (OVA, n = 3 mice). Some mice were treated with γ-irradiation followed by intraperitoneal administration of MF (1.5 million cells per mouse) on day 0 and pOVA in the presence of either anti-TGFβ (IRR + OVA + MF + αTGFβ n = 3 mice) or isotype control antibody treatment (IRR + OVA + MF + contrl Ab, n = 3 mice). All mice received intraperitoneal injection of DO11.10xRag-/- TCR-transgenic CD4+CD25- T cells (KJ1-26+) at day 1. At day 4, all mice were immunized with OVA/FCA on the food pad. At day 11, all mice were sacrificed. The Figure 31A upper panel shows the experimental scheme, while the lower panel shows a representative flow cytometry profile of splenic CD4+KJ1-26+Foxp3+ Treg cells in BALB/c recipients. Figures 31B to 31D show the frequency of Foxp3+, IL-17+, and IFN-γ+ cells in the same CD4+KJ1-26+ T cells in Figure 31A (mean ± SD, n = 3 mice per group). Statistical analysis was determined by Student's t test. Data of a representative example selected from two independent experiments are shown.

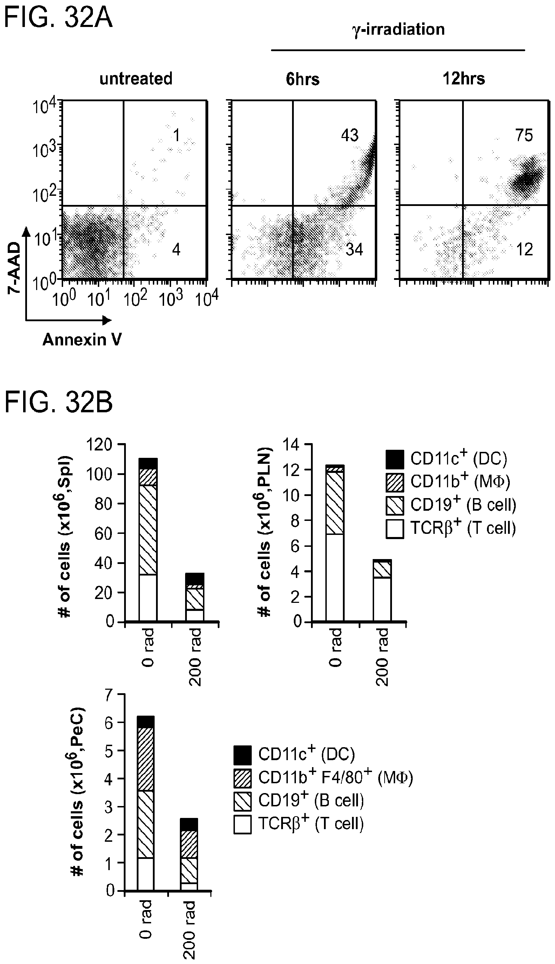

***Figures 32A and 32B*** show that γ-irradiation induced immune cell apoptosis *in vitro* and *in vivo.* In Figure 32A, thymus was harvested from C57BL/6 mice and thymocytes were irradiated (γ-irradiation) with a dose of 1000 rad. Cells were collected from either untreated mice or irradiated mice (6 and 12 hrs after γ-irradiation), and the frequency of apoptotic cells and dead cells was assessed with Annexin V and 7-AAD staining. In Figure 32B, C57BL/6 mice were irradiated with γ-irradiation with a dose of 200 rad or untreated, and cells were collected from the spleen (Spl), peripheral lymph nodes (PLN), and peritoneal cavity (PeC) 2 days after γ-irradiation. Cells from spleen and peripheral lymph nodes were treated with collagenase. Cells were stained with TCRb, CD19, CDllb, CD11c, F4/80 for further analysis. In Figure 32A, data of a single, representative example selected from two independent experiments are shown, while in Figure 32B, the data of a single, representative example selected from three independent experiments are shown.

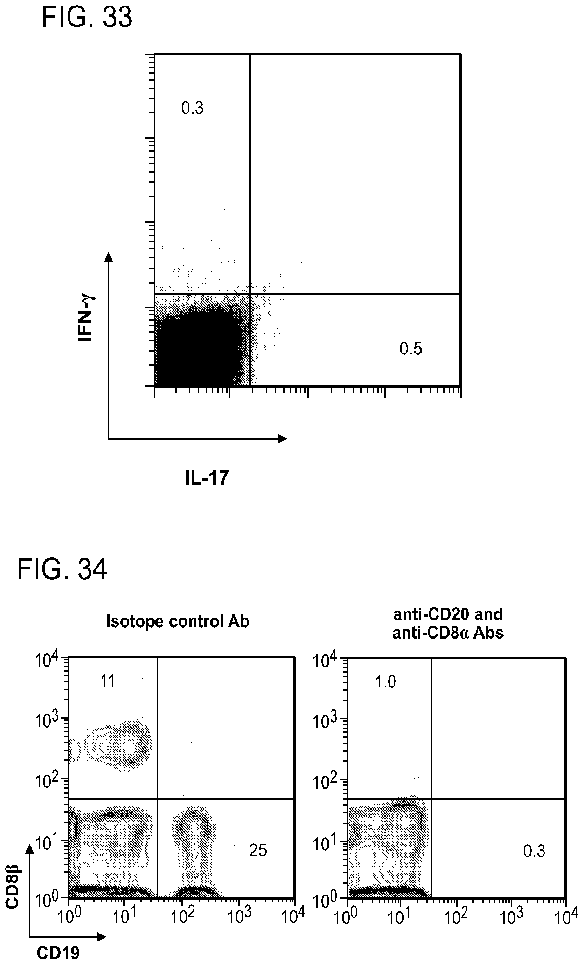

***Figure 33*** demonstrates gating control of CD4+ lymphocytes stained with isotype control antibodies of IL-17 and IFN-γ. CD4+ lymphocytes were isolated from CNS of the SJL mice shown in Fig. 3a, and stained with isotype control Abs for IL-17 (rat IgG2a) and IFN-γ (rat IgGl).
***Figure 34*** shows that *in vivo* treatment with anti-CD20 and anti-CD8a antibodies depleted B and CD8+T cells *in vivo.* C57BL/6 mice were injected i.p. with anti-CD8a (100 µg) and CD20 antibodies (Abs) (250 µg) or their isotype control antibodies and peripheral blood mononuclear cells (PBMC) were collected two days after treatment. PBMC were stained with anti-CD19 and CD8b for analysis by FACS. Data of a representative example selected from two independent experiments are shown.

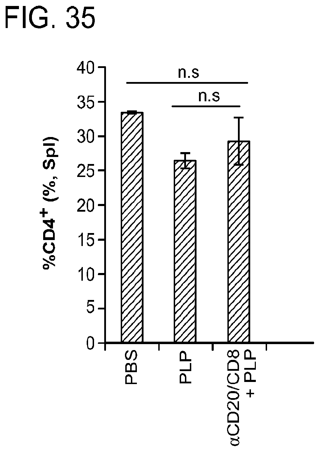

***Figure 35*** shows that *in vivo* treatment with anti-CD20 and anti-CD8a antibodies did not affect the frequency of CD4+ T cells in the whole lymphocytes. Splenocytes were isolated from the SJL mice shown in Figure 2a at the end of the experiment (day 49), and frequency of CD4+ T cells in indicated groups were determined by flow cytometry. Data of a representative group selected from two independent groups are shown.

### DETAILED DESCRIPTION OF THE INVENTION

This invention is based, at least in part, on the discovery that tolerization to antigens by T cell depletion using anti-CD4 and/ or anti-CD8 antibodies or other apoptotic cell induction methods to produce apoptosis, followed by antigen administration could be used for the tolerization of a dysfunctional immune system.

### Methods

Disclosed are methods of tolerizing a subject suffering from an autoimmune or autoinflammatory disease or disorder to an antigen associated with the autoimmune disease or disorder comprising steps a to c in order: a) identifying a subject as suffering from an autoimmune disease or disorder; b) administering an effective amount of an anti-CD4 antibody, anti-CD8 antibody, or both to the subject to induce apoptosis in T cells of the subject suffering from the autoimmune disease or disorder; and c) administering an autoantigen specific to the autoimmune disease or disorder that the subject is suffering from, whereby the subject is tolerized to the antigen of the autoimmune or autoinflammatory disease. Also disclosed are methods of treating a subject suffering from an autoimmune or autoinflammatory disease or disorder comprising steps a to c in order: a) identifying a subject as suffering from an autoimmune disease or disorder; b) administering an effective amount of an anti-CD4 antibody, anti-CD8 antibody, or both to the subject to induce apoptosis in T cells of the subject suffering from the autoimmune disease or disorder; and c) administering an autoantigen specific to the autoimmune disease or disorder that the subject is suffering from, whereby the subject is tolerized to the autoantigen, thereby treating the autoimmune or autoinflammatory disease or disorder.

It has further been discovered and is disclosed herein that step b) of the above method can optionally be substituted with or supplemented by a step of b) administering an effective amount of low-dose irradiation and macrophage to the subject suffering from the autoimmune disease or disorder to induce apoptotic cells with adoptive transfer of the macrophage or b) administering an effective amount of an anti-CD8 antibody and/or an anti-CD20 antibody to the subject to induce depletion and/or apoptosis of B cells and/or T cells of the subject suffering from the autoimmune disease or disorder. Following such alternate administering steps, an autoantigen specific to the autoimmune disease or disorder that the subject is suffering from can then be administered, with the effect of tolerizing the subject to the autoantigen, thereby treating the autoimmune or autoinflammatory disease or disorder in the subject.

In certain embodiments, step b is performed more than once prior to the performance of step c. In other further embodiments, the time for performance of step b and the time of performance of step c are separated by 1 to 21 days, more preferably 3 to 14 days.

The immune system develops tolerance to self-antigens early in life, primarily through the process of deleting self-reactive T cell clones in the thymus. This means that in order to impose tolerance in the adult to new antigens, such as those on an allograft, it is necessary either to ablate the entire immune system and attempt to recapitulate development with presentation of the new antigens in the thymus with a fresh source of haemopoietic stem cells, or to find a means to reprogramme the peripheral T cell repertoire in situ. The development of monoclonal antibodies that can deplete or modulate T cell function in vivo have made both of these routes to tolerance a practical possibility. Monoclonal antibodies that could deplete either CD4+ or CD8+ T cells in mice became available in the 1980s and were found to be able to suppress the rejection of allogeneic skin or bone marrow grafts.³⁵ While T cell depletion strategies of immunosuppression are still practically useful in clinical bone marrow³⁶ and organ transplantation to this day³⁷, it was the discovery that a brief treatment with non-depleting CD4 antibodies could induce a permanent state of antigen specific tolerance in mice³⁸ that has provided a potential route to true therapeutic reprogramming of the adult immune system.

Cluster of differentiation 4 (hereinafter, referred to as "CD4") is a glycoprotein having a molecular weight of about 55 kDa, which is expressed on the cell surface of most of thymic cells, about 2/3 of peripheral blood T cells, monocytes, and macrophage. CD4 is a type I transmembrane protein in which four immunoglobulin superfamily domains (designated in order as D1 to D4 from the N terminal to the cell membrane side) are present on the outside of the cells, and two N-linked sugar chains in total are bound to the domains D3 to D4. CD4 binds to a major histocompatibility complex (MHC) class II molecule through D1 and D2 domains, and then activates the T cells. Further, it is also known that CD4 polymerizes through D3 and D4 domains. CD4 is also known as T4, and the gene has been cloned in 1985, and the DNA sequence, the amino acid sequence and the three-dimensional structure of CD4 are publicly available from a known database. For example, these can be obtained by reference to Accession Nos. P01730 (SWISSPROT), M12807 (EMBL).

Although antibodies against CD4 were the first to be found capable of inducing tolerance to protein antigens, it has become clear that other antibody specificities are capable, either when used alone or in combinations, of reprogramming the immune system³⁹. While non-depleting CD4 antibody used alone is sufficient to achieve tolerance to long-lived protein antigens, such as foreign IgG, it was found to be essential to combine this with anti-CD8 antibodies to achieve reliable tolerance to skin grafts³⁸.

CD4- and CD8-depleting antibodies can be used to induce T cell apoptosis. It has been shown here that only with the combination of apoptosis, phagocytes, and antigen can antigen-specific T_{reg} cells be optimally generated and long-term immune tolerance developed, i.e., the proper antigenic peptide needs to be introduced in a timely manner into subjects in which an immunoregulatory milieu was created by apoptosis-triggered phagocytes.

Anti-CD3 antibodies, or fragments thereof, have been employed in the treatment of autoimmune diseases, including diabetes. For example, U.S. Patent No. 7,041,289 and published Canadian Patent Application No. 2,224,256 teach the treatment of autoimmune diseases, including diabetes, by administering an anti-CD3 antibody, or fragment thereof. However, in the present invention, use of anti-CD4 or anti-CD8 antibodies is preferable to the use of anti-CD3 antibodies.

It has previously been shown that CD3-specific antibody is able to deplete large numbers of T cells and consequently induce remission of EAE through an apoptosis-mediated mechanism¹⁴. However, CD3-specifc antibody-mediated immune tolerance has two possible unwanted side effects. One is that it can transiently yet powerfully trigger TCR on T cells to release large amounts of pro-inflammatory cytokines including IPNγ, TNFα, and IL-6 *in vivo,* which may not only interfere with the generation of T_{reg} cells, but also is a major barrier to translate the therapy into the future clinical settings. The other potential drawback of the CD3-specific antibody treatment is that the antibody engages TCR on *all* T cells indiscriminately, which could theoretically direct all T cells to differentiate into T_{reg} cells or other T cell subsets depending on the environmental cytokine milieu. This might lead to T_{reg} cells lacking antigen specificity, which would potentially render unwanted side effects to the animals and patients.

### Subject Monitoring

Steps of monitoring the subject suffering from an autoimmune disease or disorder may be included in the disclosed methods. The disclosed methods of tolerizing or treating a subject further comprise monitoring the subject for amelioration of at least one sign or symptom of an autoimmune disease.

Monitoring can be by specific diagnostic methods with quantitative measures of disease severity. Art-recognized diagnostic methods are preferably used. For example, in multiple sclerosis, the Expanded Disability Status Scale and two quantitative tests (the timed 25-foot walk test and the nine-hole peg test) can be used separately and in combination, to detect improvement. In diabetes, an oral glucose tolerance test (OGTT) can be used to monitor how well the body handles a standard amount of glucose. HbA1C (A1C or glycosylated hemoglobin test) measures average blood glucose control for the past 2 to 3 months. Diabetes is diagnosed when the A1C is 6.5% or higher. The fasting plasma glucose test (FPG) is used to determine the amount of glucose in the plasma, as measured in mg/dL. In rheumatoid arthritis, The American College of Rheumatology (ACR) Core Data Set was developed to provide a consistent group of outcome measures for RA. ACR20, 50, and 70 responses have been used. The Disease Activity Score (DAS) and its derivatives, DAS28 (a 28-joint count) and DAS-CRP (using CRP in place of ESR), are widely used. The Simplified Disease Activity Index (SDAI) and an even further simplified version (no acute phase reactant needed), the Clinical Disease Activity Index (CDAI), have also been proposed. The Global Arthritis Score (GAS) is a sum of three measures, patient pain, the raw mHAQ score, and tender joint count, and is closely correlated with both the SDAI and DAS.

### Diseases and Disorders

The present invention is useful for treating autoimmune diseases, and in particular embodiments, any autoimmune diseases with at least one known specific autoantigen. The present invention is also contemplated as useful for preventing or treating allogenic transplantation rejection *via* depletion of the immune cells of a receipient by one or more of the methods disclosed elsewhere herein, followed by administration of the allogeneic antigens from a donor that would otherwise trigger (non-self) transplantation rejection.

In both autoimmune and inflammatory diseases the condition arises through aberrant reactions of the human adaptive or innate immune systems. Autoinflammatory diseases are a relatively new category of diseases that are different from autoimmune diseases. However, autoimmune and autoinflammatory diseases share common characteristics in that both groups of disorders result from the immune system attacking the body's own tissues, and also result in increased inflammation. The term "autoimmune disease" is meant to refer to a disease state caused by an inappropriate immune response that is directed to a self-encoded entity which is known as an autoantigen. An autoimmune disease results when a host's immune response fails to distinguish foreign antigens from self-molecules (autoantigens) thereby eliciting an aberrant immune response. The immune response towards self-molecules in an autoimmune disease results in a deviation from the normal state of self-tolerance, which involves the destruction of T cells and B cells capable of reacting against autoantigens, which has been prevented by events that occur in the development of the immune system early in life. The cell surface proteins that play a central role in regulation of immune responses through their ability to bind and present processed peptides to T cells are the major histocompatibility complex (MHC) molecules (Rothbard, J. B. et al., 1991. Annu. Rev. Immunol. 9:527). Autoimmune diseases are further considered cell mediated or antibody mediated. Cell mediated autoimmune diseases arise from activities of lymphocytes such as T cells and natural killer cells, while antibody mediated diseases are caused by attack of antibodies produced by B cells and secreted into the circulatory system. Examples of cell mediated autoimmune conditions or diseases are diabetes, multiple sclerosis, and Hashimoto's thyroiditis. Examples of antibody mediated conditions or diseases are systemic lupus erythematosus and myasthenia gravis.

Exemplary autoimmune diseases that can be treated by the methods of the invention include, but are not limited to, autoimmune disease selected from the group consisting of rheumatoid arthritis, systemic lupus erythematosus, alopecia greata, anklosing spondylitis, antiphospholipid syndrome, autoimmune addison's disease, autoimmune hemolytic anemia, autoimmune hepatitis, autoimmune inner ear disease, autoimmune lymphoproliferative syndrome (alps), autoimmune thrombocytopenic purpura (ATP), Behcet's disease, bullous pemphigoid, cardiomyopathy, celiac sprue-dermatitis, chronic fatigue syndrome immune deficiency, syndrome (CFIDS), chronic inflammatory demyelinating polyneuropathy, cicatricial pemphigoid, cold agglutinin disease, Crest syndrome, Crohn's disease, Dego's disease, dermatomyositis, dermatomyositis-juvenile, discoid lupus, essential mixed cryoglobulinemia, fibromyalgia-fibromyositis, grave's disease, guillain-barre, hashimoto's thyroiditis, idiopathic pulmonary fibrosis, idiopathic thrombocytopenia purpura (ITP), Iga nephropathy, insulin dependent diabetes (Type I), juvenile arthritis, Meniere's disease, mixed connective tissue disease, multiple sclerosis, myasthenia gravis, pemphigus vulgaris, pernicious anemia, polyarteritis nodosa, polychondritis, polyglancular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, sarcoidosis, scleroderma, Sjögren'ssyndrome, stiff-man syndrome, Takayasu arteritis, temporal arteritis/giant cell arteritis, ulcerative colitis, uveitis, vasculitis, vitiligo, and Wegener's granulomatosis.

In certain embodiments, the autoimmune disease or disorder is preferably selected from the group consisting of multiple sclerosis, diabetes mellitus and rheumatoid arthritis, graft versus host diseases (GVHD) in bone marrow transplantation, organ transplantation such as kidney, liver, heart, skin, and others, in transplantation the antigen can be simply apoptotic donor leukocytes from blood; allergy/asthma, the antigen can be whatever allergen the individual is sensitive; other autoimmune diseases such as RA and systemic sclerosis.

In further embodiments, the method is useful for the treatment of an autoimmune disease that is in a later stage. Frequently, autoimmune diseases are recognized at later stages of the disease. For example, as organ-specific autoimmune diseases do not become manifest until well-advanced, interventive therapies must inhibit late-stage disease processes. While not to be limited by a particular theory, one reason for this is because the method "resets" the immune system.

In one embodiment, the autoimmune disease or disorder is Sjogren's syndrome ("SS"). Experimental Sjogren's syndrome ("ESS") can be induced in mice using an exemplary procedure set forth in, *e.g.,* Lin et al. (Ann. Rheum Dis 2014; 0: 1-9). Specifically, an ESS mouse model can be induced in 8-week-old female wildtype mice (*e.g.,* C57BL/6 mice) by introduction of salivary gland proteins as described in Lin et al. (Int Immunol 2011; 23: 613-24). For ESS induction of such mice, each mouse received subcutaneous multiinjections on the back with 0.1 mL of the emulsion on days 0 and 7, respectively. On day 14, a booster injection was carried out with a dose of 1 mg/mL salivary gland (SG) proteins emulsified in Freund's incomplete adjuvant (Sigma-Aldrich). Mice immunized with either proteins extracted from pancreas or adjuvant alone can serve as controls. Phenotypes associated with development of ESS in such mice include reduced saliva secretion, elevated serum autoantibody production and tissue destruction with lymphocytic infiltration in submandibular gland. Performance of the methods disclosed herein for treating or preventing autoimmune or autoinflammatory diseases by first breaking down the dysregulated immune system and then reprogramming the immune system to restore tolerance to the patient's self-antigens by induction of antigen specific regulatory T cells is contemplated upon both model mice such as those described above and upon subjects having or at risk of developing Sjogren's syndrome.

### Autoantigens

It has been shown herein that with the combination of apoptosis, phagocytes, and antigen can antigen-specific T_{reg} cells be optimally generated and long-term immune tolerance developed, i.e., the proper antigenic peptide needs to be introduced in a timely manner into subjects in which an immunoregulatory milieu was created by apoptosis-triggered phagocytes. In addition, the specificity of the antigenic peptide is also critical in tolerance induction.

Accordingly, certain aspects of the invention include methods and compositions concerning antigenic compositions including segments, fragments, or epitopes of polypeptides, peptides, nucleic acids, carbohydrates, lipids and other molecules that provoke or induce an antigenic response, generally referred to as antigens. As used herein, an "autoantigen" is a cellular molecule and usually is a protein. An autoantigen is typically not antigenic because the immune system is tolerized to its presence in the body under normal conditions. An autoantigen can be produced by natural cells, using recombinant methods, or through chemical synthesis, as appropriate. In particular, autoantigens, or antigenic segments or fragments of such autoantigens, which lead to the destruction of a cell via an autoimmune response, can be identified and used in the methods claimed herein.

Multiple sclerosis (MS) is an autoimmune inflammatory disease of the central nervous system (CNS) caused by lymphocyte and macrophage infiltrations into the white matter resulting in demyelination. The disease is commonly observed in young Caucasian adults with Northern European ancestry and is associated with the HLA-DR2 haplotype. Myelin basic protein (MBP) is thought to be one of the major target antigens in the pathogenesis of MS. Particularly, T cell reactivity to the immunodominant MBP 85-99 epitope is found in subjects carrying HLA-DR2, a genetic marker for susceptibility to MS. MS has been linked to the autoimmune response of T cells to myelin self-antigens presented by HLA-DR2 with which MS is genetically associated. Myelin basic protein (MBP) is a major candidate autoantigen in this disease. Its immunodominant epitope, MBP85-99, forms a complex with HLA-DR2. Copolymer 1 (Copl, Copaxone.RTM., Glatiramer Acetate, poly(Y, E, A, K) n), a random amino acid copolymer [poly (Y,E,A,K)n or YEAK] as well as two new synthetic copolymers [poly (F,Y,A,K)n or FYAK, and poly (V,W,A,K)n or VWAK] also form complexes with HLA-DR2 (DRA/DRB1*1501) and compete with MBP85-99 for binding. US 20070264229, provides MS autoantigens that can be used in the disclosed method.

The understanding of the T cell-mediated pathological process leading to MS has been advanced by the development of an animal model known as experimental autoimmune encephalomyelitis (EAE). EAE in mice mimics the inflammatory infiltrate, the neurological paralytic symptoms and demyelination observed in MS. EAE is mediated by CD4 T cells and can be induced actively by immunization with myelin antigens or their immunodominant peptides emulsified in complete Freund's adjuvant in combination with pertussis toxin injections. The myelin components myelin basic protein (MBP), proteolipid protein and myelin oligodendrocyte glycoprotein are the most studied encephalitogenic self-antigens. In certain emodiments, theself-antigen is myelin proteolipid protein (PLP).

Type 1 diabetes is an organ-specific autoimmune disease caused by chronic inflammation (insulitis), which damages the insulin producing β-cells of the pancreatic Islets of Langerhans. Dendritic cells (DCs) are generally the first cells of the immune system to process β-cell autoantigens and, by promoting autoreactivity, play a major role in the onset of insulitis. Although no cure for diabetes presently exists, the onset of insulitis can be diminished in the non-obese diabetic (NOD) mouse type 1 diabetes model by inoculation with endogenous β-cell autoantigens. These include the single peptide vaccines insulin, GAD65 (glutamic acid decarboxylase), and DiaPep277 (an immunogenic peptide from the 60-kDa heat shock protein).

Rheumatoid arthritis (RA) is a major systemic autoimmune disease. Etiology of the disease most likely involves genetic risk factors, activation of autoimmune response as well as environmental factors. The disease is systemic at all stages, characterized by inflammatory cell infiltration, synovial cell proliferation, destruction of cartilage and aberrant post-translational modifications of self-proteins that may play a role in breaking T and B cell tolerance. However, in patients with established disease, a synovial manifestation clearly dominates.

The early clinical presentation may not be specific since RA is initially indistinguishable from other forms of arthritis. So far, there is no single biomarker for the early detection of RA. The characteristic feature of this disorder is the presence of autoantibodies in the patient serum that distinguishes it from non-autoimmune joint pathogenesis like reactive arthritis or osteoarthritis (OA).

Several autoantibodies have been described in RA including antibodies against heat-shock proteins (Hsp65, Hsp90, DnaJ), immunoglobulin binding protein (BiP), heterogeneous nuclear RNPs, annexin V, calpastatin, type II collagen, glucose-6-phosphate isomerase (GPI), elongation factor human cartilage gp39 [7] and mannose binding lectin (MBL). There are some antigens such as citrullinated vimentin, type II collagen, fibrinogen and alpha enolase against which high titers of autoantibodies are specifically found in RA patients' sera. More recent discoveries include antibodies to carbamylated antigens (anti-CarP), to peptidyl arginine deiminase type 4 (PAD4), to BRAF (v raf murine sarcoma viral oncogene homologue B1) and to 14 autoantigens identified by phage display technology.

### Pharmaceutical Compositions in the Methods of the Invention

A pharmaceutically acceptable carrier includes any and all solvents, dispersion media, coatings, antimicrobials such as antibacterial and antifungal agents, isotonic and absorption delaying agents and the like that are physiologically compatible. Preferably, the carrier is suitable for intravenous, intramuscular, oral, intraperitoneal, transdermal, or subcutaneous administration, and the active compound can be coated in a material to protect it from inactivation by the action of acids or other adverse natural conditions.

The disclosure includes administering an effective amount of an anti-CD4 antibody, anti-CD8 antibody, or both to the subject and administering an autoantigen specific to the autoimmune disease or disorder that the subject is suffering from. Accordingly, the disclosed methods include an anti-CD4 antibody, anti-CD8 antibody, or both, and an autoantigen, as provided herein into a pharmaceutical composition suitable for administration to a subject. A composition of the present invention can be administered by a variety of methods known in the art as will be appreciated by the skilled artisan. The active compound can be prepared with carriers that will protect it against rapid release, such as a controlled release formulation, including implants, transdermal patches, and microencapsulated delivery systems. Many methods for the preparation of such formulations are patented and are generally known to those skilled in the art. See, e.g., Sustained and Controlled Release Drug Delivery Systems, J. R. Robinson, Ed., Marcel Dekker, Inc., NY, 1978. Therapeutic compositions for delivery in a pharmaceutically acceptable carrier are sterile, and are preferably stable under the conditions of manufacture and storage. The composition can be formulated as a solution, microemulsion, liposome, or other ordered structure suitable to high drug concentration.

Dosage regimens can be adjusted to provide the optimum desired response (e.g., tolerizing a subject and/or a therapeutic response). For example, a single bolus or oral dose can be administered, several divided doses can be administered over time, or the dose can be proportionally reduced or increased as indicated by the exigencies of the disease situation.

A physician or veterinarian having ordinary skill in the art can readily determine and prescribe the effective dose of the pharmaceutical composition required. For example, the physician or veterinarian could start doses of the compound of the invention employed in the pharmaceutical composition at a level lower than that required in order to achieve the desired therapeutic effect, and increase the dosage with time until the desired effect is achieved.

In another embodiment, the pharmaceutical composition may also include also an additional therapeutic agent, i.e. in combination with an additional agent or agents. Examples of materials that can be used as combination therapeutics for treatment of autoimmune disease as additional therapeutic agents include: an antibody or an antibody fragment that can bind specifically to an inflammatory molecule or an unwanted cytokine such as interleukin-6, interleukin-8, granulocyte macrophage colony stimulating factor, and tumor necrosis factor-.alpha.; an enzyme inhibitor which can be a protein, such as alpha₁-antitrypsin, or aprotinin; an enzyme inhibitor which can be a cyclooxygenase inhibitor; an engineered binding protein, for example, an engineered protein that is a protease inhibitor such an engineered inhibitor of a kallikrein; an antibacterial agent, which can be an antibiotic such as amoxicillin, rifampicin, erythromycin; an antiviral agent, which can be a low molecular weight chemical, such as acyclovir; a steroid, for example a corticosteroid, or a sex steroid such as progesterone; a non-steroidal anti-inflammatory agent such as aspirin, ibuprofen, or acetaminophen; an anticancer agent such as methotrexate, cis-platin, 5-fluorouracil, or adriamycin; a cytokine blocking agent; an adhesion molecule blocking agent; or a cytokine.

An additional therapeutic agent can be a cytokine, which as used herein includes without limitation agents which are naturally occurring proteins or variants and which function as growth factors, lymphokines, interferons particularly interferon-beta, tumor necrosis factors, angiogenic or antiangiogenic factors, erythropoietins, thrombopoietins, interleukins, maturation factors, chemotactic proteins, or the like.

An improvement in the symptoms as a result of such administration is noted by a decrease in frequency of recurrences of episodes of the autoimmune condition such as MS, by decrease in severity of symptoms, and by elimination of recurrent episodes for a period of time after the start of administration. Quantitative measures of disease severity are disclosed herein. A therapeutically effective dosage preferably reduces symptoms and frequency of recurrences by at least about 20%, for example, by at least about 40%, by at least about 60%, and by at least about 80%, or by about 100% elimination of one or more symptoms, or elimination of recurrences of the autoimmune disease, relative to untreated subjects. The period of time can be at least about one month, at least about six months, or at least about one year.

The invention also contemplates administration of an additional agent.

Exemplary agents include, but are not limited to non-steroidal anti-inflammatory drugs (NSAIDs), such as Aspirin, Choline and magnesium salicylates, Choline salicylate, Celecoxib, Diclofenac potassium, Diclofenac sodium, Diclofenac sodium with misoprostol, Etodolac , Fenoprofen calcium, Flurbiprofen, Ibuprofen, Indomethacin, Ketoprofen, Magnesium salicylate, Meclofenamate sodium, Mefenamic acid, Meloxicam, Nabumetone, Naproxen, Naproxen sodium, Oxaprozin, Piroxicam, Rofecoxib, Salsalate, Sodium salicylate, Sulindac Tolmetin sodium, Valdecoxib.

Other exemplary agents include disease-modifying antirheumatic drugs (DMARDs), for example, but not limited to, abatacept, adalimumab, azathioprine, chloroquine and hydroxychloroquine (antimalarials), ciclosporin (Cyclosporin A), D-penicillamine, etanercept, golimumab, gold salts (sodium aurothiomalate, auranofin), infliximab, leflunomide, methotrexate (MTX), rituximab, sulfasalazine (SSZ).

Other exemplary agents include metformin, glipizide, glyburide, glimepiride, acarbose, pioglitazone, Sitagliptin, Saxagliptin, Repaglinide, Nateglinide, Exenatide, Liraglutide.

Other exemplary agents include corticosteroids, beta-interferons, glatiramer acetate, fingolimod, natalizumab, mitoxantrone, teriflunomide.

### Kits

Also provided are kits. Kits according to the present invention can contain pharmaceutical compositions for use in the methods of the Invention. In preferred embodiments, the kits contain all of the components necessary to perform the disclosed methods, including directions for performing the methods, and any necessary software for analysis and presentation of results.

### In Vivo-Generated Antigen Specific Regulatory T Cells Were Identified to Treat Autoimmunity Without Compromising Antibacterial Immune Response

The instant application describes development of a process/pathway for generating autoantigen-specific Treg cells in vivo, which showed therapeutic effects on experimental autoimmune encephalomyelitis and nonobese diabetes in mice, and is applicable to autoimmune disease more generally. Specifically, apoptosis of immune cells was induced by systemic sublethal irradiation or depleted B and CD8+ T cells with specific antibodies and then autoantigenic peptides were administered to mice possessing established autoimmune diseases. It was mechanistically demonstrated that apoptotic cells triggered professional phagocytes (e.g., neutrophils, monocytes, macrophages, dendritic cells, and mast cells, having receptors on their surfaces which can detect harmful objects, such as bacteria) to produce transforming growth factor-β, under which the autoantigenic peptides directed naïve CD4+ T cells to differentiate into Foxp3+ Treg cells, instead of into T effector cells, in vivo. These antigen-specific Treg cells specifically ameliorated autoimmunity without compromising immune responses to bacterial antigen. Thus, antigen-specific Treg cells with therapeutic activity toward autoimmunity were successfully generated. The present findings can be broadly applied to development of antigen-specific Treg cell-mediated immunotherapy for multiple sclerosis and type 1 diabetes and also other autoimmune diseases.

An antigen-specific therapy for autoimmune disease that does not compromise the overall immune response is the ultimate goal for medical researchers studying treatment of autoimmune disesease. The current application provides a process/pathway to generate autoantigen-specific Treg cells *in vivo* in mouse autoimmunity models in which mice exhibit disease before therapeutic intervention. Herein it was identified that apoptosis-antigen therapy could suppress autoimmune T cell responses to the target tissues, without compromising the overall immune response. The dysregulated immune system was reprogrammed and, importantly, the disease was controlled. This apoptosis-antigen-mediated immune tolerance occurred in both TH17-mediated EAE and TH1-mediated T1D. Hence, apoptosis-antigen therapy represents a new therapeutic approach that could be used in the treatment of autoimmune diseases.

Although it has been discovered that the apoptosis-antigen treatment described herein induces autoantigen-specific Treg cells, it remains possible that the already present autoantigen-specific tTreg cells may also participate in suppressing autoimmune responses in the tolerized mice. Nonetheless, adaptation of this therapy to human patients is contemplated, with dose, time, and length of the specific self-peptide injection being important factors for evaluation in clinical application to patients.

Thus, a process/pathway for reprogramming the dysregulated immune system to promote tolerance in EAE and T1D has been discovered and described herein. It is contemplated that the apoptotic induction approach described herein can be performed upon patients with autoimmunity disease or disorder. For example, anti-CD20 antibody (rituximab) has been used in patients with autoimmune diseases, and it is contemplated that such therapy can be combined with the antibody-mediated depletion (apoptosis) of B cells together with administration of known autoantigenic peptide(s) to achieve additional and better therapeutic effects for patients. Similarly, one-time low/middle dose of irradiation with the aim to induce sufficient number of apoptotic cells with adoptive transfer of autologous macrophages can also be used to induce apoptosis in patients. Total body irradiation followed by hematopoietic stem cell transplantation has been conducted previously in patients with severe autoimmune disease (Nash et al. Blood 102: 2364-2372); therefore, low-dose irradiation together with macrophage and autoantigenic peptide administration is contemplated as providing therapeutic benefits for these patients. Nonetheless, this discovery relies on the induction of autoantigen-specific Treg cells that functionally suppress autoimmunity in the target tissues without compromising the overall immune response in the host. Thus, the currently identified protocol can be applied to other types of autoimmune disease, provided that one or more self-peptides are identified.

### EXAMPLES

It should be appreciated that the invention should not be construed to be limited to the examples that are now described; rather, the invention should be construed to include any and all applications provided herein and all equivalent variations within the skill of the ordinary artisan.

### Example 1: Therapy of experimental autoimmune encephalomyelitis by inducing antigen-specific regulatory T cells in vivo

Described herein is an immunotherapy on experimental autoimmune encephalomyelitis (EAE) in mice by generating autoantigen-specific T_{reg} cells *in vivo.* Mechanistically, this was accomplished by first inducing apoptotic immune cells that trigger professional phagocytes (*e.g.*, neutrophils, monocytes, macrophages, dendritic cells, and mast cells, having receptors on their surfaces which can detect harmful objects, such as bacteria) to produce TGFβ, and then administering auto- antigenic peptides, which directed naive CD4+ T cells to differentiate into Foxp3+ T_{reg} cells instead of into T effector cells in vivo. Importantly, these antigen-specific T_{reg} cells suppressed T cell response to the autoantigens, but not to bacterial antigen. Thus, antigen-specific T_{reg} cells with therapeutic activity toward EAE have been successfully generated. These findings have clinical implications for the development of therapy for various autoimmune diseases, including multiple sclerosis and diabetes.

The present invention describes, in part, the development of a novel pathway to induce antigen-specific T_{reg} cells in vivo that have therapeutic effects on mice with EAE. The principle of the experimental design is to first "break down" the dysregulated, autoimmune immune system, and then "reprogram" it to be immune-tolerant to self-antigens. This specific immune tolerance was accomplished by a combination of immune cell apoptosis followed by specific antigenic-peptide administration (herein apoptosis-antigen) in mice, which induced antigen-specific T_{reg} cells (Figure 7). The generated antigen-specific T_{reg} cells selectively suppressed T effector cells responsive to auto-antigens, but not to bacterial antigens, and showed no compromise of overall T cell immune responses.

### T cell apoptosis and peptide administration leads to long-term suppression of EAE

First, the hypothesis was tested of apoptosis-antigen combination to induce tolerance in a model of relapsing-remitting EAE in proteolipid protein peptide PLP139-151(pPLP)-susceptible SJL mice^{10,11}. CD4- and CD8-depleting antibodies were used (herein α CD4/CD8) to induce T cell apoptosis^{12,13}. Indeed, the antibody treatment depleted 90% of CD4+ and 50% of CD8+ T cells, which were recovered in about 3 weeks (data not shown).

In both autoimmune and inflammatory diseases the condition arises through aberrant reactions of the human adaptive or innate immune systems. Autoinflammatory diseases are a relatively new category of diseases that are different from autoimmune diseases. However, autoimmune and autoinflammatory diseases share common characteristics in that both groups of disorders result from the immune system attacking the body's own tissues, and also result in increased inflammation. The term "autoimmune disease" is meant to refer to a disease state caused by an inappropriate immune response that is directed to a self-encoded entity which is known as an autoantigen. An autoimmune disease results when a host's immune response fails to distinguish foreign antigens from self-molecules (autoantigens) thereby eliciting an aberrant immune response. The immune response towards self-molecules in an autoimmune disease results in a deviation from the normal state of self-tolerance, which involves the destruction of T cells and B cells capable of reacting against autoantigens, which has been prevented by events that occur in the development of the immune system early in life. The cell surface proteins that play a central role in regulation of immune responses through their ability to bind and present processed peptides to T cells are the major histocompatibility complex (MHC) molecules (Rothbard, J. B. et al., 1991. Annu. Rev. Immunol. 9:527). Autoimmune diseases are further considered cell mediated or antibody mediated. Cell mediated autoimmune diseases arise from activities of lymphocytes such as T cells and natural killer cells, while antibody mediated diseases are caused by attack of antibodies produced by B cells and secreted into the circulatory system. Examples of cell mediated autoimmune conditions or diseases are diabetes, multiple sclerosis, and Hashimoto's thyroiditis. Examples of antibody mediated conditions or diseases are systemic lupus erythematosus and myasthenia gravis.

Exemplary autoimmune diseases that can be treated by the disclosed methods include, but are not limited to, autoimmune disease selected from the group consisting of rheumatoid arthritis, systemic lupus erythematosus, alopecia greata, anklosing spondylitis, antiphospholipid syndrome, autoimmune addison's disease, autoimmune hemolytic anemia, autoimmune hepatitis, autoimmune inner ear disease, autoimmune lymphoproliferative syndrome (alps), autoimmune thrombocytopenic purpura (ATP), Behcet's disease, bullous pemphigoid, cardiomyopathy, celiac sprue-dermatitis, chronic fatigue syndrome immune deficiency, syndrome (CFIDS), chronic inflammatory demyelinating polyneuropathy, cicatricial pemphigoid, cold agglutinin disease, Crest syndrome, Crohn's disease, Dego's disease, dermatomyositis, dermatomyositis-juvenile, discoid lupus, essential mixed

### Combination of B cell depletion and peptide administration suppresses EAE

To exclude the possibility that the tolerance effects seen in the aforementioned therapy of EAE were due to the signaling and/or depletion of CD4+ effector T cells (and to further validate that the tolerance effects in the therapy of EAE, as described elsewhere herein, were triggered by cell apoptosis and mediated by phagocytes), B cells and CD8+ T cells were depleted with respective antibodies followed by pPLP injection in SJL mice with established EAE (Fig. 2a). Single injection of CD20- and CD8-specific antibodies (herein α CD20/CD8) eliminated more than 90% of B cells and 50% of CD8+ T cells without affecting the frequency of CD4+ T cells (Figures 34 and 35). It was found that αCD20/CD8 plus pPLP administration suppressed the severity and prevented relapses of chronic EAE in SJL mice (Fig. 2a). αCD20/CD8 treatment alone also resulted in therapeutic effects on the chronic EAE (Fig. 2a), which was again in contrast to the failure of suppressing EAE and accompanying pPLP-specific T cell proliferation and inflammatory cytokine production in the spleen in the prevention model using the same regimen (αCD20/CD8+PBS, Figure 10; specifically, aCD20/CD8 plus pPLP administration into naive mice before EAE was induced significantly suppressed EAE (Figure 10a), which was accompanied by down-regulation of antigen-specific inflammatory T cell responses and increased Treg cells (Fig 10d, e, h)). Consistent with reduction of the disease, the total number of infiltrating T cells in the spinal cords was decreased in the tolerized mice (Fig. 2f). Significantly, the frequency of Th17 cells was dramatically decreased, but Foxp3+ T_{reg} cells were increased in the spinal cords of the tolerized mice (αCD20/CD8 alone or plus pPLP) compared to untreated mice (Fig. 2b). In the spleen, tolerized mice showed increased frequencies of T_{reg} cells, but no changes of CD4+ T cells in whole lymphocytes (Fig. 2g). Additionally, the pPLP-driven T cell proliferation and inflammatory cytokine production in the cultures were specifically suppressed in these tolerized mice (Fig. 2c-e), whereas the MT-specific T cell responses were not affected (Fig. 2d). These data together provided further evidence that the suppression of EAE by immune cell depletion together with autoantigen peptide treatment was attributable to apoptotic cell-triggered- and antigen-instructive tolerance.

### A critical function of phagocytes in apoptosis-antigen-mediated suppression of EAE

Next, the underlying mechanisms responsible for the long-term EAE remission were examined. It was hypothesized that professional phagocytes (in certain embodiements, particularly macrophages and immature DCs), by sensing and digesting apoptotic cells^{15,16} played essential roles in the tolerance induction presented here (Figure 7). First, phagocytes were pre-depleted with clodronade-loaded liposomes before αCD4/CD8 and pPLP administration in SJL mice with established EAE. The data shows that elimination of phagocytes reversed apoptosis-antigen-induced suppression of EAE (Figure 11). Next, sublethal whole body γ-irradiation was used to eliminate immune cells, followed by injection of peptides plus normal syngeneic phagocytes. This approach would not only directly validate the crucial function of phagocytes, but also completely exclude the possibility that the immune tolerance seen in the antibody-treatment experiments was due to signaling by CD4, CD8, or CD20 molecules triggered by the antibodies. In contrast to specific antibody injection, γ-irradiation indiscriminately caused the apoptosis of a substantial number (40-80% or 60-80%) of immune cells (especially T and B cells and macrophages; Fig. 2b). Because professional phagocytes were also reduced, if these cells played a critical function in the induction of long-term tolerance in the current test system, it would be expected that irradiation plus peptide injection in the absence of replenishing exogenous phagocytes would not suppress EAE. The hypothesis was first tested in SJL mice with established EAE. At the peak of acute disease, mice received γ-irradiation plus pPLP and normal professional phagocytes (IRR+MΦ+PLP) (Fig. 3a). The control groups included mice with immunization alone (PBS), irradiation plus pPLP (IRR+PLP), irradiation plus phagocytes (IRR+MΦ), or irradiation plus phagocytes and pOVA (IRR+MΦ+OVA). All the mice receiving irradiation showed a transient remission in disease; however, differences were evident when the mice started to relapse. While PBS, IRR+PLP or IRR+MΦ+OVA-treated mice developed typical relapsing and remitting EAE, IRR+MΦ alone or plus pPLP-treated mice (tolerized mice) showed significant suppression of chronic EAE (Fig. 3a). These results were similar to those in T cell (Fig. 1a) or B cell (Fig. 2a) apoptosis-antigen therapies. Analysis of the CNS showed a substantial reduction in inflammatory cell infiltration (Fig. 3b) in tolerized mice. In the spinal cords, although all mice receiving irradiation increased their frequency of Foxp3+ T_{reg} cells compared with untreated mice, tolerized mice had a considerably lower frequency of Th17 cells (Fig. 3c). In marked contrast, mice treated with IRR+PLP or IRR+MΦ+OVA exhibited increased Th17 cells (Fig. 3c). The changes in Th1 cells, however, were inconclusive (Fig. 3c). Consequentially, the ratio of Th17 cells to Foxp3+ T_{reg} cells was lowest in IRR+MΦ+PLP-treated mice (tolerized mice). In the spleens, CD4+ Foxp3+ T_{reg} cells were increased in IRR+MΦ+PLP-treated mice (Figures 2f, 26 and Figures 12 a and b). pPLP-specific T cell proliferation and inflammatory cytokine production were significantly inhibited in the spleens of IRR+MΦ+PLP-treated mice compared to untreated mice (Fig. 3d,e). These data indicated that only the combined presence of: (1) apoptotic cells and (2) phagocytes along with (3) the EAE-specific self-peptide was capable of suppressing disease in mice with established EAE.

This question was also addressed in the prevention model of the relapsing-remitting EAE model in SJL mice. The data showed that indeed co-transfer of normal SJL splenic macrophages and iDCs with pPLP, but not with PBS, into irradiated mice before EAE was induced significantly suppressed acute and chronic EAE (Figure 13 a). As expected, irradiation plus pPLP injection without co-transfer of professional phagocytes failed to prevent or inhibit acute and chronic EAE; in fact, the acute phase of the disease was much more severe than in control immunized mice (Figure 13 a). Consistent with inhibition of the disease, the tolerized mice treated with irradiation plus phagocytes and pPLP showed a substantial reduction in infiltrated inflammatory cells in the spinal cords and significant reduction of pPLP- specific T cell proliferation and proinflammatory cytokine production in the splenocytes (Figure 13 c,e,f). *Mycobacterium tuberculosis* (MT) antigen-specific T cell proliferation in the same tolerized mice was not affected (Figure 13d). The data together indicated a crucial function for professional phagocytes in apoptosis-antigen-mediated immune tolerance (specifically, γ-irradiation-induced apoptosis of immune cells was the primary trigger of EAE suppression, but professional phagocytes and specific autoantigen must be present). It was concluded that cell apoptosis rather than molecular signaling in immune cells was the primary trigger of EAE suppression.

### TGFβ is key in apoptosis-antigen-mediated therapy of EAE and Type 1 Diabetes (T1D)

Since TGFβ is one of the primary cytokines produced by phagocytes upon digestion of apoptotic cells^{11,15,16}, the function of TGFβ in apoptosis-antigen-mediated suppression in EAE and T1D was determined.

TGFβ *in vivo* completely reversed the tolerance in NOD mice induced by apoptosis-antigen therapy (IRR+MΦ+GAD65 + αTGFβ) Anti-TGFβ-treated mice exhibited earlier-onset and more severe disease than untreated (PBS) NOD mice (Fig. 18e). The abrogation of tolerance after administration of anti-TGFβ was largely attributed to the reversion of increased Treg cells and of decreased IFN-γ-producing TH1 cells in the tolerized mice (Fig. 18f). Moreover, anti-TGFβ treatment reversed decreased GAD65-specific splenic T cell proliferation in tolerized mice (Fig. 19F). Anti-CD3-driven T cell proliferation was comparable in tolerized mice compared with untreated (PBS) mice or anti-TGFβ-treated mice (Fig. 10g).

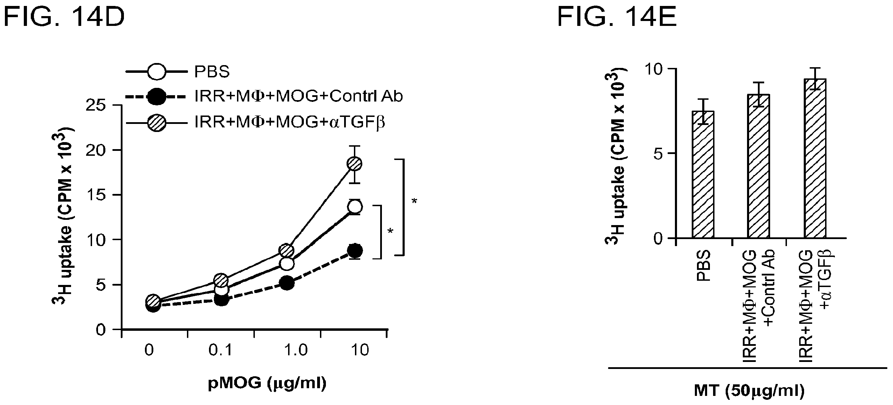

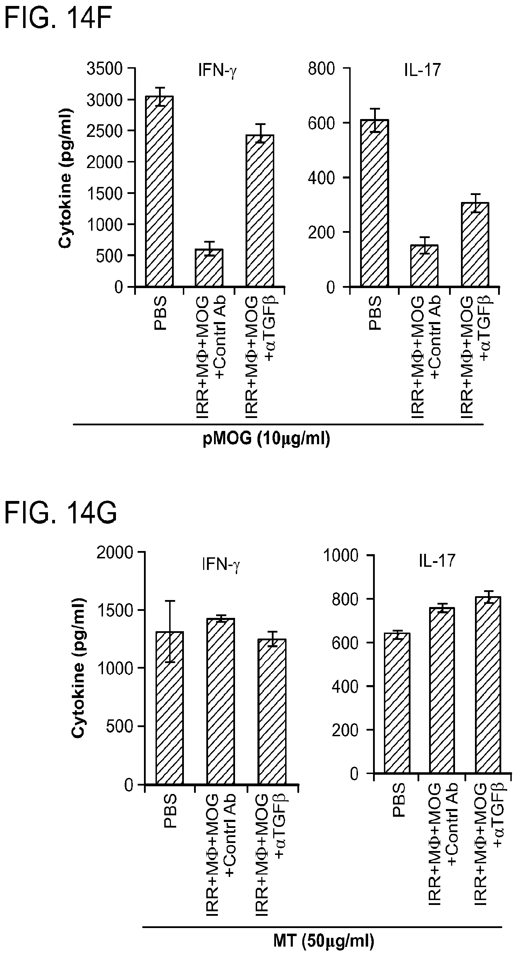

The role of TGFβ in apoptosis-antigen-mediated suppression of EAE was examined, using the myelin oligodendrocyte glycoprotein peptide₃₅₋₅₅ (pMOG)-induced EAE model in C57BL/6 mice. EAE mice were treated at the peak of acute EAE with αCD4/CD8 and pMOG (herein αCD4/CD8/MOG) in the absence (αCD4/CD8/MOG+Contrl Ab) and presence of anti-TGFβ neutralizing antibody (αCD4/CD8/MOG+αTGFβ) (Fig. 4a, upper panel). As expected, all mice with T cell depletion and pMOG injection showed rapid remission of EAE, which lasted for about a week. However, the difference in EAE disease emerged at 10-14 days after the treatment. While control antibody-treated mice (tolerized) continued to show remission of EAE, the mice treated with anti-TGFβ started to show relapses of EAE, and the disease scores soon reached and even overtook the levels of mice receiving only immunization (Fig. 4a). In the spinal cords, the total number of infiltrating immune cells in αTGFβ-treated mice was substantially higher than that in tolerized mice (Figure 14 a). Significantly, the decreased frequency of Th17 cells and the increased frequency of Foxp3+ T_{reg} cells in tolerized mice were both completely reversed by anti-TGFβ injection (Fig. 4b). The changes of Th1 cells were inconclusive (data not shown). In the spleens, the increase in CD4+ Foxp3+ T_{reg} cells in tolerized mice was completely abrogated by αTGFβ treatment (Figure 14 b). In cell cultures, αTGFβ treatment reversed the inhibition of pMOG-specific T cell proliferation (Fig.4d-f) and of inflammatory cytokine secretion in tolerized mice (Fig. 4g). However, IL-10, another immunoregulatory cytokine produced by phagocytes after digesting apoptotic cell17 seemed dispensable in the apoptosis- antigen-mediated therapy of EAE. Blockade of IL-10 signaling with anti-IL-10 receptor antibody failed to reverse the suppression of EAE in C57BL/6 mice induced by T cell depletion and pMOG injection compared to its isotype control antibodies (data not shown). Thus, the findings indicated that TGFβ but not IL-10 was critical in the therapeutic effects on EAE by the apoptosis-antigen combination.

EAE mice were also treated at the peak of acute EAE with γ-irradiation plus phagocytes and pMOG in the absence (IRR + MΦ + MOG + contrl Ab) or presence of anti-TGFβ-neutralizing antibody (IRR + MΦ + MOG + αTGFβ (Fig. 23A). All mice treated with γ-irradiation plus phagocytes and pMOG injection showed rapid remission of EAE, which lasted for about a week. However, the difference in EAE disease emerged at 10 to 14 days after the treatment. Whereas control antibody-treated mice (tolerized) continued to show remission of EAE, the mice treated with anti-TGFβ started relapse, and the disease scores soon reached the levels of mice receiving only immunization (Fig. S23A). In the spinal cords, the total number of infiltrating CD4+ in anti-TGFβ-treated mice was increased (Fig. S22B). Notably, anti-TGFβ treatment reversed the increased pMOG-specific (determined by MOG38-49 tetramer staining) Treg cells (Fig. 24A), and also reversed the increased ratios of Treg cells to TH17 and TH1 cells in the spinal cord (Fig. 24A to C). In the spleen, the pMOG-specific T cell proliferation and cytokine production in tolerized mice were completely abrogated by anti-TGFβ treatment *in vivo* (Fig. 23C and D). In contrast, MT-driven T cell proliferation and cytokine production were unchanged in tolerized mice compared to untreated (PBS) or anti-TGFβ-treated mice (Fig. 25A and B).

### Antigen-specific Treg cells were generated in apoptosis-antigen tolerized EAE mice

Next, validation of the function of TGFβ in irradiation- phagocytes -pMOG therapeutic model of EAE in C57BL/6 mice was performed, and similar results were observed (Figure 14 d-g). The data collectively indicated that TGFβ was key in the apoptosis-antigen-mediated therapy of EAE. Generation of antigen-specific T_{reg} cells in apoptosis-antigen tolerized mice As TGFβ was found to be essential in mediating the therapeutic effects (Fig. 4), and TGFβ is the critical factor in generating Foxp3+ T_{reg} cells in vitro⁵, it was hypothesized that the apoptosis-antigen treatment induced antigen-specific CD4+ Foxp3+ T_{reg} cells. Since CD4+ CD25+ Foxp3+ T cells in the SJL mice with established EAE are a pool of T_{reg} cells recognizing many different antigens, it was impossible to identify pPLP-specific T_{reg} cells with the markers of CD25 and Foxp3. An *in vitro* experimental culture system was therefore developed to determine the presence of an increase in pPLP-specific T_{reg} cells. CD4+ T cells and their CD4+ CD25- and CD4+ CD25+ subsets from the spleens of EAE mice after apoptosis-antigen therapy were isolated, and their antigen-specific T cell proliferation and cytokine production was examined by culturing them with pPLP and splenic antigen-presenting cells (APCs) isolated from the immunized (PBS) mice. As controls, the same T cell subpopulations were also re-stimulated with MT antigen or with anti- CD3. The rationale for this experimental approach was the fact that the Foxp3+ T_{reg} cell requires TCR stimulation by specific antigen in order to suppress its target cells^{18,19}. If pPLP-specific Foxp3+ T_{reg} cells were generated and functioned as suppressor T cells in the tolerized mice, it would have been expected to see decreased CD4+ T cell responses to pPLP in these mice relative to responses in the untreated (PBS) mice. However, when CD4+ CD25+ Foxp3+ T cells were removed from CD4+ T cells, the remaining CD4+ CD25-T cells in the tolerized mice exhibited similar or even stronger T cell responses to pPLP. The CD4+ CD25+ T subpopulation in the same tolerant mice would also exhibit weaker responses, if any, to pPLP compared to their counterparts in the untreated mice.

On the other hand, the same subpopulations of CD4+ T cells would exhibit no significant alterations of T cell responses to MT or CD3 antibody compared to untreated control mice. Indeed, non-separated CD4+ T cells from the spleens of IRR+MΦ+PLP-treated tolerized mice (Fig. 3) showed significantly decreased CD4+ T cell proliferation to pPLP (Fig. 5a), but not to MT antigen (Fig. 5b) or to anti-CD3 (Fig. 5c) stimulation. However, CD4+ CD25- T cells strikingly regained their proliferation to pPLP at levels the same as (or even higher than) those from untreated mice (Fig. 5a). pPLP-specific inflammatory cytokines production was also inhibited in splenic CD4+ T cells in tolerized mice. Again, the inhibition was completely restored to the levels of untreated CD4+ cells when the CD4+ CD25+ cells were removed (Fig. 5d). In marked contrast, CD4+ T cells from IRR+MΦ+OVA-treated mice showed no inhibition of pPLP-specific T cell responses (Fig. 5a-d), consistent with their failure to suppress EAE (Fig. 3).

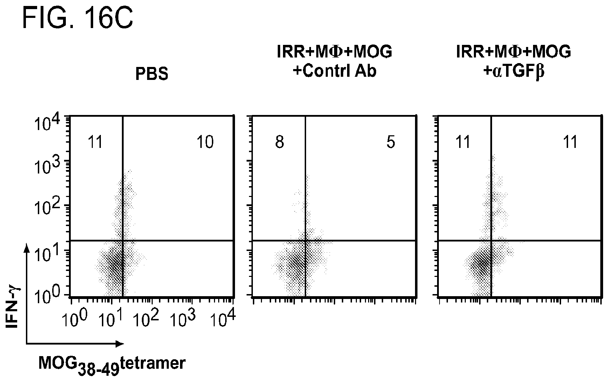

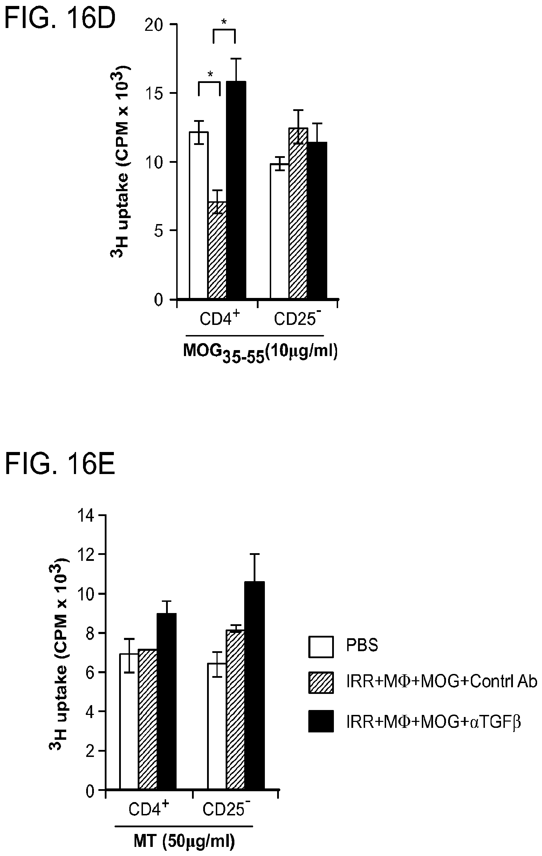

pPLP-specific T_{reg} cell generation in other therapy models of SJL mice was then investigated with αCD20/CD8 plus pPLP (Fig. 5e-g) or with αCD4/CD8 plus pPLP (Fig. 5h-j) and similar results were observed. This generation of autoantigen-specific T_{reg} cells was confirmed in apoptosis-antigen-induced remission of pMOG-induced EAE (Figure 16 d,e and Fig. 28A, B). Tetramers recognizing MOG38-49-specific CD4+ T cells were also used with Foxp3 staining to determine the specificity of Treg cells in C57BL/ 6 mice with established EAE. Indeed, the frequency of MOG38-49 tetramer-positive Treg cells was substantially increased, and the ratios of these Treg cells to TH17 or TH1 cells were also increased in the spinal cords of tolerized mice (IRR + MΦ + MOG) compared to untreated (PBS) mice (Fig. 24A to C).

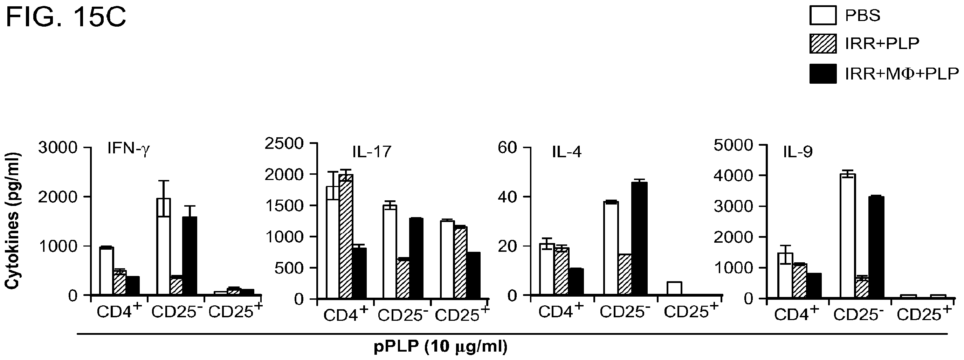

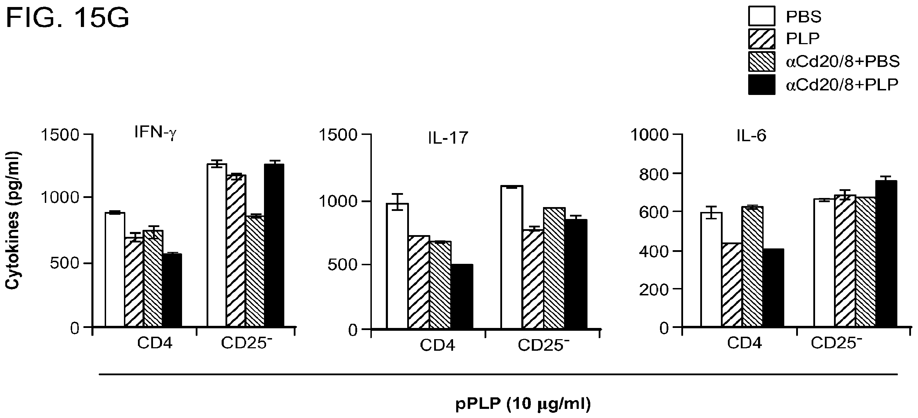

Furthermore, the generation and function of autoantigen-specific Foxp3+ T_{reg} cells in the tolerized mice induced by irradiation plus professional phagocytes and peptides or by αCD20/CD8 plus peptides in the prevention model of EAE in SJL mice was also determined (Figure 15). These data collectively indicated that the apoptosis-antigen treatment generated autoantigen-specific T_{reg} cells in therapeutic and preventive models of EAE. The antigen-specific T_{reg} cells selectively suppressed autoimmune T cell responses without compromising the overall immune responses.

Tetramers recognizing MOG(38-49)-specific CD4+ T cells were used with Foxp3 staining to determine the specific T_{reg} cells in C57BL/6 mice with established EAE that were suppressed with irradiation plus transfer of phagocytes plus pMOG injection (IRR+Mϕ+MOG+Control Ab). The frequency of CD4+ Foxp3+ tetramer-positve T_{reg} cells was indeed substantially increased, and that of tetramer-positive Th17 or Th1 cells was decreased in the spinal cords of tolerized mice compared to untreated groups (Figure 16 a-c). Significantly, αTGFβ completely abrogated the increase in tetramer-positive T_{reg} cells and reversed the decrease in tetramer-positive Th17 or Th1 cells in CNS. (Figure 16 a-c). Consistent with these results, the pMOG-specific CD4+ T cell proliferation in IRR+Mϕ+MOG+Control Ab-treated mice was completely abrogated by αTGFβ treatment in vivo (Figure 16 d), suggesting an indispensible function of TGFβ in the antigen-specific T_{reg} cell generation in the tolerance.

To determine the cellular sources of TGFβ in treated (tolerized) mice, cell membrane-bound TGFβ was examined in both macrophages and Treg cells (Perruche et al. Nat. Med. 14: 528-535; Nakamura et al. J. Exp. Med. 194: 629-644; Belghith et al. Nat. Med. 9: 1202-1208). C57BL/6 mice were immunized with pMOG plus FCA to develop EAE. At the peak of EAE, mice were treated with IRR + MΦ + MOG or untreated (PBS). It was observed that macrophages expressed higher amounts of latency-associated peptide (LAP)- TGFβ1 than did control macrophages on the second day after apoptosis induction (day 16; Fig. 27A). The LAP-TGFβ1+ macrophages in the tolerized mice gradually decreased, suggesting that macrophage production of TGFβ was transient (Fig. 27A). In contrast, LAP-TGFβ1 expression by Treg cells was kinetically different. There was no increase in Foxp3+ TGFβ1+ Treg cells at day 16 or day 23, but LAP-TGFβ1+ Treg cells were increased at day 32 (Fig. 27B). These data suggested that TGFβ in tolerized mice could be produced by both macrophages and Treg cells. However, macrophage-derived TGFβ was most likely caused by exposure to apoptotic cells after irradiation, whereas Treg-TGFβ1+ cells were likely generated later and were antigen-induced/expanded antigen-specific Treg cells (Chen et al. J. Exp. Med. 198: 1875-1886; Perruche et al. Nat. Med. 14: 528-535). Nevertheless, these data indicated that TGFβ was key to establishing tolerance in apoptosis-antigen therapy.

### Treg cells play a key role in apoptosis-antigen therapy

The role of Treg cells was also determined for apoptosis-antigen therapy in pMOG-induced EAE. CD4+CD25+ Treg cells were depleted using anti-CD25 antibody (Sakaguchi et al. Immunol. Rev. 212: 8-27) in EAE mice that were also treated with IRR + MΦ + MOG (Fig. 26A). Depletion of Treg cells completely abolished apoptosis-antigen-driven tolerance in mice with established EAE (Fig. 26A). Treg cell-depleted mice showed enhanced numbers of infiltrating cells in the spinal cords compared to tolerized mice (IRR+MΦ+MOG + contrl Ab) (Fig. 26B). MOG-specific proliferation and cytokine production were also restored in the spleens of Treg cell-depleted mice (Fig. 26C and D). These data indicated that Treg cells were vital for tolerance in apoptosis-antigen therapy.

Thus, these data have provided strong evidence that autoantigen-specific T_{reg} cells were indeed generated and functional in suppressing autoantigen-specific T effector cell responses, and this plays a major role in the therapy of EAE induced by apoptosis-antigen combination.

### Antigen-specific T_{reg} cells converted from naïve CD4+ T cells in apoptosis-antigen tolerized mice

To determine if antigen-specific T_{reg} cells were indeed converted from naive T cells in vivo in the immunosuppressive milieu triggered by apoptosis-antigen administration, TCR transgenic naive T cells (2D2) specific to pMOG were injected into syngeneic C57BL/6 mice either treated with IRR+Mϕ+MOG or untreated before immunization. IRR+Mϕ+MOG-treated mice showed suppression of EAE compared to the untreated mice (Figure 17 a). 2D2 T cells in the CNS showed an increased frequency of Foxp3+ Treg cells and a decrease in Th17 and Th1 cells in mice with suppressed EAE (Figure 17 b), suggesting that apoptosis and antigen treatment drove more antigen-specific T_{reg} cell generation from naive T cells. To further confirm that apoptosis-antigen treatment induced antigen-specific iTreg cells in nontransgenic settings, Treg cells were stained for neuropilin 1 (Nrp-1), a marker to identify tTreg cells (Nrp-1+) from iTreg cells (Nrp-1-) (Yadav et al. J. Exp. Med. 209: 1713-1722, S1-S19). It was demonstrated that MOG₃₈₋₄₉ tetramer+ Nrp-1- Foxp3+ Treg cells were significantly increased in the spleen and spinal cords of tolerized mice (Fig. 30A), indicating that apoptosis-antigen treatment induced iTreg cells. This was further supported by the fact that neutralization of TGFβ substantially reduced Foxp3+ Nrp-1-MOG₃₈₋₄₉ tetramer+ Treg cells (Fig. 30B).

To provide unambiguous evidence that the apoptosis-antigen combination indeed promoted Foxp3 T_{reg} cell conversion from naive CD4+ T cells in vivo, TCR transgenic CD4+ CD25- T cells (KJ1-26+ , specifically recognizing pOVA) isolated from DO11.10xRag-/- mice (which have no endogenous Foxp3+ Treg cells) were injected into syngeneic 7-week-old BALB/c mice. To avoid depletion of transferred transgenic T cells in BALB/c mice, two immune cell depletion models were used: by anti-CD8/CD20 antibody injection or systemic γ-irradiation. Here, it was shown that anti-CD8/CD20 injection and pOVA administration resulted in significantly more KJ1-26+ Foxp3+ T_{reg} cells (Fig 6a-c, Figure 17 c), but less KJ1-26+ IL-17+ cells compared to pOVA treatment alone in BALB/c mice (Fig. 6d). It also dramatically decreased pOVA-specific inflammatory cytokines in the spleen cell cultures (Figure 17 d). Similar results were obtained when BALB/c mice were treated with irradiation plus phagocytes and pOVA (Fig. 6e-h, Fig. 31A to D). Importantly, anti-TGFβ antibody injection completely abrogated the increase in KJ1-26+ Foxp3+ T_{reg} cells and the decrease in KJ1-26+ IL-17+ cells in the treated/tolerized BALB/C mice (Fig. 6 and Figure 17 c).

The above data collectively provided clear evidence that apoptosis-antigen treatment converted naive CD4+ T cells to antigen-specific Foxp3+ T_{reg} cells, and this conversion required TGFβ and also phagocytes that sense and digest the apoptotic cells.

An antigen-specific therapy for autoimmune disease that does not compromise overall immune response is the ultimate goal for medical researchers and clinicians. In the above described experiments, a novel pathway was discovered for generation of autoantigen-specific T_{reg} cells in vivo that specifically suppress autoimmune T cell responses to the target tissues without compromising the overall immune response in mice. The dysregulated immune system was reprogrammed and, importantly, the disease was controlled.

Several novel conclusions can be drawn from the current studies. First, apoptosis, rather than signaling in immune cells, is a key to initiating long-term immune tolerance. The apoptosis process requires transient yet sufficient apoptosis of cells *in vivo.* Supporting this conclusion is the fact that tolerance can be induced irrespective of the procedure for apoptotic cell induction or the type of apoptotic cells, as long as the phagocytes are present. Depletion of CD4+ T cells to suppress EAE was reported more than 20 years ago^{13,14}, but the mechanisms underlying the effects were unknown. The studies here have identified that the depletion of T cells can serve as an initiator of a series of events that ultimately produces long-term immune tolerance. Indeed, depletion of non-CD4+ immune cells lead to similar therapy of EAE. The mechanisms of apoptosis-triggered tolerance reported here are also different from recent studies using non-depleting CD4-specific antibody treatment. The non-depletion CD4-specific antibody is based on the blockade of CD4 molecules on T cells and also does not involve administration of peptide²¹, whereas the present study relied on the transient and sufficient extent of cell apoptosis that initiated the whole tolerance process. Second, apoptosis-antigen treatment was not linked to inflammatory cytokine release by immune cells. This differs from CD3-antibody-mediated T cell depletion, which can transiently yet powerfully trigger TCR on T cells to release proinflammatory cytokines *in vivo*^{11,22,23}. This lack of overt inflammatory cytokines in a TGFβ-rich immunoregulatory milieu could provide a precondition for the ensuing generation of T_{reg} cells. Third, phagocytes²⁴ were key in mediating the long-term immune tolerance and therapy of EAE presented here. This notion was supported by experiments of either depletion of endogenous phagocytes in tolerized mice induced by T cell depletion plus self-peptide treatment, or by adoptive transfer of syngeneic normal splenic macrophages and DCs plus self-peptide in irradiated mice. This conclusion was further supported by the data showing that depletion of immune cells alone or plus self-peptide in the absence of a sufficient number of professional phagocytes failed to generate antigen-specific T_{reg} cells and immune tolerance, which led to no suppression of EAE. In fact, these conditions may exacerbate EAE by enhancing T effector cells, likely due to empty space-driven T cell expansion. Fourth, TGFβ, but not IL-10, was vital to inducing long-term immune tolerance and remission of EAE induced by apoptosis-antigen therapy through generation of antigen-specific T_{reg} cells *in vivo.* Although many cells can produce TGFβ *in vivo*^{25,26}, macrophages are likely the major cellular source of TGFβ in apoptosis-mediated immune tolerance immediately upon contact/digestion of apoptotic cells. Treg cells (likely antigen-specific Treg cells), however, can be another cellular source of TGFβ, especially at the later stage of the apoptosis-antigen therapy. Fifth, proper and timely antigenic peptide introduction into the transiently established immunosuppressive milieu in mice was shown to be key to induce antigen-specific T_{reg} cells. It has been shown here that only with the combination of apoptosis, phagocytes, and antigen can the antigen-specific T_{reg} cells be optimally generated and long-term immune tolerance developed, i.e., the proper antigenic peptide needs to be introduced in a timely manner into mice in which an immunoregulatory milieu was created by apoptosis-triggered phagocytes. In addition, the specificity of the antigenic peptide is also critical in tolerance induction. It was found here that injecting the same amounts of an irrelevant control peptide such as pOVA instead of self-peptide (pPLP or pMOG) failed to suppress EAE in SJL or B6 mice, respectively. The administration of pOVA could theoretically induce pOVA-specific T_{reg} cells in an apoptosis-triggered TGFβ-rich immunosuppressive microenvironment. However, these pOVA-specific T_{reg} cells could not survive, expand, and function sufficiently to suppress the disease, as there is no continuous pOVA stimulation in EAE mice^{7,18,27-31}. This finding has implications in translating the study to human patients, as even if some unwanted peptide was present during the transient immunosuppressive milieu and T_{reg} cells specific to that antigen was induced by byproduct, it would not affect immune response to the antigen as long as the unwanted peptide does not stay around. Another important point to mention is that, unlike in prevention models, depletion of immune cells in the presence of phagocytes without addition of exogenous autoantigenic-peptides in the therapy models (after immunization) did result in some suppression of EAE. This phenomenon might be attributable to the fact that the mice with established EAE likely have some endogenous self-peptide present that was derived from the immunization step. Thus, it is conceivable that dose, time, and length of the specific self-peptide injection will be important factors to consider in future clinical application to patients.

Importantly, it was determined that the apoptosis-antigen combination treatment induced and increased antigen-specific T_{reg} cells in vivo. TGFβ is absolutely required for this process. These antigen-specific T_{reg} cells could serve the major force for inducing and maintaining long-term immune tolerance, and thus inhibition of EAE and T1D. These findings were significant, at least because this appears to be the first identification that antigen-specific T_{reg} cells can be induced in mice with established autoimmune disease and suppress and prevent relapses of the disease, which should have clinical implication in patients with multiple sclerosis (MS) and T1D. These studies suggested that the dysregulated immune responses in patients with autoimmune diseases can be reprogrammed.

Another significant feature of this approach is that there is no obvious nonspecific overall immunosuppression or immune defect to antigens from pathogens in this induced tolerance. This conclusion is supported by the current data that the CD4+ T cells isolated from mice with long-term remission of EAE and T1D showed intact T cell responses to the pan-TCR stimulation using CD3 antibody, suggesting no overall immune defect occurred in the tolerized mice. Importantly, CD4+ T cells that exhibit tolerance to auto-antigen stimulation showed normal T cell proliferation and effector differentiation to bacterial antigens. How this occurs in vivo remains unknown, but several possible explanations can be postulated. First, immune cell depletion and the consequent suppressive immune environment are transient, and T cells recover. Second, if, in the TGFβ- immunoregulatory milieu, the body by chance also encounters bacterial or virus antigens, the pathogen-specific T cells might be unlikely to direct to T_{reg} differentiation, but instead to T effector cells, because the pathogens could through their TLR pathways trigger inflammatory cytokines that abrogate T_{reg} cells^{30,32-34}.

In sum, described herein is the development of a novel pathway for reprogramming the dysregulated immune system and responses to EAE and T1D therapy in mice, with an ultimate use as therapy for patients with T1D, MS and other autoimmune diseases or disorders. This discovery relies on the induction of autoantigen-specific T_{reg} cells that functionally suppress autoimmunity in the target tissues without ompromising the overall immune response in the host. This protocol has applications to other types of autoimmune disease, as long as one or more self-peptides are identified.

### Example 2: Therapy of diabetes by inducing antigen-specific regulatory T cells in vivo

Apoptosis-antigen mediated therapy of type 1 diabetes model in NOD mice was examined. 9 wk-old NOD mice were irradiated with γ-irradiation (IRR) with dose of 200 rad. Some mice received normal splenic macrophages and DCs (MODC). Some mice were administered 5µg of Glutamic acid decarboxylase 65 (GAD₆₅) peptide or PBS every other day as indicated. (a), upper panel, the experimental scheme; Lower panel, the frequency of diabetes free mice. PBS (untreated control, n=3), GAD₆₅ (GAD65 alone, n=3), IRR+MODC+GAD65 (irradiation plus MODC plus GAD₆₅, n=5), IRR+MODC (irradiation plus MODC, n=5). (b), The frequency of Foxp3+ (left) and IFN-γ+ (right) cells within CD4+ T cells in the pancreas draining lymph nodes (DLN) are shown, as determined by flow cytometry. *P<0.05, determined by Student's t test (two-tail).

### Apoptosis-antigen therapy suppressed T1D in non-obese diabetic mice

The generality of apoptosis-antigen therapy in other autoimmune settings was assessed by examining T1D in nonobese diabetic (NOD) mice (Anderson and Bluestone, Annu. Rev. Immunol. 23: 447-485). T1D develops from 12 weeks of age in female NOD mice as a result of insulitis, a leukocytic infiltrate in the pancreatic islets. GAD₆₅ has been identified as one of the autoantigens in NOD mice and in patients with T1D (Kaufman et al. Nature 366: 69-72; Lohmann et al. Lancet 356: 31-35). NOD mice were treated at the age of 9 weeks, when the mice are considered diabetic without hyperglycemia, as the inflammatory process has been initiated and is in progress, yet the levels of glucose in the blood are still within the normal range (Anderson and Bluestone). NOD mice were either untreated (PBS) or treated with GAD₆₅ peptide (GAD₆₅) or with γ-irradiation followed by administration of GAD₆₅ peptides plus phagocytes (IRR + MΦ + GAD₆₅) (Fig. 18a). As expected, untreated NOD mice as well as GAD₆₅-treated mice started to develop diabetes at 12 weeks of age, and all of them were diabetic by the age of 23 weeks (Fig. 18a). Strikingly, however, NOD mice that were treated with IRR +MC+GAD₆₅ were diabetes-free through 23 weeks of age when the experiment was terminated (Fig. 18a). Consistent with the levels of blood glucose, the IRR +MΦ + GAD₆₅-treated (tolerized) mice showed considerably less insulitis than untreated NOD or GAD₆₅-treated mice (Fig. 18c). The frequency and absolute number of interferon-y (IFN-y)-producing CD4+ and CD8+ T cells were markedly decreased, whereas the frequency of Foxp3+ Treg cells was increased in the pancreatic draining lymph node (DLN) in the tolerized mice (Fig. 2f and 2g, and Fig. 19A). TH17 cells were undetectable in all the groups (Fig. 20), consistent with the dispensable role of TH17 cells in diabetes in NOD mice (Joseph et al. J. Immunology 188: 216-21). The total number of T cells in the spleen was comparable between tolerized mice and untreated mice (Fig. 21). GAD₆₅-specific T cell proliferation and IFN-γ production in the spleen were specifically suppressed in tolerized mice (Figs. 19B and 19D). Notably, anti-CD3-driven T cell proliferation and IFN-γ production were comparable between tolerized and untreated spleens (Figs. 19C and 19E).

To investigate whether the apoptosis antigen therapy was effective in NOD mice with established T1D, hyperglycemic NOD mice with glucose levels >200 mg/dl were treated with γ-irradiation plus phagocytes and GAD65 peptide injection. Strikingly, it was found that the treatment blocked the progress of diabetes, preventing further increases in blood glucose levels, which remained at 200 to 300 mg/dl, whereas the untreated mice quickly exhibited elevated blood glucose levels (>600 mg/dl) (Fig. 22A). Histological analysis of the pancreases revealed that the treated mice still had preserved islets in the pancreases (Fig. 22, B to D). The untreated NOD mice, however, hardly showed any islets (Fig. 22, B to D). Analysis of pancreatic DLNs revealed that the treated (tolerized) mice showed significantly higher frequencies of Treg cells and lower TH1 cells than did untreated mice (Fig. 22, E and F). Thus, apoptosis-antigen therapy not only prevented prediabetic NOD mice from developing diabetes but also halted diabetes progression in recently hyperglycemic NOD. Together, these data demonstrated that apoptosis-antigen-mediated immune tolerance occurred in a T1D setting.

### Example 3: GAD65-specific Treg cells were induced in tolerized NOD mice

To confirm that observed tolerance was mediated by induction and increase of GAD₆₅-specific Treg cells in NOD mice, CD4+ T cells and CD4+CD25-T cells were stimulated with GAD₆₅ peptide in the same manner as outlined in the EAE study. Significantly decreased GAD₆₅-driven IFN-γ production was observed by CD4+ T cell in the IRR + MΦ + GAD₆₅ + contrl Ab-treated tolerized mice compared to untreated (PBS) mice. Levels of anti-CD3-driven CD4+ T cell IFN-γ production were comparable between these two groups (Fig. 29A and B). When CD4+CD25+Treg cells were removed, the remaining CD4+CD25- T cells in the tolerized mice regained their GAD₆₅-stimulated IFN-γ production (Fig. 29A and B). Again, neutralization of TGFβ *in vivo* completely abrogated the suppression of GAD65-specific IFN-γ in tolerized CD4+ T cells (Fig. 29A). These data indicated that apoptosis-antigen therapy suppressed T1D by inducing antigen-specific Treg cells *in vivo.* Together, these data provided compelling evidence that autoantigen-specific Treg cells could be induced by apoptosis-antigen therapy. Specifically, it was shown that macrophages, when encountering apoptotic cells, could drive the development of tolerance in mice with existing autoimmunity and that this was effective in both EAE and T1D.

An exemplary application of this approach involves administration of single-dose irradiation to a subject (*e.g.*, 200-400 rad dosage of radiation, resulting in depletion of all types of immune cells, such as T cells, B cells, and macrophages, etc.) followed by adoptive transfer of normal macrophages and administration of autoantigenic peptides (Kasagi et al., Science Translational Medicine 6(241): 241ra78). A similar exemplary application of a different aspect of the invention involves administration of anti-CD20 and anti-CD8 antibodies to a subject (resulting in depletion of B lymphocytes and CD8+ T cells, without depleting CD4+ T cells) and administration of autoantigenic peptides (Kasagi et al., Science Translational Medicine 6(241): 241ra78).

### Example 4: Apoptosis-phagocytosis induced immune tolerance for the treatment of Collagen-Induced Arthritis

Despite the development of biological agents, a large portion of patients with arthritis still suffer from disability. Collagen-Induced Arthritis (CIA) is the prototype model of autoimmune arthritis, which shares many features with rheumatoid arthritis.

Mice used in the following experiments are preferably DBA/1LacJ in SPF condition; however, other options include B10.RIII, B10.M-DR1 and C57BL/6, although their susceptibility varies.

Reagents for immunization are type II collagen (CII) and complete Freund's adjuvant (CFA).

Preparation is as follows:
1. Dissolve the 100 µg CII in 10 mM acetic acid to final concentration 4 mg/ml by stirring overnight at 4°C.
2. Grind heat-killed Mycobacterium tuberculosis finely in a mortar and pestle and combine with IFA at a 4 mg/ml final concentration to prepare complete Freund's adjuvant (CFA). Commercially prepared CFA may also be used, but may result in a lower incidence and severity of arthritis.
3. Using a Virtis high-speed homogenizer emulsify CII in an equal volume of CFA just prior to immunization.

Administration is as follows: (day 0): inject with 100 µg CII and 100 µg CFA emulsion in a total volume of 50 µl intradermally at the base of the tail.

Disease course is as follows:
Onset: 21-28 days after immunization
Peak: 35-50 days after immunization
Incidence: 90-100%

Assessments are made three times a week as follows:
1. Visual Assessment: Disease severity score (0-4)
2. Plethysmography Assessment: Plethysmometer or water displacement for measuring paw volume
3. Radiological Assessment (Optional): Joint cortical bone volume (JCBV), as determined by microcomputed tomography analysis of metatarsophalangeal and metacarpophalangeal joints.
4. Histological Assessment: H&E stains of hind paws spanning metatarsal, tarsal and calcaneous bones.

Treatment Groups are as follows:
Arm A: No;
Arm B: sublethal dose (200rad) of γ-Irradiation (day 35);
Arm C: sublethal dose (200rad) of γ-Irradiation (day 35);
   ▪ administration of professional phagocytes (day 35);
Arm D: sublethal dose (200rad) of γ-Irradiation (day 35);
   ▪ CII (dose is to be determined) (day 35);
Arm E: sublethal dose (200rad) of γ-Irradiation (day 35);
   II administration of professional phagocytes (day 35);
   ▪ CII (dose is to be determined) (day 35);

Screening and grouping into comparable arms is carried out prior to the beginning of treatment. Based on the incidence and the number of groups, the estimated total number of mice in one experiment is 20.

The Endpoint is at day 56, and the following are assessed:
1. Total disease severity score in each group
2. Total joint swollen change in each group
3. JCBV (Optional) in each group
4. Histological Change in each group

### Example 5: Apoptotic cells induce tolerance in prevention and therapy of lung fibrosis and systemic sclerosis

Systemic sclerosis (SSc) is a connective tissue disease characterized by excessive extracellular matrix deposition with an autoimmune background. Presence of autoantibodies is a central feature of SSc, antinuclear antibodies (ANAs), such as anti-DNA topoisomerase I (anti-topo I) antibody, are detected of patients. Furthermore, abnormal activation of several immune cells has been identified in SSc. Prognosis is very poor in patients with diffuse type SSc (10-year survival of 55%) because of limited application of medication. Therefore, establishing treatment is urgent need for these patients.

Mice used in these experiments are C57BL/6 mice (The Jackson Laboratory) in a SPF condition.

For immunization, recombinant human topo I (TopoGEN) was dissolved in saline (500 units/ml). The topo I solution was mixed 1:1 (volume/volume) with CFA H37Ra (Sigma-Aldrich). These solutions (300 µl) were injected 4 times subcutaneously into a single location on the shaved back of the mice with a 26-gauge needle at an interval of 2 weeks. Human serum albumin (Protea Biosciences) was used as an irrelevant control human protein.

For treatment, mice were treated with either sublethal dose (200rad) of γ-Irradiation or anti-CD4/CD8 T cell depletion antibody followed by administration of professional phagocytes at the peak of disease (around day 42).

The End point for these experiments is the time point in which the differences in dermal thickness between treated mice and untreated mice are clinically apparent.

This model is chosen because Topo1 is recognized as an antigen in patients with SSc. Human topo I has 93% sequence identity to mouse topo 1. Further, titers of anti-topo I antibody are positively correlated with disease activity in 20% of patients with SSc. Titers of anti-topo I antibody are selectively upregulated after immunization, which has correlation with lung and skin disease in this model mice. This phenomenon is similar to human disease. Finally, IL-6 and IL-17 seem to play a pathological role in this model mice. As there is evidence that irradiation plus professional phagocyte treatment induce T_{regs} and suppress IL-6 or IL-17 mediated inflammation in EAE model mice, this therapy is promising and expected to fit this mouse model.

### Example 6: Sjogren's Syndrome (SS) Therapy

A subject having or at risk of developing Sjogren's syndrome ("SS") is obtained or identified (exemplary subjects for Sjogren's syndrome therapy as described herein include a human subject having or at risk of developing SS, or a mouse model subject, such as mice having induced, experimental Sjogren's syndrome ("ESS") as described, *e.g.,* in Lin et al. (Ann. Rheum Dis 2014; 0: 1-9)). One or more of the following is administered to the subject:
(1) anti-CD4 and anti-CD8 antibodies (thereby inducing T cell apoptosis), followed by administration of auto-antigenic peptides (e.g., salivary gland peptides as described in Lin et al. (Int Immunol 2011; 23: 613-24) for ESS mice);
(2) Anti-CD20 and anti-CD8 antibodies (thereby depleting B lymphocytes and CD8+ T cells, without depleting CD4+ T cells), followed by administration of autoantigenic peptides; and/or
(3) irradiation (optionally single dose irradiation, e.g., 200-400 rad), thereby depleting all types of immune cells such as T cells, B cells, and macrophages, etc., with adoptive transfer of normal macrophages, followed by administration of autoantigenic peptides.

The subject is then monitored for reduction, alleviation and/or therapeutic mitigation of markers and/or phenotypes of SS, and an effective anti-SS therapeutic regimen is thereby identified.

### Materials and Methods

The results reported herein were obtained using the following methods and materials. *Mice.* C57BL/6 C57BL/6-Tg (Tcra 2D2, Tcrb 2D2)1Kuch (2D2), BALB/c, SJL, and CD45.1 (C57BL/6) mice were purchased from the Jackson Laboratory. DO11.10xRag1-/- mice were purchased from Taconic. Mice were maintained under specific pathogen-free conditions according to the National Institutes of Health guidelines for the use and care of live animals.

*Flow Cytometry.* Single-cell suspension were stained with the following flourochrome-conjugated antibodies; from eBioscience: CD8 (clone 53-6.7), DO11.10 TCR (clone KJ1-26), TNF-α (clone MP6-XT22), IL-17 (clone ebio17B7), CD4 (clone RM4-5), Foxp3 (clone FJK-16s), and from BD Biosciences; Vα3.2 (Clone RR3-16), Vβ11 (clone RR3- 15), and IFN-γ (clone XMG1.2). Foxp3 expression was examined using the eBioscience Foxp3 mouse T_{reg} kit. MOG38-49-specific TCR tetramer (PE-labeled I-A (b) GWYRSPFSRVVH (SEQ ID NO: 1) tetramer) and I-A (b)/hCLIP tetramers (negative control) were provided by NIH Tetramer Core Facility at Emory University. Cells from spinal cord or spleen were incubated with a 1:300 dilution of MOG38-49-specific TCR tetramer in DMEM plus 10% FBS for 3 hour at 4°C. Cells were then washed and stained for cell surface markers described above. For intracellular cytokine measurement cells were incubated with PMA (5 ng/ml, Sigma), ionomyocine (250 ng/ml, Sigma) and GolgiPlug (1µl/ml, BD Biosciences) to determine intracellular expression of IL-17, IFN-γ, and TNF-α. All samples were analyzed using a FACSCalibur flow cytometer (BD Biosciences) and data were analyzed using Flowjo software (Treestar).

*Peptides.* PLP₁₃₉₋₁₅₁ (HSLGKWLGHPDKF; SEQ ID NO: 2), MOG₃₅₋₅₅ (MEVGWYRSPFSRVVHLYRNGK; SEQ ID NO: 3) and OVA₃₂₃₋₃₃₉ (ISQAVHAAHAEINEAGR; SEQ ID NO: 4) were purchased from Invitrogen. GAD₅₂₄₋₅₄₃ (SRLSKVAPV1KARMMEYGTT; SEQ ID NO: 5) was purchased from Anaspec.

*Cell isolation.* CD4+ T cells, CD4+ CD25+ T cells, CD4+ CD25- T cells, CD11b+ cells, and CD11c+ cells were isolated from spleens via either positive or negative selection using MACS isolation kits (Miltenyi Biotech) following the manufacturer's protocols. Briefly, CD4+ CD25- T cells were isolated by the CD4+ CD25+ regulatory T cell isolation kit (T_{reg} kit). For isolation of CD11b+ cells (Mϕ) and CD11c+ cells (DCs), spleens were incubated for 20 min at 37 °C in a DMEM including 8mg/ml collagenase. Then cells were gently meshed through a cell strainer (70µm, BD Falcon). Mϕ and DCs were isolated via positive selection by CD11b and CD11c microbeads, respectively. Non-CD4+ T cells were isolated via negative selection by T_{reg} kit, and used as antigen presenting cells (APCs) after irradiation with 3000 rad of γ-irradiation (Gammacell 1000, Best Theratronics).

*Cytokine assays.* Splenocytes were cultured at 37°C in 5% CO2 for 2-3 days with either soluble CD3-specific antibody (anti-CD3) (0.5µg/ml) or MT (heat-killed M.tuberculosis, H37RA, DIFCO) (50µg/ml) or peptides (pMOG, pPLP)(0-50 µg/ml as indicated). Cytokines were quantified in culture supernatants by ELISA; TNF-α, IL-6, and IFN-γ (BioLegend) and IL-17 (eBioscience). EAE induction, scoring, analysis and in vitro cell cultures. Peptide-induced EAE was induced in SJL mice and C57BL/6 mice as previously reported ^{11,20}. Individual mice were observed daily and clinical scores were assessed on a 0-5 scale as follows: 0, no abnormality; 1, limp tail or hind limb weakness; 2, limp tail and hind limb weakness; 3, hind limb paralysis; 4, hind limb paralysis and forelimb weakness; and 5, moribund. 7-wk- old male C57BL/6 or CD45.1 mice were immunized subcutaneously with 200 µg/mouse of pMOG emulsified in CFA (IFA supplemented with 300 µg/ml MT). 7-wk-old female SJL mice were immunized subcutaneously with 75 or 100 µg/mouse of pPLP emulsified in CFA (MT 300 µg/mice). Mice also received 200 ng of Bordetella pertussis (List Biological Lab) i.p. on the day of immunization and 2 days later. At the end of each experiment spinal cords and brain were harvested and a part was fixed in neutral 10% formalin, extracted, embedded in paraffin and cut in 5 µm sections for H&E staining. Cells were isolated from brain and spinal cord as previously reported¹¹. Spleen was also harvested for further staining and culture. For cell cultures, splenocytes were cultured at 37°C in 5% CO2 for 3 days with either soluble anti-CD3 (0.5µg/ml) or MT (50µg/ml) or peptides (pMOG, pPLP). After 3 days culture, cells were pulsed with 1 µCi [3H] thymidine for 8-16h. Radioactive incorporation was counted using a flatbed β-counter (Wallac). To examine the function of peptide-specific CD4+ CD25+ T_{reg} cells in the spleen of mice, CD4+ , CD4+ CD25- , and CD4+ CD25+ T cells were MACS sorted and cultured with irradiated APCs from peptide-immunized EAE mice in the presence of either pPLP or pMOG (10 µg/ml), or MT (50 µg/ml), or anti-CD3 (0.5 µg/ml).!After 3 days of culture cells and supernatant were collected for cytokine assays and determination of T cell proliferation.

*Antibodies used for in vivo.* Anti-CD4 (clone Gk1.5), anti-CD8 antibody (clone 53- 6.72), anti-TGFβ antibody (clone 1D11) and mouse IgG1 (clone MOPC-21) were purchased from Bio X cell. Anti-CD20 antibody (clone 5D2) was a gift from Genentech. T cell depletion studies in EAE disease models. For EAE prevention studies SJL/J mice were either untreated or treated with CD4- (100 µg/mouse) and CD8- (50 µg/mouse) specific antibody (T cell depletion antibody). Some mice were immediately injected i.p. with pPLP or pOVA (25 µg/mouse) or PBS every other day for 16 days. All mice were immunized with pPLP and CFA (day 0). For EAE treatment studies, SJL mice were treated with CD4- and CD8-specific antibody and 5 µg of pPLP, pOVA or PBS i.p. every other day from day 12 to day 26 following immunization with pPLP (day 0). For EAE prevention studies, SJL mice were treated with CD4- and CD8- specific antibody at day -21, and 25 µg of pPLP, pOVA or PBS i.p. every other day from day -18 to day -2 before immunization with pPLP (day 0). In C57BL/6 mice, same T cell depletion regimen was utilized but pMOG (10 µg/mouse) was administered via i.p.. In some mice, anti-TGFβ or isotype control antibody (mIgG1) (200 µg/mice each day) were injected i.p. one day after T cell depletion. To examine the role of phagocytes in apoptosis-antigen combined treatment of EAE, mice were treated with either 300 µl of Clodronate liposome to deplete phagocytes as reported^{11,15,16}. B cell and CD8+ T cell depletion in EAE disease model. All mice were then immunized with pPLP and CFA (day 0) followed by twice injection of pertussis (day 0, 2). Mice were monitored for clinical score of disease and sacrificed at indicated time points. For therapy experiments, SJL mice were treated with anti-CD8/CD20 antibodies (day 9 after immunization), followed by i.p. administration of pPLP (5µg/mouse) every other day from day 10 to 23.

For prevention experiments, SJL mice were treated with anti- CD8/CD20 antibodies at day -21, and 5 µg of pPLP or PBS i.p. every other day from day -18 to day -2 before immunization with pPLP and CFA (day 0).

γ-irradiation *in EAE disease model.* For therapeutic experiments, mice were irradiated with 200 rad of γ-irradiation (Gammacell 40 Exactor, Best Theratronics) at the peak of acute EAE (usually day 10 after immunization) followed by normal splenic Mϕ/DC transfer (3 million/ mice). Some mice were given 5µg of peptides i.p. every other day from day 10-21. For prevention experiments, SJL mice were irradiated followed by normal splenic Mϕ/DC transfer (7 x10⁶/mouse). Some mice were given 25µg of pPLP i.p. every other day from -18 to day -2 before immunization with pPLP and CFA (day 0). In certain experiments, CD4+ CD25- T cells isolated from pMOG-specific TCR transgenic mice (2D2) (CD45.2+) were adoptively transferred into CD45.1+ C57BL/6 mice after the recipients were irradiated. The irradiated mice were then injected i.p. with pMOG (25 µg) every other day for 4 times. Statistical analyses. Group comparisons of parametric data were made by Student's t-test (unpaired two-tail). Data was tested for normality and variance and considered a P value of <0.05 significant.

γ-irradiation *in T1D disease model.* Nine-week-old female NOD mice were irradiated with 200 or 450 rads of γ-irradiation followed by transfer of MF/DCs (2 to 3 million per mouse). Some mice were given 5 mg of GAD₆₅ peptides intraperitoneally every other day for six times.

*Conversion of pOVA-specific Treg cells by B cell and CD8*+ *T cell depletion antibodies.* Balb/c mice were either untreated or treated with single i.p. injection of anti-CD8 (100 mg/mouse) and anti-CD20 (250 mg/mouse) at day 0. Then all mice were treated i.p. with pOVA (50mg/day/mouse) and i.p. injection of DO11.10xRag-/- TCR-transgenic CD4+CD25- T cells (KJ1-26+, pOVA-specific). The mice treated with aCD8/20 depletion antibodies were either treated with anti-TGFβ (αTGFβ or isotype control Ab (200 mg/day/mouse) once a day from day 2 to 4 (total 3 times, invert triangles). At day 8, all mice received i.p. injection of splenic DC (0.4 million cells/mouse). All mice were sacrificed at day 13.

*Conversion of pOVA-specific Treg cells by γ-irradiation.* Balb/c mice were either untreated or treated with 200 rad of γ-irradiation on day 0. All mice received i.p. injection of 5mg of pOVA on day 1. Some mice were treated with γ-irradiation followed by i.p. injection of splenic macrophages (Mf, 1.5 million cells/mouse) on day 0, and i.p. injection of 5mg of pOVA either in the presence of anti-TGFβ or isotype control Ab treatment. Anti-TGFβ or isotype control Ab (200 mg/day/mouse) was administered once a day from day 0 to 2 (invert triangles). All mice received i.p. injection of DO11.10xRag-/- TCR transgenic CD4+CD25-T cells (KJ1-26+) day 2. At day 4, all mice were immunized with pOVA (100 mg/mouse) and CFA (200 mg/mice) subcutaneously in the food pad. At day 11, all mice were sacrificed.

### Other Embodiments

From the foregoing description, it will be apparent that variations and modifications may be made to the invention described herein to adopt it to various usages and conditions.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

### References

1. Sakaguchi, S., Powrie, F. & Ransohoff, R.M. Re-establishing immunological self-tolerance in autoimmune disease. Nat Med 18, 54-58 (2012).
2. Littman, D.R. & Rudensky, A.Y. Th17 and regulatory T cells in mediating and restraining inflammation. Cell 140, 845-858 (2010).
3. Bluestone, J.A. & Abbas, A.K. Natural versus adaptive regulatory T cells. Nat Rev Immunol 3, 253-257 (2003).
4. Sakaguchi, S., Miyara, M., Costantino, C.M. & Hafler, D.A. FOXP3+ regulatory T cells in the human immune system. Nat Rev Immunol 10, 490-500 (2010).
5. Chen, W., et al. Conversion of peripheral CD4+CD25- naive T cells to CD4+CD25+ regulatory T cells by TGF-beta induction of transcription factor Foxp3. J Exp Med 198, 1875-1886 (2003).
6. Bilate, A.M. & Lafaille, J.J. Induced CD4+Foxp3+ regulatory T cells in immune tolerance. Annu Rev Immunol 30, 733-758 (2012).
7. Daniel, C. & von Boehmer, H. Extrathymic generation of regulatory T cellschances and challenges for prevention of autoimmune disease. Adv Immunol 112, 177-213 (2011).
8. Huter, E.N., Stummvoll, G.H., DiPaolo, R.J., Glass, D.D. & Shevach, E.M. Cutting edge: antigen-specific TGF beta-induced regulatory T cells suppress Th17- mediated autoimmune disease. J Immunol 181, 8209-8213 (2008).
9. Chen, W. Tregs in immunotherapy: opportunities and challenges. Immunotherapy 3, 911-914 (2011).
10. Kennedy, M.K., Clatch, R.J., Dal Canto, M.C., Trotter, J.L. & Miller, S.D. Monoclonal antibody-induced inhibition of relapsing EAE in SJL/J mice correlates with inhibition of neuroantigen-specific cell-mediated immune responses. J Neuroimmunol 16, 345-364 (1987).
11. Perruche, S., et al. CD3-specific antibody-induced immune tolerance involves transforming growth factor-beta from phagocytes digesting apoptotic T cells. Nat Med 14, 528-535 (2008).
12. Cobbold, S.P. & Waldmann, H. Therapeutic potential of monovalent monoclonal antibodies. Nature 308, 460-462 (1984).
13. Waldor, M.K., et al. Reversal of experimental allergic encephalomyelitis with monoclonal antibody to a T-cell subset marker. Science 227, 415-417 (1985).
14. Cobbold, S.P., Jayasuriya, A., Nash, A., Prospero, T.D. & Waldmann, H. Therapy with monoclonal antibodies by elimination of T-cell subsets in vivo. Nature 312, 548-551 (1984).
15. Fadok, V.A., et al. Macrophages that have ingested apoptotic cells in vitro inhibit proinflammatory cytokine production through autocrine/paracrine mechanisms involving TGF-beta, PGE2, and PAF. J Clin Invest 101, 890-898 (1998).
16. Chen, W., Frank, M.E., Jin, W. & Wahl, S.M. TGF-beta released by apoptotic T cells contributes to an immunosuppressive milieu. Immunity 14, 715-725 (2001).
17. Chung, E.Y., et al. Interleukin-10 expression in macrophages during phagocytosis of apoptotic cells is mediated by homeodomain proteins Pbx1 and Prep-1. Immunity 27, 952-964 (2007).
18. Shevach, E.M. Mechanisms of foxp3+ T regulatory cell-mediated suppression. Immunity 30, 636-645 (2009).
19. Chen, W. & Wahl, S.M. TGF-beta: the missing link in CD4+CD25+ regulatory T cell-mediated immunosuppression. Cytokine Growth Factor Rev 14, 85-89 (2003).
20. Bettelli, E., et al. Myelin oligodendrocyte glycoprotein-specific T cell receptor transgenic mice develop spontaneous autoimmune optic neuritis. J Exp Med 197, 1073-1081 (2003).
21. Duarte, J., et al. T cell apoptosis and induction of Foxp3+ regulatory T cells underlie the therapeutic efficacy of CD4 blockade in experimental autoimmune encephalomyelitis. J Immunol 189, 1680-1688 (2012).
22. Chatenoud, L. & Bluestone, J.A. CD3-specific antibodies: a portal to the treatment of autoimmunity. Nat Rev Immunol 7, 622-632 (2007).
23. Ferran, C., Bluestone, J., Bach, J.F. & Chatenoud, L. In vivo T lymphocyte activation induced in mice following the injection of anti-CD3 monoclonal antibody. Transplant Proc 22, 1922-1923 (1990).
24. Serhan, C.N. & Savill, J. Resolution of inflammation: the beginning programs the end. Nat Immunol 6, 1191-1197 (2005).
25. Wan, Y.Y. & Flavell, R.A. 'Yin-Yang' functions of transforming growth factor beta and T regulatory cells in immune regulation. Immunol Rev 220, 199-213 (2007).
26. Chen, W. & Wahl, S.M. TGF-beta: receptors, signaling pathways and autoimmunity. Curr Dir Autoimmun 5, 62-91 (2002).
27. Hsieh, C.S., Lee, H.M. & Lio, C.W. Selection of regulatory T cells in the thymus. Nat Rev Immunol 12, 157-167 (2012).
28. Wan, Y.Y. & Flavell, R.A. Regulatory T-cell functions are subverted and converted owing to attenuated Foxp3 expression. Nature 445, 766-770 (2007).
29. Zheng, Y. & Rudensky, A.Y. Foxp3 in control of the regulatory T cell lineage. Nat Immunol 8, 457-462 (2007).
30. Zhou, L., et al. TGF-beta-induced Foxp3 inhibits T(H)17 cell differentiation by antagonizing RORgammat function. Nature 453, 236-240 (2008).
31. Ohkura, N. & Sakaguchi, S. Regulatory T cells: roles of T cell receptor for their development and function. Semin Immunopathol 32, 95-106 (2010).
32. Bettelli, E., et al. Reciprocal developmental pathways for the generation of pathogenic effector TH17 and regulatory T cells. Nature 441, 235-238 (2006).
33. Dong, C. Diversification of T-helper-cell lineages: finding the family root of IL 17-producing cells. Nat Rev Immunol 6, 329-333 (2006).
34. Veldhoen, M., Hocking, R.J., Atkins, C.J., Locksley, R.M. & Stockinger, B. TGFbeta in the context of an inflammatory cytokine milieu supports de novo differentiation of IL-17-producing T cells. Immunity 24, 179-189 (2006).
35. Cobbold S.P, et al. 1986. Monoclonal antibodies to promote marrow engraftment and tissue graft tolerance. Nature. 323, 164-166.
36. Hale G, et al. 2001 CAMPATH-1 antibodies in stem-cell transplantation. Cytotherapy. 3, 145-164.
37. Calne R, et al. 2000 Prope tolerance with induction using Campath 1H and low-dose cyclosporin monotherapy in 31 cadaveric renal allograft recipients.
38. Qin S, et al. 1993 "Infectious" transplantation tolerance. Science. 259, 974-977.
39. Waldmann H, Cobbold S 2001 Regulating the immune response to transplants. A role for CD4+ regulatory cells?. Immunity. 14, 399-406.

## Claims

1. A pharmaceutical composition for use in the treatment of an autoimmune disease or disorder in a subject, the composition comprising:
(i) antibodies for inducing apoptosis in T cells, wherein the antibodies are an anti-CD8 antibody and an anti-CD20 antibody; and
(ii) an autoantigen specific to the autoimmune disease or disorder, selected from the group consisting of myelin basic protein (MBP), myelin proteolipid protein (PLP), insulin, GAD65 (glutamic acid decarboxylase), DiaPep227, heat-shock proteins, preferably Hsp65, Hsp90 and DnaJ, immunoglobulin binding protein (BiP), heterogeneous nuclear RNPs, annexin V, calpastatin, type II collagen, glucose-6-phosphate isomerase (GPI), elongation factor human cartilage gp39, and mannose binding lectin (MBL);
wherein the subject is tolerized to an antigen of the autoimmune disease.

2. The pharmaceutical composition for use according to claim 1 for tolerizing a subject suffering from an autoimmune disease or disorder to an antigen associated with the autoimmune disease or disorder.

3. The pharmaceutical composition for use according to claim 1 or 2, wherein the autoimmune disease or disorder is selected from the group consisting of multiple sclerosis, diabetes mellitus, rheumatoid arthritis, Sjogren syndrome and systemic sclerosis, preferably a late stage of the aforementioned diseases, more preferably type 1 diabetes mellitus.

4. The pharmaceutical composition for use according to any of claims 1 to 3, wherein the anti-CD 8 and anti-CD 20 antibodies induce depletion and/or apoptosis of B cells and T cells.

5. The pharmaceutical composition for use according to any of claims 1 to 4, wherein the subject is an animal, preferably a mammal, more preferably a human or non-human mammal selected from the group consisting of a human, primate, murine, bovine, equine, canine, ovine and feline.

6. The composition for use according to any of claims 1 to 5, wherein the disease or disorder is Sjogren's Syndrome.

## Patentansprüche

1. Eine pharmazeutische Zusammensetzung zur Verwendung in der Behandlung einer Autoimmunerkrankung oder -störung in einem Subjekt, wobei die Zusammensetzung Folgendes umfasst:
(i) Antikörper zur Induktion der Apoptose in T-Zellen, wobei die Antikörper ein Anti-CD8-Antikörper und ein anti-CD20-Antikörper sind; und
(ii) ein Autoantigen, das spezifisch für die Autoimmunerkrankung oder -störung ist, ausgewählt aus der Gruppe bestehend aus Myelin-Basisches Protein (MBP), Myelin-Proteolipid-Protein (PLP), Insulin, GAD65 (Glutaminsäuredecarboxylase), DiaPep227, Hitzeschockproteine, vorzugsweise Hsp65, Hsp90 und DnaJ, Immunoglobulin-bindendes Protein (BiP), heterogene nukleare RNPs, Annexin V, Calpastatin, Typ II Kollagen, Glukose-6-phosphat-Isomerase (GPI), Verlängerungsfaktor humaner Knorpel gp39, und Mannose bindendes Lektin (MBL);
wobei das Subjekt gegenüber einem Antigen der Autoimmunerkrankung tolerant gemacht wird.

2. Die pharmazeutische Zusammensetzung zur Verwendung gemäss Anspruch 1 zur Toleranzinduktion eines Subjekts, das an einer Autoimmunerkrankung oder -störung leidet, gegenüber einem Antigen, das mit der Autoimmunerkrankung oder -störung assoziiert ist.

3. Die pharmazeutische Zusammensetzung zur Verwendung gemäss Anspruch 1 oder2, wobei die Autoimmunerkrankung oder -störung ausgewählt ist aus der Gruppe bestehend aus Multipler Sklerose, Diabetes mellitus, rheumatische Arthritis, Sjögren Syndrom und systemischer Sklerose, vorzugsweise ein spätes Stadium der zuvor genannten Erkrankungen, mehr bevorzugt Typ 1 Diabetes mellitus.

4. Die pharmazeutische Zusammensetzung zur Verwendung gemäss einem der Ansprüche 1 bis 3, wobei die Anti-CD 8- und Anti-CD 20-Antikörper die Verringerung und/oder die Apoptose von B-Zellen und T-Zellen induzieren.

5. Die pharmazeutische Zusammensetzung zur Verwendung gemäss einem der Ansprüche 1 bis 4, wobei das Subjekt ein Tier, vorzugsweise ein Säugetier, mehr bevorzugt ein menschliches oder nicht-menschliches Säugetier ausgewählt aus der Gruppe bestehend aus Mensch, Primat, Maus, Rind, Pferd, Hund, Schaf und Katze ist.

6. Die pharmazeutische Zusammensetzung zur Verwendung gemäss einem der Ansprüche 1 bis 5, wobei die Erkrankung oder Störung Sjogren's Syndrom ist.

## Revendications

1. Composition pharmaceutique pour une utilisation dans le traitement d'une maladie ou d'un trouble auto-immuns chez un sujet, la composition comprenant:
(i) des anticorps pour induire une apoptose dans des lymphocytes T, où les anticorps sont un anticorps anti-CD8 et un anticorps anti-CD20; et
(ii) un auto-antigène spécifique à la maladie ou au trouble auto-immuns, choisi dans le groupe constitué de protéine basique de la myéline (MBP), protéine lipidoprotidique de la myéline (PLP), insuline, GAD65 (acide glutamique décarboxylase), DiaPep227, protéines de choc thermique, de préférence Hsp65, Hsp90 et DnaJ, protéine de liaison d'immunoglobuline (BiP), RNPs nucléaires hétérogènes, annexine V, calpastatine, collagène de type II, glucose-6-phosphate isomérase (GPI), facteur d'élongation de cartilage humain gp39 et lectine liant le mannose (MBL);
où le sujet est rendu tolérant à un antigène de la maladie auto-immune.

2. Composition pharmaceutique pour une utilisation selon la revendication 1 pour rendre tolérant un sujet souffrant d'une maladie ou d'un trouble auto-immuns à un antigène associé à la maladie ou au trouble auto-immuns.

3. Composition pharmaceutique pour une utilisation selon la revendication 1 ou 2, où la maladie ou le trouble auto-immuns est choisi dans le groupe constitué de sclérose en plaques, diabète sucré, polyarthrite rhumatoïde, syndrome de Sjögren et sclérose systémique, de préférence une phase tardive des maladies mentionnées ci-dessus, plus préférablement un diabète sucré de type 1.

4. Composition pharmaceutique pour une utilisation selon l'une quelconque des revendications 1 à 3, dans laquelle les anticorps anti-CD8 et anti-CD20 induisent l'épuisement et/ou l'apoptose de lymphocytes B et de lymphocytes T.

5. Composition pharmaceutique pour une utilisation selon l'une quelconque des revendications 1 à 4, où le sujet est un animal, de préférence un mammifère, plus préférablement un humain ou un mammifère non humain choisi dans le groupe constitué d'un humain, primate, murin, bovin, équin, canin, ovin et félin.

6. Composition pharmaceutique pour une utilisation selon l'une quelconque des revendications 1 à 5, où la maladie ou le trouble est un syndrome de Sjögren.


### SEQUENCE LISTING

<110> INSERM
<120> Combination of a ligand of HVEM and an immunotoxin for use in therapy
<130> BEP130213 EP
<160> 1
<170> PatentIn version 3.5
<210> 1
   <211> 25
   <212> PRT
   <213> Artificial Sequence
<220>
   <223> epitope
<400> 1
   Cys Pro Lys Cys Ser Pro Gly Tyr Arg Val Lys Glu Ala Cys Gly Glu
   1               5                   10                  15
   Leu Thr Gly Thr Val Cys Glu Pro Cys
               20                  25